(19) Europäisches Patentamt
European Patent Office
Office européen des brevets

(11) EP 4 204 551 B1

(12) **EUROPEAN PATENT SPECIFICATION**

(45) Date of publication and mention of the grant of the patent:
**17.09.2025 Bulletin 2025/38**

(21) Application number: **21769102.1**

(22) Date of filing: **24.08.2021**

(51) International Patent Classification (IPC):
***C12N 9/24*** *(2006.01)* ***C11D 3/386*** *(2006.01)*
***C12N 9/42*** *(2006.01)*

(52) Cooperative Patent Classification (CPC):
**C12Y 302/01004; C11D 3/38645; C12N 9/2405; C12N 9/2437; C12Y 302/01151;** C11D 2111/12; C11D 2111/14

(86) International application number:
**PCT/EP2021/073379**

(87) International publication number:
**WO 2022/043321 (03.03.2022 Gazette 2022/09)**

(54) **VARIANTS OF A FAMILY 44 XYLOGLUCANASE**

VARIANTEN EINER FAMILIE44-XYLOGLUCANASE

VARIANTS DE XYLOGLUCANASE DE LA FAMILLE 44

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.08.2020 IN 202041036857**
**26.05.2021 IN 202141023517**

(43) Date of publication of application:
**05.07.2023 Bulletin 2023/27**

(73) Proprietor: **Novozymes A/S**
**2880 Bagsvaerd (DK)**

(72) Inventors:
- **POULSEN, Thomas, Agersten**
**2880 Bagsvaerd (DK)**
- **DESHPANDE, Aishwarya**
**Bangalore 560066 (IN)**
- **SAINATHAN, Rajendra, Kulothungan**
**Bangalore 560066 (IN)**
- **CASSLAND, Bjoern, Lennart, Pierre, Alexander**
**2880 Bagsvaerd (DK)**
- **VENKATAKRISHNAN, Balasubramanian**
**Bangalore 560066 (IN)**
- **SHUKLA, Jinal, Kaushikkumar**
**Bangalore 560066 (IN)**
- **BHATTACHARYA, Simanti**
**Bangalore 560066 (IN)**
- **RAMANAND, Sidharth**
**Bangalore 560066 (IN)**
- **KRISHNA, Subith**
**Bangalore 560066 (IN)**

(74) Representative: **NVS EPO Representatives**
**Krogshoejvej 36**
**2880 Bagsvaerd (DK)**

(56) References cited:
**WO-A1-01/62903**
**WO-A1-2009/147210**
**WO-A1-2009/148983**
**WO-A1-2010/056652**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 204 551 B1

## Description

### FIELD OF THE INVENTION

The present invention relates to variants of a xyloglucanase belonging to family 44 of glycosyl hydrolases, polynucleotides encoding the variants and methods of producing the variants.

### BACKGROUND OF THE INVENTION

Xyloglucan is a major structural polysaccharide in the primary (growing) cell wall of plants. Structurally, xyloglucans consist of a cellulose-like beta-1,4-linked glucose backbone which is frequently substituted with various side chains. Xyloglucan is believed to function in the primary wall of plants by cross-linking cellulose micro fibrils, forming a cellulose-xyloglucan network.

Xyloglucanses are capable of catalyzing the solubilization of xyloglucan to xyloglucan oligosaccharides. Some xyloglucanases only exhibit xyloglucanase activity, whereas others exhibit both xyloglucanase and cellulase activity. Xyloglucanses may be classified in EC 3.2.1.4 or EC. 3.2.1.151. Enzymes with xyloglucanase activity are for example described in Vincken et al. (1997) Carbohydrate Research 298(4):299-310, wherein three different endoglucanases Endol, EndoV and EndoVI from *Trichoderma viride* (similar to *T. reesei*) are characterized. Endol, EndoV and EndoVI belongs to family 5, 7 and 12 of glycosyl hydrolases, respectively, see Henrissat, B. (1991) Biochem. J. 280: 309-316, and Henrissat, B. and Bairoch, A. (1993) Biochem. J. 293: 781-788. WO 94/14953 discloses a family 12 xyloglucanase (EG II) cloned from the fungus *Aspergillus aculeatus.* WO 99/02663 discloses family 12 and family 5 xyloglucanases cloned from *Bacillus licheniformis* and *Bacillus agaradhaerens,* respectively. WO 01/062903 discloses family 44 xyloglucanases.

In particular, WO 99/02663 and WO 01/062903 suggest that xyloglucanases may be used in detergents. WO 2009/147210, WO 2009/148983 and WO 2010/056652 of provide xyloglucanase variants.

It is an object of the present invention to provide variants of xyloglucanases belonging to family 44 of glycosyl hydrolases.

### SUMMARY OF THE INVENTION

The present invention relates to a xyloglucanase variant of the polypeptide of SEQ ID NO: 2 comprising the substitutions P111Q+S123P+V159M+A129T, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity to the polypeptide of SEQ ID NO: 1, SEQ ID NO: 2, or SEQ ID NO: 3, wherein the variant has a half-life improvement factor (HIF) and/or a stability improvement factor (SIF) of at least 1.1, such as at least 1.2, at least 1.3, at least 1.4, at least 1.5 compared to a reference polypeptide of SEQ ID NO: 2 or SEQ ID NO: 3.

The present invention also relates to isolated polynucleotides encoding the variants; nucleic acid constructs, vectors, and host cells comprising the polynucleotides; and methods of producing the variants.

The present invention also relates to cleaning methods and compositions comprising the variants of the invention.

### Sequences

SEQ ID NO: 1 mature polypeptide obtained from *Paenibacillus polymyxa.*
SEQ ID NO: 2 variant polypeptide.
SEQ ID NO: 3 variant polypeptide.
SEQ ID NO: 4 protease protein sequence from *Bacillus lentus.*

### Definitions

In accordance with this detailed description, the following definitions apply. Note that the singular forms "a," "an," and "the" include plural references unless the context clearly dictates otherwise.

Reference to "about" a value or parameter herein includes aspects that are directed to that value or parameter *per se.* For example, description referring to "about X" includes the aspect "X".

Unless defined otherwise or clearly indicated by context, all technical and scientific terms used herein have the same meaning as commonly understood by one of ordinary skill in the art to which this invention belongs.

Allelic variant: The term "allelic variant" means any of two or more alternative forms of a gene occupying the same

chromosomal locus. Allelic variation arises naturally through mutation and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

Amino acid: The term 'amino acid' as used herein, refers to the standard twenty genetically-encoded amino acids and their corresponding stereoisomers in the 'd' form (as compared to the natural 'l' form), omega-amino acids other naturally-occurring amino acids, unconventional amino acids (*e.g.* $\alpha$, $\alpha$ -disubstituted amino acids, N-alkyl amino acids, *etc.*) and chemically derivatised amino acids. Chemical derivatives of one or more amino acids may be achieved by reaction with a functional side group. Such derivatised molecules include, for example, those molecules in which free amino groups have been derivatised to form amine hydrochlorides, p-toluene sulphonyl groups, carboxybenzoxy groups, *t*-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (*e.g.* acetylation or thioglycolic acid amidation), terminal carboxylamidation (*e.g.* with ammonia or methylamine), and the like terminal modifications.

When an amino acid is being specifically enumerated, such as 'alanine' or 'Ala' or 'A', the term refers to both l-alanine and d-alanine unless explicitly stated otherwise. Other unconventional amino acids may also be suitable components for polypeptides of the present invention, as long as the desired functional property is retained by the polypeptide. For the peptides shown, each encoded amino acid residue, where appropriate, is represented by a single letter designation, corresponding to the trivial name of the conventional amino acid. In one embodiment, the polypeptides of the invention comprise or consist of l-amino acids

Cellulolytic enzyme or cellulase: The term "cellulolytic enzyme" or "cellulase" means one or more (*e.g.*, several) enzymes that hydrolyze a cellulosic material. Such enzymes include endoglucanase(s) (*e.g.* EC 3.2.1.4), cellobiohydrolase(s), beta-glucosidase(s), or combinations thereof. The two basic approaches for measuring cellulolytic enzyme activity include: (1) measuring the total cellulolytic enzyme activity, and (2) measuring the individual cellulolytic enzyme activities (endoglucanases, cellobiohydrolases, and beta-glucosidases) as reviewed in Zhang et al., 2006, Biotechnology Advances 24: 452-481. Total cellulolytic enzyme activity can be measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Pure Appl. Chem. 59: 257-68).

Cellulosic material: The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

cDNA: The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

Coding sequence: The term "coding sequence" means a polynucleotide, which directly specifies the amino acid sequence of a variant. The boundaries of the coding sequence are generally determined by an open reading frame, which begins with a start codon such as ATG, GTG or TTG and ends with a stop codon such as TAA, TAG, or TGA. The coding sequence may be a genomic DNA, cDNA, synthetic DNA, or a combination thereof.

Control sequences: The term "control sequences" means nucleic acid sequences necessary for expression of a polynucleotide encoding a variant of the present invention. Each control sequence may be native (*i.e.*, from the same gene) or foreign (*i.e.*, from a different gene) to the polynucleotide encoding the variant or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a variant.

Dish washing composition: The term "dish washing composition" as used herein, refers to all forms of compositions for cleaning hard surfaces. The present invention is not restricted to any particular type of dish wash composition or any particular detergent. Thus, in one embodiment, the dish washing composition is a liquid dish washing composition, a powder dish washing composition, wherein the composition may optionally be in the form of a unit dose.

Detergent component: the term "detergent component" is defined herein to mean the types of chemicals which can be used in detergent compositions. Examples of detergent components are surfactants, hydrotropes, builders, co-builders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants, and solubilizers. The detergent composition may comprise of one or more of any type of detergent component.

Detergent composition: the term "detergent composition" refers to compositions that find use in the removal of undesired compounds from items to be cleaned, such as textiles, dishes, and hard surfaces. The detergent composition may be used to e.g. clean textiles, dishes and hard surfaces for both household cleaning and industrial cleaning and/or for fabric care. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; hard surface cleaning formulations, such as for glass, wood, plastic, ceramic and metal counter tops and windows; carpet cleaners; oven cleaners; fabric fresheners; fabric softeners; and textile and laundry pre-spotters, as well as dish wash detergents). In addition to containing an enzyme of the invention, the detergent formulation may contain one or more additional enzymes (such as amylases, proteases, peroxidases, cellulases, betaglucanases, xyloglucanases, hemicellulases, xanthanases, xanthan lyases, lipases, acyl transferases, phospholipases, esterases, laccases, catalases, aryl esterases, amylases, alpha-amylases, glucoamylases, cutinases, pectinases, pectate lyases, keratinases, reductases, oxidases, phenoloxidases, lipoxygenases, ligninases, carrageenases, pullulanases, tannases, arabinosidases, hyaluronidases, chondroitinases, xyloglucanases, xylanases, pectin acetyl esterases, polygalacturonases, rhamnogalacturonases, endo-beta-mannanases, exo-beta-mannanases (GH5 and/or GH26), licheninases, phosphodiesterases, pectin methylesterases, cellobiohydrolases, transglutaminases, nucleases, and combinations thereof, or any mixture thereof), and/or components such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti corrosion agents, enzyme inhibitors or stabilizers, enzyme activators, transferase(s), hydrolytic enzymes, oxido reductases, bluing agents and fluorescent dyes, antioxidants, and solubilizers.

Dish wash: The term "dish wash" refers to all forms of washing dishes, e.g. by hand dish wash (HDW) or automatic dish wash (ADW). Washing dishes includes, but is not limited to, the cleaning of all forms of crockery such as plates, cups, glasses, bowls, all forms of cutlery such as spoons, knives, forks and serving utensils as well as ceramics, plastics, metals, china, glass and acrylics.

Enzyme Detergency benefit: The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and/or cleaning, prevention or reduction of redeposition of soils released in the washing process (an effect that also is termed anti-redeposition), restoring fully or partly the whiteness of textiles which originally were white but after repeated use and wash have obtained a greyish or yellowish appearance (an effect that also is termed whitening). Textile care benefits, which are not directly related to catalytic stain removal or prevention of redeposition of soils, are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric-softness, colour clarification of the fabric and removal of particulate soils which are trapped in the fibers of the fabric or garment. Enzymatic bleaching is a further enzyme detergency benefit where the catalytic activity generally is used to catalyze the formation of bleaching components such as hydrogen peroxide or other peroxides.

Expression: The term "expression" includes any step involved in the production of a variant including, but not limited to, transcription, post-transcriptional modification, translation, post-translational modification, and secretion.

Expression vector: The term "expression vector" means a linear or circular DNA molecule that comprises a polynucleotide encoding a variant and is operably linked to control sequences that provide for its expression.

Fragment: The term "fragment" means a polypeptide having one or more (e.g., several) amino acids absent from the amino and/or carboxyl terminus of a mature polypeptide; wherein the fragment has xyloglucanase activity. In one aspect, a fragment contains at least 445 amino acid residues at least 471 amino acid residues, or at least 497 amino acid residues.

Fusion polypeptide: The term "fusion polypeptide" is a polypeptide in which one polypeptide is fused at the N-terminus or the C-terminus of a variant of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779). A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

Hard surface cleaning: The term "Hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, cutlery such as spoons, knives, forks, serving utensils, ceramics, plastics, metals, china, glass and acrylics.

Host cell: The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

Hybrid polypeptide: The term "hybrid polypeptide" means a polypeptide comprising domains from two or more polypeptides from different sources (origins), e.g., a binding module from one polypeptide and a catalytic domain from another polypeptide. The domains may be fused at the N-terminus or the C-terminus. Of particular interest herein are polypeptides comprising a binding module from one polypeptide (which may be naturally occurring or further modified), an engineered linker region, such as a proline-rich linker region, which is a synthetic construct, and a catalytic domain from another polypeptide (which may be naturally occurring or further modified).

Hybridization: The term "hybridization" means the pairing of substantially complementary strands of nucleic acids, using standard Southern blotting procedures. Hybridization may be performed under medium, medium-high, high or very high stringency conditions. Medium stringency conditions means prehybridization and hybridization at 42°C in 5X SSPE, 0.3% SDS, 200 micrograms/ml sheared and denatured salmon sperm DNA, and 35% formamide for 12 to 24 hours, followed by washing three times each for 15 minutes using 0.2X SSC, 0.2% SDS at 55°C. Medium-high stringency conditions means prehybridization and hybridization at 42°C in 5X SSPE, 0.3% SDS, 200 micrograms/ml sheared and denatured salmon sperm DNA, and 35% formamide for 12 to 24 hours, followed by washing three times each for 15 minutes using 0.2X SSC, 0.2% SDS at 60°C. High stringency conditions means prehybridization and hybridization at 42°C in 5X SSPE, 0.3% SDS, 200 micrograms/ml sheared and denatured salmon sperm DNA, and 50% formamide for 12 to 24 hours, followed by washing three times each for 15 minutes using 0.2X SSC, 0.2% SDS at 65°C. Very high stringency conditions means prehybridization and hybridization at 42°C in 5X SSPE, 0.3% SDS, 200 micrograms/ml sheared and denatured salmon sperm DNA, and 50% formamide for 12 to 24 hours, followed by washing three times each for 15 minutes using 0.2X SSC, 0.2% SDS at 70°C.

Improved property: The term "improved property" means a characteristic associated with a variant that is improved compared to the reference enzyme/parent enzyme. Such improved properties include, but are not limited to improved wash performance, improved enzyme detergency benefit, improved stability, and/or improved whiteness.

Improved stability: The term "improved stability" means a variant enzyme displaying retention of enzymatic activity after a period of incubation in the presence of a chemical or chemicals, either naturally occurring or synthetic, which reduces the enzymatic activity of the parent enzyme. Improved stability means that the variant enzyme has better stability in the presence of protease relative to the stability of a reference enzyme/parent enzyme, and includes, for example, proteolytic stability, in-detergent storage stability, in-detergent storage stability in the presence of a chelator or chelating agent, improved stability during production of the detergent composition, as well as in-wash stability. The improved detergent stability is, in particular, an improved stability of the xyloglucanase activity when a xyloglucanase variant of the present invention is mixed into a liquid detergent formulation or a unit dose detergent formulation and then stored at temperatures between 15 and 50 °C.

In the present invention liquid detergents are particular useful as liquid laundry detergents and/or unit dose laundry detergents.

The improvement in stability can be quantified, for example, by the detergent stability assay as described in Example 3.

Improved wash performance: The term "improved wash performance" is defined herein as an enzyme displaying

an increased wash performance in a detergent composition relative to the wash performance of a reference enzyme/-parent enzyme, e.g., by increased color clarification and/or anti-pilling effect, when evaluating the fresh samples and/or after the samples have been stored under the same conditions. The term "improved wash performance" includes wash performance in laundry but also in, e.g., hard surface cleaning such as automated dish wash (ADW).

Isolated: The term "isolated" means a polypeptide, nucleic acid, cell, or other specified material or component that is separated from at least one other material or component with which it is naturally associated as found in nature, including but not limited to, for example, other proteins, nucleic acids, cells, etc. An isolated polypeptide includes, but is not limited to, a culture broth containing the secreted polypeptide.

Laundering: The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

Mature polypeptide: The term "mature polypeptide" means a polypeptide in its mature form following N-terminal processing (*e.g.*, removal of signal peptide).

Mature polypeptide coding sequence: The term "mature polypeptide coding sequence" means a polynucleotide that encodes a mature polypeptide having xyloglucanase activity.

Mutant: The term "mutant" means a polynucleotide encoding a variant.

Modification: The term "modification", in the context of the polypeptides of the invention, means that one or more amino acids within the reference amino acid sequence (*i.e.* SEQ ID NO: 1, 2 or 3) are altered by substitution with a different amino acid, by insertion of an amino acid or by deletion, preferably by at least one deletion. The terms "modification", "alteration", and "mutation" may be used interchangeably and constitute the same meaning and purpose.

Nucleic acid construct: The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

Operably linked: The term "operably linked" means a configuration in which a control sequence is placed at an appropriate position relative to the coding sequence of a polynucleotide such that the control sequence directs expression of the coding sequence.

Parent or parent xyloglucanase: The term "parent" or "parent xyloglucanase" means any polypeptide with xyloglucanase activity, to which an alteration, e.g., substitution(s), insertion(s), deletion(s) and/or truncation(s) is made to produce the xyloglucanase variants of the present invention. The parent may be a naturally occurring (wild-type) polypeptide or a variant or fragment thereof. The parent may be a naturally occurring (wild-type) polypeptide such as the enzyme of SEQ ID NO: 1 or a polypeptide which having at least 60%, more preferably at least 65%, more preferably at least 70%, more preferably at least 75%, more preferably at least 80%, more preferably at least 85%, even more preferably at least 90%, at least 91%, at least 92%, at least 93%, at least 94%, at least 95%, at least 96%, at least 97%, at least 98%, or at least 99% sequence identity thereto. The parent polypeptide may also be a variant of a naturally occurring polypeptide which has been modified or altered in the amino acid sequence, such as the polypeptide of SEQ ID NO: 2 or SEQ ID NO: 3 herein. A parent may also be an allelic variant, which is a polypeptide encoded by any of two or more alternative forms of a gene occupying the same chromosomal locus.

Purified: The term "purified" means a nucleic acid or polypeptide that is substantially free from other components as determined by analytical techniques well known in the art (*e.g.*, a purified polypeptide or nucleic acid may form a discrete band in an electrophoretic gel, chromatographic eluate, and/or a media subjected to density gradient centrifugation). A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (*e.g.*, percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

Recombinant: The term "recombinant," when used in reference to a cell, nucleic acid, protein or vector, means that it has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e.g.*, a heterologous promoter in an expression vector. Recombinant proteins may differ from a native sequence by one or more amino acids and/or are fused with heterologous sequences. A vector comprising a nucleic acid encoding a polypeptide is a recombinant vector. The term "recombinant" is synonymous with "genetically modified" and "transgenic".

Sequence identity: The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment - Total Number of Gaps in Alignment)

Subsequence: The term "subsequence" means a polynucleotide having one or more nucleotides absent from the 5' and/or 3' end of a mature polypeptide coding sequence; wherein the subsequence encodes a fragment having xyloglucanase activity.

Textile: The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used, it is intended to include the broader term textiles as well.

Variant: The term "variant" means a polypeptide having xyloglucanase activity comprising an alteration at one or more (*e.g.*, several) positions and retaining the activity of the parent. A substitution means replacement of the amino acid occupying a position with a different amino acid. The variants of the present invention have at least 90%, at least 91%, at least 92%, at least 93%, at least 94%, at least 95%, at least 96%, at least 97%, at least 98%, at least 99%, or at least 100% of the xyloglucanase activity of the polypeptide of SEQ ID NO: 1.

Wash liquor: The term "wash liquor" refers to an aqueous solution containing a detergent composition in dilute form, such as but not limited to a detergent solution containing a laundry detergent composition in dilute form such as the wash liquor in a laundry process.

Whiteness: The term "Whiteness" is defined herein as a broad term with different meanings in different regions and for different consumers. Loss of whiteness can e.g. be due to greying, yellowing, or removal of optical brighteners/hueing agents. Greying and yellowing can be due to soil redeposition, body soils, coloring from e.g. iron and copper ions or dye transfer. Whiteness might include one or several issues from the list below: colorant or dye effects; incomplete stain removal (e.g. body soils, sebum etc.); redeposition (greying, yellowing or other discolorations of the object) (removed soils re-associate with other parts of textile, soiled or unsoiled); chemical changes in textile during application; and clarification or brightening of colors.

Wild-type: The term "wild-type" in reference to an amino acid sequence or nucleic acid sequence means that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild- type sequence).

Xyloglucanase activity: The term "xyloglucanase activity" is defined herein as an enzyme catalyzed hydrolysis of

xyloglucan. The reaction involves endo hydrolysis of 1,4-beta-D-glucosidic linkages in xyloglucan. For purposes of the present invention, xyloglucanase activity is determined using AZCL-xyloglucan (from Megazyme) as the reaction substrate. The assay can be performed in several ways, e.g. as described in Example 2 of the present application or as described in WO 01/62903. One unit of xyloglucanase activity (XyloU) is defined by reference to the assay method described in WO 01/62903, page 60, lines 3 - 17.

**Conventions for Designation of Variants**

For purposes of the present invention, the amino acid sequence of the xyloglucanase disclosed in SEQ ID NO: 1 is used to determine the corresponding amino acid residue in another xyloglucanase. The amino acid sequence of another xyloglucanase is aligned with the amino acid sequence of the xyloglucanase disclosed in SEQ ID NO: 1, and based on the alignment the amino acid position number corresponding to any amino acid residue in the amino acid sequence of the xyloglucanase disclosed in SEQ ID NO: 1 can be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

In describing the variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), *e.g.*, "Gly205Arg + Ser411Phe" or "G205R + S411F", representing substitutions at positions 205 and 411 of glycine (G) with arginine I and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.*, "Gly195* + Ser411*" or "G195* + S411*".

Insertions. For an amino acid insertion, the following nomenclature is used: Original amino acid, position, original amino acid, inserted amino acid. Accordingly the insertion of lysine after glycine at position 195 is designated "Gly195-GlyLys" or "G195GK". An insertion of multiple amino acids is designated [Original amino acid, position, original amino acid, inserted amino acid #1, inserted amino acid #2; etc.]. For example, the insertion of lysine and alanine after glycine at position 195 is indicated as "Gly195GlyLysAla" or "G195GKA".

In such cases the inserted amino acid residue(s) are numbered by the addition of lower case letters to the position number of the amino acid residue preceding the inserted amino acid residue(s). In the above example, the sequence would thus be:

| Parent: | Variant: |
|---|---|
| 195 | 195 195a 195b |
| G | G - K - A |

Multiple alterations. Variants comprising multiple alterations are separated by addition marks ("+"), *e.g.*, "Arg170Tyr+Gly195Glu" or "R170Y+G195E" representing a substitution of arginine and glycine at positions 170 and 195 with tyrosine and glutamic acid, respectively.

Different alterations. Where different alterations can be introduced at a position, the different alterations are separated by a comma, *e.g.*, "Arg170Tyr,Glu" represents a substitution of arginine at position 170 with tyrosine or glutamic acid. Thus, "Tyr167Gly,Ala + Arg170Gly,Ala" designates the following variants:
"Tyr167Gly+Arg170Gly", "Tyr167Gly+Arg170Ala", "Tyr167Ala+Arg170Gly", and "Tyr167 Ala+Arg 170Ala".

## DETAILED DESCRIPTION OF THE INVENTION

### Xyloglucanase Variants

The present invention relates to a xyloglucanase variant of the polypeptide of SEQ ID NO: 2 comprising the substitutions P111Q+S123P+V159M+A129T, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity to the polypeptide of SEQ ID NO: 1, SEQ ID NO: 2, or SEQ ID NO: 3,

wherein the variant has a half-life improvement factor (HIF) and/or a stability improvement factor (SIF) of at least 1.1, such as at least 1.2, at least 1.3, at least 1.4, at least 1.5 compared to a reference polypeptide of SEQ ID NO: 2 or SEQ ID NO: 3..

In the invention the variant has a sequence identity of at least 85%, at least 90%, at least 91%, at least 92%, at least 93%, at least 94%, at least 95%, at least 96%, at least 97%, at least 98%, or at least 99%, but less than 100%, to the amino acid sequence of the parent xyloglucanase.

In another embodiment, the variant has at least 85%, at least 90%, at least 91%, at least 92%, at least 93%, at least 94%, at least 95%, such as at least 96%, at least 97%, at least 98%, or at least 99%, but less than 100%, sequence identity to the polypeptide of SEQ ID NO: 1.

In another embodiment, the variant has at least 85%, at least 90%, at least 91%, at least 92%, at least 93%, at least 94%, at least 95%, such as at least 96%, at least 97%, at least 98%, or at least 99%, but less than 100%, sequence identity to the polypeptide of SEQ ID NO: 2.

In another embodiment, the variant has at least 85%, at least 90%, at least 91%, at least 92%, at least 93%, at least 94%, at least 95%, such as at least 96%, at least 97%, at least 98%, or at least 99%, but less than 100%, sequence identity to the polypeptide of SEQ ID NO: 3.

In one aspect, the number of alterations is 1-50, e.g., 1-45, 1-40, 1-35, 1-30, 1-25, 1-20, 1-15, 1-10 or 1-5, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 alterations.

In one aspect, the number of substitutions is 1-50, e.g., 1-45, 1-40, 1-35, 1-30, 1-25, 1-20, 1-15, 1-10 or 1-5, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 substitutions.

In one aspect, the number of deletions is 1-50, e.g., 1-45, 1-40, 1-35, 1-30, 1-25, 1-20, 1-15, 1-10 or 1-5, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 deletions.

In one aspect, the substituted amino acid residue is different from the naturally-occurring amino acid residue in that position. In one embodiment, the substitution is selected from the group consisting of A, C, D, E, F, G, H, I, K, L, M, N, P, Q, R, S, T, V, W and Y, with the proviso that the substituted amino acid residue is different from the naturally-occurring amino acid residue in that position.

In one embodiment the xyloglucanase variants of the invention are isolated variants.

In another aspect, a variant comprises alteration at each positions corresponding to positions selected from the group consisting of 111, 123, 129, 159, 256, 294, 8, 18, 20, 41, 42, 76, 82, 83, 87, 94, 103, 104, 105, 121, 125, 126, 127, 136, 137, 146, 147, 148, 152, 153, 155, 165, 168, 169, 177, 184, 189, 203, 206, 210, 211, 214, 217, 219, 220, 226, 237, 238, 240, 243, 244, 248, 251, 252, 267, 271, 276, 289, 295, 298, 300, 302, 322, 329, 339, 347, 347, 353, 383, 384, 392, 394, 395, 402, 414, 427, 431, 445, 447, 459, 473, 474, 476, 482, 488, 489, 491, 492, 503 and 505 of the polypeptide of SEQ ID NO: 1, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In another aspect, a variant comprises alteration at each positions corresponding to positions selected from the group consisting of 111, 123, 129, 159, 256, 294, 8, 18, 20, 41, 42, 76, 82, 83, 87, 94, 103, 104, 105, 121, 125, 126, 127, 136, 137, 146, 147, 148, 152, 153, 155, 165, 168, 169, 177, 184, 189, 203, 206, 210, 211, 214, 217, 219, 220, 226, 237, 238, 240, 243, 244, 248, 251, 252, 267, 271, 276, 289, 295, 298, 300, 302, 322, 329, 339, 347, 347, 353, 383, 384, 392, 394, 395, 402, 414, 427, 431, 445, 447, 459, 473, 474, 476, 482, 488, 489, 491, 492, 503 and 505 of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises one or more of the following alterations at positions corresponding to positions P111, S123, V159, S256, I294, K8, K18, R20, A41, A42, S76, Q82, A83, K87, S94, G103, T104, Y105, A118, N121, Q125, E126, S127, A129, N136, Q137, F146, Q147, L148, L152, N153, N155, F165, N168, K169, A177, L184, A189, V203, K206, D210, R211, S214, K217, V219, K220, A226, G237, A238, K240, Q243, T244, W248, V251, K252, R267, Q271, R276, A289, R295, N298, V300, N302, K322, Q329, P339, K347, R347, K353, R353, N383, D384, K392, K394, D395, P395, S402, K414, T427, V431, K445, L447, A459, 1473, S474, K476, K482, K488, E489, A491, P492, Y503 and V505 of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises one or more of the following alterations at positions corresponding to positions P111Q, S123P, V159M, S256E, S256Q, I294E, I294Q, K8E, K8R, K18E, R20K, A41L, A41E, A41R, A42V, S76E, Q82E,

A83E, K87E, S94R, G103V, T104G, T104R, Y105E, A118K, N121E, Q125F, Q125K, Q125L, Q125P, Q125S, E126P, S127H, S127L, S127W, S127D, A129T, N136D, Q137E, Q137K, F146D, Q147G, Q147K, L148P, L152*, L152D, L152E, L152P, N153E, N155D, N155E, F165H, N168R, K169E, K169R, A177G, L184M, A189G, V203T, K206E, K206R, D210H, D210R, R211K, S214Q, K217R, K217T, V219A, V219T, K220R, A226D, A226K, G237M, A238S, A238T, K240F, K240L, Q243E, T244E, T244R, W248V, V251E, K252E, R267C, R267H, R267K, Q271D, Q271E, R276K, A289T, R295K, N298D, V300L, N302H, K322E, Q329E, P339S, K347E, K347R, K353R, N383E, N383Q, D384G, K392E, K394R, D395P, S402Q, K414E, T427V, V431E, K445E, L447M, A459P, I473T, S474E, K476R, K482R, K488T, E489K, E489R, A491E, A491V, P492D, Y503L, Y503V and V505L of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: S123P+R211K+K217R+S256Q+K488T+A129T,R20K+Y105E+S123P+Q147K+R267K+A129T, K87E+P111Q+S123P+V159M+S402Q+A129T,K87E+P111Q+V159M+I294Q+I473T+A129T,K8 E+K18E+P111Q+V159M+K206E+A129T,R20K+P111Q+S123P+S127D+V159M+A129T,P111 Q+V159M+K206E+1294Q+K347E+A129T,P111Q+Q137K+Q147K+V159M+K252E+A129T,P11 1Q+Q137K+Q147K+N155D+K252E+A129T,P111Q+Q137K+L152D+V203T+K217R+A129T,P1 11Q+S123P+Q137K+V159M+K488T+A129T,P111Q+S123P+Q137K+V159M+S256Q+A129T, K87E+P111Q+Q147K+L152D+V159M+A129T,R20K+A83E+S123P+K220R+S256E+A129T,R2 OK+A83E+S123P+K220R+K252E+A129T,R20K+Q82E+S123P+Q147K+K220R+A129T,R20K+ A83E+S123P+K220R+S256Q+A129T,R20K+Q82E+S123P+N155D+K220R+A129T,R20K+S12 3P+V203T+K220R+K252E+A129T,R20K+A41L+Q82E+S123P+K220R+A129T,R20K+A42V+S 76E+S123P+K220R+A129T,R20K+A42V+A83E+S123P+K220R+A129T, A83E+P111Q+S123P+V159M+K252E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: Y105E+A118K+S123P+K206R+K220R+R267K+A129T,A41L+P111Q+S123P+Q147K+V159M +V203T+A129T,A41L+P111Q+S123P+Q147K+V159M+K217R+A129T,P111Q+S123P+Q147K +V159M+1294Q+S402Q+A129T,P111Q+Q137K+Q147K+V159M+V203T+K217R+A129T,K87E +P111Q+L1520+V159M+V203T+1294Q+A129T,R20K+S76E+A83E+S123P+K220R+K252E+A 129T,R20K+A42V+S123P+K220R+K252E+1294E+A129T,R20K+A83E+S123P+V203T+K220R +K252E+A129T,R20- K+A42V+S76E+S123P+V203T+K220R+A129T,A83E+P111Q+S123P+V1 59M+S256E+1294E+A129T,Q82E+P111Q+S123P+V159M+S256E+1294E+A129T,Q82E+P111 Q+S123P+Q147K+V159M+I294E+A129T,K87E+P111Q+S123P+V159M+K217T+I294E+A129 T,A83E+P111Q+S123P+V159M+K240F+K252E+A129T,K87E+P111Q+S123P+V159M+S256Q +1294E+A129T,K87E+P111Q+S123P+V159M+K347E+N383E+A129T,Q82E+P111Q+S123P+ N1550+S256E+1294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: P111Q+S123P+Q137K+Q147K+V159M+S256Q+S402Q+A129T,A41L+P111Q+Q137K+V159 M+N168R+Q271D+K488T+A129T,S76E+Q82E+K87E+P111Q+S123P+V159M+V203- T+A129T ,Q82E+P111Q+S123P+Q147K+V159M+S256E+1294E+A129T,K8E+Q82E+P111Q+S123P+V1 59M+S256E+1294E+A129T,Q82E+P111Q+S123P+V159M+K240F+S256E+1294E+A129T,Q82 E+P111Q+S123P+V159M+G237M+V251E+1294E+A129T of the polypeptide of SEQ ID NO: 2, wherein the variant has xyloglucanase activity and wherein said variant has, at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: A83E+P111Q+S123P+Q137K+Q147K+V159M+S256Q+S402Q+A129T,P111Q+S123P+Q137K +Q147K+N155D+S256Q+A289T+N302H+A129T,P111Q+S123P+Q137K+Q147K+V159M+K25 2E+S256Q+S402Q+A129T,P111Q+S123P+Q137K+Q147K+N155D+S256Q+1294E+S402Q+A 129T,S76E+P111Q+S123P+Q137K+Q147K+V159M+S256Q+S402Q+A129T,K8R+P111Q+S1 23P+Q137K+Q147K+V159M+S256Q+S402Q+A129T,S123P+S1270+N1360+Q137K+Q147K+ L152E+N153- E+N155E+A129T,R20K+S123P+K169R+K217T+K240F+S256Q+R267H+I294E+

A129T,Q82E+P111Q+S123P+V159M+V203T+G237M+S256E+1294E+A129T,Q82E+P111Q+S 123P+N155D+K169R+G237M+S256E+1294E+A129T,Q82E+P111Q+S123P+Q137K+V159M+ G237M+S256E+1294E+A129T,Q82E+P111Q+S123P+Q137K+V159M+K240F+S256E+1294E+ A129T,Q82E+P111Q+S123P+Q147K+N155D+K240F+S256E+1294E+A129T,A83E+P111Q+S 123P+Q147K+V159M+S256E+1294E+Q329E+A129T,Q82E+P111Q+S123P+V159M+K169R+ S256E+1294E+V431E+A129T,A41L+Q82E+P111Q+S123P+V159M+S256E+1294E+N383E+A1 29T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: S76E+P111Q+S123P+Q137E+Q147K+V159M+K252E+S256Q+S402Q+A129T,P111Q+N121E +Q137K+Q147K+V159M+K169R+S256Q+1294E+S402Q+A129T,P111Q+S123P+Q137K+Q14 7K+V159M+K169R+S256Q+I294E+S402Q+A129T,P111Q+S123P+Q137K+Q147K+V159M+L 184M+V219T +S256Q+S402Q+A129T,A83E+P111Q+S123P+Q137K+Q147K+V159M+L 184M+ S256Q+S402Q+A129T,Q82E+P111Q+S123P+Q137K+Q147K+V159M+K252E+S256Q+S402 Q+A129T,S123P+S127D+N136D+Q137K+Q147K+L152E+N153E+N155E+A491E+A129T,Q82 E+P111Q+S123P+V159M+A177G+K240F+S256E+1294E+0384G+A129T,K8R+Q82E+P111Q +S123P+V159M+K169R+K240F+S256E+I294E+A129T,R20K+S123P+Q137K+K169R+K217T +K240F+S256Q+R267H+1294E+A129T,R20K+S123P+N1550+K169R+K217T+K240F+S256Q +R267H+1294E,R20K+S123P+Q147K+K169R+V219T+K240F+S256Q+R267H+1294E+A129T, Q82E+P111Q+S123P+V159M+V203T+G237M+K252E+S256E+1294E+A129T,S76E+Q82E+P 111Q+S123P+V159M+V203T+G237M+S256E+1294E+A129T,Q82E+P111Q+S123P+Q147K+ V159M+V203T+G237M+S256E+1294E+A129T,Q82E+P111Q+S123P+V159M+V203T+G237M +T244R+S256E+I294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: A83E+P111Q+S123P+Q137K+Q147K+V159M+L184M+S256Q+1294E+S402Q+A129T,A83E+ P111Q+S123P+Q137K+Q147K+N1550+L184M+S256Q+I294E+S402Q+A129T,R20K+S123P+ Q147K+N155D+K169R+K217T+K240F+S256Q+R267H+I294E+A129T,R20K+S123P+N155D+ K169R+K217T+K240F+S256Q+R267H+I294E+S474E+A129T,R20K+A41L+S123P+L152P+K1 69R+K217T+K240F+S256Q+R267H+I294E+A129T,K8R+R20K+S123P+K169R+D210H+K217 T+K240F+S256Q+R267H+1294E+A129T,P111Q+S123P+Q137K+Q147K+V159M+G237M+T2 44R+S256Q+1294E+S402Q+A129T,P111Q+S123P+Q137K+Q147K+V159M+K169R+V203T+S 256Q+1294E+S402-Q+A129T,Q82E+P111Q+S123P+Q137K+V159M+V203T+0210H+G237M+ S256E+1294E+A129T,Q82E+P111Q+S123P+V159M+K169R+V203T+G237M+T244R+S256E+ 1294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of: Q82E+P111Q+S123P+Q137K+Q147K+V159M+L184M+A238S+S256Q+1294E+S402-Q+A129T ,A83E+P111Q+S123P+Q137K+Q147K+N1550+L184M+G237M+S256Q+1294E+S402Q+A129 T,A83E+P111Q+S123P+Q137K+Q147K+V159M+L184M+G237M+S256E+1294E+S402Q+A12 9T,R20K+S123P+N1550+K169R+K217T+K240F+S256Q+R267H+1294E+0384G+E489R+A12 9T,R20K+S123P+K169R+K217T+K240F+S256Q+R267H+I294E+Q329E+V431E+E489R+A12 9T,P111Q+S123P+Q137K+Q147K+V159M+K169R+K252E+S256Q+I294E+K322E+S402Q+A1 29T,P111Q+S123P+Q137K+Q147K+V159M+K169R+K240F+S256Q+I294E+K322E+S402Q+A 129T,S76E+P111Q+S123P+Q137K+Q147K+V159M+K169R+G237M+S256Q+I294E+S402Q+ A129T,P111Q+S123P+Q137K+Q147K+V159M+K169R+G237M+T244R+S256Q+I294E+S402 Q+A129T,P111Q+S123P+Q137K+Q147K+V159M+K169R+V219T+K240F+S256Q+I294E+S40 2Q+A129T,P111Q+S123P+Q137K+Q147K+V159M+V251E+S256E+Q271E+1294E+Q329E+S 402Q+A129T,Q82E+P111Q+S123P+L152P+V159M+K169R+V203T+G237M+T244R+S256E+1 294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1,

SEQ ID NO: 2, or SEQ ID NO: 3.

In one aspect, a variant comprises alterations at positions corresponding to positions selected from a group consisting of:AB3E+P111Q+S123P+Q137K+F146D+Q147G+L148P+V159M+L184M+S5256Q+I294E+S402Q+A129T,A83E+P111Q+S123P+Q137K+Q147K+V159M+L184M+S256Q+I294E+N2980+V300L+S402Q+A129-T,A83E+P111Q+S123P+Q137K+Q147K+V159M+K169R+L184M+K240L+S256Q+1294E+S402Q+A129-T,A83E+P111Q+S123P+Q137K+Q147K+V159M+L184M+G237M+S256Q+1294E+S402Q+K488T+A129-T,A83E+P111Q+S123P+Q137K+Q147K+V159M+L184M+V203T+K240F+S256Q+1294E+S402Q+A129-T,A83E+P111Q+S123P+Q137K+Q147K+V159M+L184M+K240F+S256Q+1294E+S402Q+E489R+A129-T,A83E+P111Q+S123P+Q137K+Q147K+V159M+F165H+L184M+S256Q+I294E+S402Q+V431E+A129-T,A83E+P111Q+S123P+Q137K+Q147K+V159M+L184M+V251E+Q271E+1294E+Q329E+S402Q,P111Q+S123P+Q137K+Q147K+V159M+K169R+0210H+K240F+S256E+I294E+S402Q+K488T+A129T,S76E+P111Q+S123P+Q137K+Q147K+V159M+K169R+V203T+G237M+S256Q+I294E+S402Q+A129T,P111Q+S123P+Q137K+Q147K+V159M+K169R+G237M+S256Q+I294E+P339S+S402Q+K488T+A129T, S76E+Q82E+P111Q+S123P+V159M+V203T+G237M+S256E+I294E+S474E+E489R+P4920+A129T,P111Q+S123P+Q137K+Q147K+V159M+A238T+V251E+S256Q+Q271E+1294E+Q329E+S402Q+A129-T,A83E+P111Q+S123P+Q137K+Q147K+V159M+L184M+S256E+Q271D+1294E+Q329E+S402-Q+A129T,Q82E+P111Q+S123P+Q137K+V159M+K169R+A189G+V203T+G237M+T244R+S256E+1294E+A129T,Q82E+P111Q+S123P+Q137K+V159M+K169R+V203T+G237TM+T244R+V251E+S256E+1294E+A129T,Q82E+P111Q+S123P+V159M+K169R+V203T+G237M+T244R+S256E+1294E+N383Q+V431E+A129T,S76E+Q82E+S94R+P111Q+S123+V159M+K169R+V203T+G237M+T244R+S256E+I294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:A83E+P111Q+S123P+Q137K+Q147K+V159M+L184M+V251E+S256Q+Q271E+I294E+Q329E+S402Q+A129T,A83E+P111Q+S123P+Q137K+Q147K+V159M+L184M+K252E+S256Q+Q271E+I294E+Q329E+S402Q+A129T,Q82E+P111Q+S123P+V159M+K169R+V203T+G237M+T244R+S256E+Q271E+I294E+Q329E+N383E+A129T,Q82E+P111Q+S123P+Q137K+N155D+V159M+K169R+V203T+G237M+T244R+S256E+I294E+K445E+A129T,K8E+A41L+S76E+Q82E+P111Q+S123P+V159M+K169R+V203T+G237M+T244R+S256E+I294E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:S76E+A83E+P111Q+S123P+Q137K+Q147K+V159M+L184M+V251E+S256Q+Q271E+I294E+Q329E+S402Q+A129T,A83E+P111Q+S123P+Q137K+Q147K+V159M+K169R+L184M+K252E+S256Q+Q271E+I294E+Q329E+S402Q+A129T,A83E+P111Q+S123P+Q137K+Q147K+V159M+L184M+V203T+A238S+S256E+Q271E+I294E+Q329E+S402Q+A129T,A83E+P111Q+S123P+Q137K+Q147K+V159M+L184M+V251E+S256Q+Q271E+I294E+Q329E+S402Q+V431E+A129T,A83E+P111Q+S123P+Q137K+Q147K+V159M+L184M+V251E+S256Q+Q271E+I294E+ Q329E+K347E+S402-Q+A129T,A83E+P111Q+S123P+Q137K+L152P+V159M+L184M+V251E+S256Q+Q271E+I294E+Q329E+S402Q+V431E+A129T,K8E+A83E+P111Q+S123P+Q137K+Q147K+V159M+L184M+V251E+S256Q+Q271E+1294E+Q329E+S402Q+A129T,A83E+P111Q+S123P+Q137K+Q147K+N155D+L184M+G237M+V251E+S256Q+Q271E+I294E+Q329E+S402Q+A129T,A41-L+A83E+P111Q+S123P+Q137K+Q147K+V159M+L184M+V251E+S256Q+Q271E+I294E+Q329E+S402Q+A129-T,A83E+P111Q+S123P+Q137K+Q147K+V159M+L184M+V251E+S256Q+Q271E+I294E+Q329E+N383Q+S402-Q+A129T,R20K+S76E+Q82E+P111Q+S123P+V159M+K169R+V203T+G237M+T244R+S256E+1294E+Q329E+P492D+A129T,S76E+Q82E+P111Q+S123P+V159M+K169R+V203T+G237M+T244R+S256E+I294E+Q329E+E489R+P492D+A129T of the polypeptide of SEQ ID NO: 2, wherein the variant has xyloglucanase activity and wherein said variant has, at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:A83E+P111Q+S123P+Q137K+Q147K+V159M+L184M+V219T+G237M+V251E+S256Q+Q2

71E+1294E+Q329E+S402Q+A129T,S76E+A83E+P111Q+S123P+Q137K+Q147K+V159M+L184M+V203T+A238S+S256E+Q271E+I294E+Q329E+S402Q+A129T,A83E+P111Q+S123P+Q137K+Q147K+V159M+L184M+D210H+T244R+K252E+S256Q+Q271E+1294E+Q329E+S402Q+A129T,A83E+P111Q+S123P+Q137K+Q147K+V159M+L184M+T244E+V251E+S256Q+Q271E+1294E+Q329E+S402Q+V431E+A129T,K8E+A83E+S94R+P111Q+S123P+Q137K+Q147K+V159M+L184M+V251E+S256Q+Q271E+1294E+Q329E+S402Q+A129T,A83E+P111Q+S123P+Q137K+Q147K+V159M+L184M+V251E+S256Q+Q271E+I294E+Q329E+S402Q+V431E+K445E+A129T,A83E+P111Q+S123P+Q137K+Q147K+V159M+L184M+D210H+K240F+V251E+S256Q+Q271E+I294E+Q329E+S402Q+A129T,A41L+A83E+P111Q+S123P+Q137K+Q147K+V159M+L184M+K240F+V251E+S256Q+Q271E+I294E+Q329E+S402Q+A129T,Q82E+P111Q+S123P+Q137K+Q147K+V159M+L184M+V203T+D210H+V251E+S256Q+Q271E+I294E+Q329E+S402 Q+A129T,A41-L+A83E+P111Q+S123P+Q137K+Q147K+V159M+L184M+V251E+S256Q+Q271 E+1294E+Q329E+N383Q+S402-Q+A129T,A83E+P111Q+S123P+Q137K+Q147K+V159M+L184M+T244R+W248V+V251E+S256Q+Q271E+1294E+Q329E+S402Q+A129T,A83E+P111Q+S123P+Q137K+Q147K+V159M+L184M+V203T+T244R+V251E+S256Q+Q271E+I294E+Q329E+S402Q+A129T,A83E+P111Q+S123P+Q137K+Q147K+V159M+L184M+V251E+S256Q+Q271E+R295K+N298D+Q329E+S402Q+L447M+A129T,A83E+P111Q+S123P+Q137K+Q147K+V159M+L184M+S256E+Q271E+I294E+Q329E+P339S+N383E+S402Q+V431E+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:A83E+P111Q+S123P+Q137K+Q147K+V159M+L184M+G237M+V251E+S256Q+Q271E+1294E+Q329E+S402Q+E489R+V505L+A129T,A83E+P111Q+S123P+Q137K+Q147K+V159M+L184M+K252E+S256Q+Q271E+1294E+Q329E+N383E+S402Q+V431E+A491V+A129T,A83E+P111Q+S123P+Q137K+Q147K+V159M+L184M+V203T+V251E+S256Q+Q271E+I294E+Q329E+N383Q+S402Q+V431E+A129T,A83E+P111Q+S123P+Q137K+Q147K+V159M+L184M+V219T+G237M+S256Q+Q271E+1294E+Q329E+S402Q+V431E+S474E+A129T,A83E+P111Q+5123P+Q137K+Q147K+V159M+L184M+V251E+S256Q+Q271E+I294E+Q329E+N383E+S402Q+V4 31E+P492D+A129-T,A83E+P111Q+S123P+Q137K+Q147K+V159M+L184M+A189G+V251E+S256Q+Q271E+1294E+Q329E+N383Q+S402Q+V431E+A129T,A83E+P111Q+S123P+Q137K+Q147K+V159M+L184M+V251E+S256Q+Q271D+1294E+Q329E+N383E+S402Q+V431E+A459 P+A129T,K8-R+A83E+P111Q+S123P+Q137K+Q147K+V159M+L184M+V251E+S256Q+Q271E+1294E+Q329E+S402Q+V431E+E489K+A129T,A83E+P111Q+S123P+Q137K+Q147K+V159M+L184M+V251E+S256Q+Q271E+I294E+Q329E+N383E+S402Q+V431E+A491V+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:A83E+P111Q+S123P+Q137K+Q147K+V159M+L184M+D210H+G237M+V251E+S256Q+Q271E+1294E+Q329E+S402Q+E489R+P492D+A129T,S76E+Q82E+P111Q+S123P+Q137K+Q147K+V159M+L184M+V203T+V251E+S256Q+Q271E+1294E+Q329E+N383Q+S402Q+V431E+A 129T,K8E+A41-L+A83E+P111Q+S123P+Q137K+Q147K+V159M+L184M+V251E+S256Q+Q271E+1294E+Q329E+K394R+S402Q+V431E+A129T,A83E+P111Q+S123P+Q137K+Q147K+V159M+L184M+V251E+S256Q+Q271E+I294E+Q329E+K347E+N383E+S402Q+V431E+A491V+A129T,K8E+A83E+P111Q+S123P+Q137K+Q147K+V159M+L184M+V251E+S256Q+Q271E+I294E+Q329E+N383E+S402Q+V431E+A491V+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations at positions corresponding to positions selected from a group consisting of:A83E+P111Q+S123P+Q137K+Q147K+V159M+L184M+V203T+K240F+V251E+S256Q+Q271E+I294E+Q329E+N383E+S402Q+V431E+A491V+A129T,S76E+A83E+P111Q+S123P+Q137K+Q147K+N155D+L184M+G237M+V251E+S256Q+Q271E+1294E+Q329E+N383E+S402Q+V 431E+A491V+A129T of the polypeptide of SEQ ID NO: 2, wherein the variant has xyloglucanase activity and wherein said variant has at least 85%, at least 90%, at least 91%, at least 92%, at least 93%, at least 94%, at least 95%, such as at least 96%, at least 97%, at

least 98%, or at least 99% sequence identity, but less than 100% sequence identity, to the polypeptide of SEQ ID NO: 1, SEQ ID NO: 2, or SEQ ID NO: 3.

In one aspect, a variant comprises alterations or combination of alterations selected from a group consisting of:

K394R,
S127D,
R211K,
S123P,
K488T,
S256Q,
K476R,
K217R,
Q271D,
S214Q,
L447M,
K482R,
K169R,
L152D,
R267K,
L152E,
D210R,
L152*,
R295K,
S127W,
E126P,
S127H,
T104G,
Q125K,
Q125P,
Q125S,
D395P,
G103V,
T104R,
Q125L,
A41E,
A41R,
Q125F,
S127L,
A226K,
A41L,
A226D,
A118K+S123P,
N155D,
Q137K,
N155E,
Q147K,
R276K,
V203T,
S94R,
K18E,
K252E,
V219T,
R267C+T427V,
Q243E,
K414E,
K445E,
R20K+S123P,
S123P+K206R,

R20K+S123P+R211K,
S123P+K347R,
S123P+K347R+K353R+D395P,
S123P+D395P,
S123P+S127D,
V159M,
K392E,
E489R+P492D,
Y503L+V505L,
Y503V+V505L,
L184M+V219A,
S123P+R211K+K217R+S256Q+K488T,
Q82E,
S76E,
A83E,
Q271E,
S256E,
I294E,
Q329E,
V431E,
R20K+Y105E+S123P+Q147K+R267K,
R20K+Y105E+S123P+R267K,
R20K+S123P+K220R,
R20K+Y105E+S123P+N136D,
R20K+S123P+Q137K+Q147K,
Y105E+A118K+S123P+K206R+K220R+R267K,
P111Q+S123P+V159M,
S94R+P111Q+S123P+V159M,
K87E+P111Q+S123P+V159M+S402Q,
K87E+P111Q+S123P+V159M,
P111Q+S123P+V159M+S402Q,
K87E+P111Q+V159M+I294Q+I473T,
K8E+P111Q+V159M,
K8E+K18E+P111Q+V159M+K206E,
R20K+P111Q+S123P+S127D+V159M,
P111Q+Q147K+V159M+K220R,
S94R+P111Q+V159M,
P111Q+V159M+K206E+I294Q,
P111Q+V159M+K206E+I294Q+K347E,
P111Q+Q137K+Q147K+V159M+K252E,
P111Q+Q137K+Q147K+N155D+K252E,
P111Q+Q137K+L152D+V203T+K217R,
P111Q+Q137K+V159M,
K8E+P111Q+N155D+V159M,
A41L+P111Q+S123P+Q147K+V159M+V203T,
A41L+P111Q+S123P+Q147K+V159M+K217R,
P111Q+S123P+Q137K+Q147K+V159M+S256Q+S402Q,
P111Q+S123P+Q147K+V159M+I294Q+S402Q,
A41L+P111Q+Q137K+V159M+N168R+Q271D+K488T,
P111Q+Q137K+Q147K+V159M+V203T+K217R,
P111Q+Q147K+V159M,
P111Q+Q137K+Q147K+V159M,
P111Q+S123P+Q137K+V159M+K488T,
P111Q+S123P+Q137K+V159M+S256Q,
K87E+P111Q+L152D+V159M+V203T+I294Q,
K87E+P111Q+Q147K+L1520+V159M,
R20K+A83E+S123P+K220R+S256E,
R20K+S76E+A83E+S123P+K220R+K252E,

R20K+A83E+S123P+K220R+K252E,
R20K+A42V+S123P+K220R+K252E+I294E,
R20K+Q82E+S123P+Q147K+K220R,
R20K+A83E+S123P+K220R+S256Q,
R20K+Q82E+S123P+N155D+K220R,
R20K+S123P+V203T+K220R+K252E,
R20K+S123P+K220R+I1294E,
R20K+A83E+S123P+V203T+K220R+K252E,
R20K+A41L+Q82E+S123P+K220R,
R20K+S123P+N155D+K220R,
R20K+A42V+S76E+S123P+K220R,
R20K+S123P+V203T+V219T,
R20K+A42V+A83E+S123P+K220R,
R20K+A42V+S76E+S123P+V203T+K220R,
A83E+P111 Q+S123P+V159M+S256E+I294E,
Q82E+P111Q+S123P+V159M+S256E+I294E,
Q82E+P111Q+S123P+Q147K+V159M+I294E,
S76E+Q82E+K87E+P111Q+S123P+V159M+V203T,
A83E+P111Q+S123P+Q137K+Q147K+V159M+S256Q+S402Q,
P111Q+S123P+Q137K+Q147K+N155D+S256Q+A289T+N302H,
S76E+P111Q+S123P+Q137E+Q147K+V159M+K252E+S256Q+S402Q,
P111Q+S123P+Q137K+Q147K+V159M+K252E+S256Q+S402Q,
P111Q+S123P+Q137K+Q147K+N155D+S256Q+I294E+S402Q,
Q82E+P111Q+S123P+Q137K+Q147K+V159M+L184M+A238S+S5256Q+I1294E+S402Q,
A83E+P111Q+S123P+Q137K+Q147K+V159M+L184M+S256Q+I294E+S402Q,
P111Q+N121E+Q137K+Q147K+V159M+K169R+S256Q+I294E+S402Q,
P111Q+S123P+Q137K+Q147K+V159M+K169R+S256Q+I294E+S402Q,
P111Q+S123P+Q137K+Q147K+V159M+L184M+V219T+S256Q+S402Q,
A83E+P111Q+S123P+Q137K+Q147K+V159M+L184M+S256Q+S402Q,
Q82E+P111Q+S123P+Q137K+Q147K+V159M+K252E+S256Q+S402Q,
S76E+P111Q+S123P+Q137K+Q147K+V159M+S256Q+S402Q,
K8R+P111Q+S123P+Q137K+Q147K+V159M+S256Q+S402Q,
S123P+S127D+N136D+Q137K+Q147K+L152E+N153E+N155E,
S123P+S127D+N136D+Q137K+Q147K+L152E+N153E+N155E+A491E,
R20K+S123P+K169R+K217T+K240F+S256Q+R267H+I294E,
K87E+P111Q+S123P+V159M+K217T+I294E,
A83E+P111Q+S123P+V159M+K240F+K252E,
A83E+P111Q+S123P+V159M+K252E,
K87E+P111Q+S123P+V159M+S256Q+I294E,
K87E+P111Q+S123P+V159M+K347E+N383E,
Q82E+P111Q+S123P+V159M+V203T+G237M+S256E+I294E,
Q82E+P111Q+S123P+N155D+S256E+I294E,
Q82E+P111Q+S123P+N155D+K169R+G237M+S256E+I294E,
Q82E+P111Q+S123P+Q147K+V159M+S256E+I294E,
Q82E+P111Q+S123P+Q137K+V159M+G237M+S256E+I294E,
Q82E+P111Q+S123P+Q137K+V159M+K240F+S256E+I294E,
Q82E+P111Q+S123P+Q147K+N155D+K240F+S256E+I294E,
K8E+Q82E+P111Q+S123P+V159M+S256E+I294E,
A83E+P111Q+S123P+Q147K+V159M+S256E+I294E+Q329E,
Q82E+P111 Q+S123P+V159M+K240F+S256EI294E,
Q82E+P111Q+S123P+V159M+A177G+K240F+S256E+I294E+D384G,
K8R+Q82E+P111Q+S123P+V159M+K169R+K240F+S256E+I294E,
Q82E+P111Q+S123P+V159M+K169R+S256E+I294E+V431E,
Q82E+P111Q+S123P+V159M+G237M+V251E+I294E,
A41L+Q82E+P111Q+S123P+V159M+S256E+I294E+N383E,
A83E+P111Q+S123P+Q137K+F1460+Q147G+L48P+V159M+L184M+S256Q+I294E+ S402Q,
A83E+P111Q+S123P+Q137K+Q147K+V159M+L184M+S256Q+I294E+N298D+V300L+ S402Q,
A83E+P111Q+S123P+Q137K+Q147K+V159M+K169R+L184M+K240L+S256Q+I294E+ S402Q,

A83E+P111Q+S123P+Q137K+Q147K+V159M+L184M+G237M+S256Q+I294E+S402Q +K488T,
A83E+P111Q+S123P+Q137K+Q147K+N155D+L184M+G237M+S256Q+I294E+S402Q,
A83E+P111Q+S123P+Q137K+Q147K+V159M+L184M+V203T+K240F+S256Q+I294E+ S402Q,
A83E+P111Q+S123P+Q137K+Q147K+V159M+L184M+G237M+S256E+I294E+S402Q,
A83E+P111Q+S123P+Q137K+Q147K+V159M+L184M+K240F+S256Q+I294E+S402Q+ E489R,
A83E+P111Q+S123P+Q137K+Q147K+V159M+F165H+L184M+S256Q+I294E+S402Q +V431E,
A83E+P111Q+S123P+Q137K+Q147K+V159M+L184M+V251E+Q271E+I294E+Q329E +S402Q,
A83E+P111Q+S123P+Q137K+Q147K+V159M+L184M+V251E+S256Q+Q271E+I294E +Q329E+S402Q,
A83E+P111Q+S123P+Q137K+Q147K+N155D+L184M+S256Q+I294E+S402Q,
R20K+S123P+Q147K+N155D+K169R+K217T+K240F+S256Q+R267H+I294E,
R20K+S123P+Q137K+K169R+K217T+K240F+S256Q+R267H+I294E,
R20K+S123P+N155D+K169R+K217T+K240F+S256Q+R267H+I294E+D384G+E489R,
R20K+S123P+N155D+K169R+K217T+K240F+S256Q+R267H+I294E+S474E,
R20K+S123P+N155D+K169R+K217T+K240F+S256Q+R267H+I294E,
R20K+S123P+Q147K+K169R+V219T+K240F+S256Q+R267H+I294E,
R20K+A41L+S123P+L152P+K169R+K217T+K240F+S256Q+R267H+I294E,
K8R+R20K+S123P+K169R+D210H+K217T+K240F+S256Q+R267H+I294E,
R20K+S123P+K169R+K217T+K240F+S256Q+R267H+I294E+Q329E+V431E+E489R,
P111Q+S123P+Q137K+Q147K+V159M+K169R+D210H+K240F+S256E+I294E+S402Q +K488T,
P111Q+S123P+Q137K+Q147K+V159M+K169R+K252E+S256Q+I294E+K322E+S402 Q,
P111Q+S123P+Q137K+Q147K+V159M+K169R+K240F+S256Q+I294E+K322E+S402Q
S76E+P111Q+S123P+Q137K+Q147K+V159M+K169R+G237M+S256Q+I294E+S402Q
S76E+P111Q+S123P+Q137K+Q147K+V159M+K169R+V203T+G237M+S256Q+I294E +S402Q,
P111Q+S123P+Q137K+Q147K+V159M+K169R+G237M+S256Q+I294E+P3395+S402 Q+K488T,
P111Q+S123P+Q137K+Q147K+V159M+K169R+G237M+T244R+S256Q+1294E+S402 Q,
P111Q+S123P+Q137K+Q147K+V159M+G237M+T244R+S256Q+I294E+S402Q,
P111Q+S123P+Q137K+Q147K+V159M+K169R+V219T+K240F+S256Q+I294E+S402Q
P111Q+S123P+Q137K+Q147K+V159M+K169R+V203T+S256Q+I294E+S402Q,
Q82E+P111Q+S123P+Q137K+V159M+V203T+D210H+G237M+S256E+I294E,
Q82E+P111Q+S123P+V159M+V203T+G237M+K252E+S256E+I294E,
S76E+Q82E+P111Q+S123P+V159M+V203T+G237M+S256E+I294E,
Q82E+P111Q+S123P+Q147K+V159M+V203T+G237M+S256E+I294E,
S76E+Q82E+P111Q+S123P+V159M+V203T+G237M+S256E+I294E+S474E+E489R+ P492D,
Q82E+P111Q+S123P+V159M+K169R+V203T+G237M+T244R+S256E+I294E,
Q82E+P111Q+S123P+V159M+V203T+G237M+T244R+S256E+I294E,
S76E+A83E+P111Q+S123P+Q137K+Q147K+V159M+L184M+V251E+S256Q+Q271E+ I294E+Q329E+S402Q,
A83E+P111Q+S123P+Q137K+Q147K+V159M+L184M+V219T+G237M+V251E+S256Q +Q271E+I294E+Q329E+S402Q,
A83E+P111Q+S123P+Q137K+Q147K+V159M+K169R+L184M+K252E+S256Q+Q271E +I294E+Q329E+S402Q,
S76E+A83E+P111Q+S123P+Q137K+Q147K+V159M+L184M+V203T+A238S+S256E+ Q271E+I294E+Q329E+S402Q,
A83E+P111Q+S123P+Q137K+Q147K+V159M+L184M+V203T+A238S+S256E+Q271E +I294E+Q329E+S402Q,
A83E+P111Q+S123P+Q137K+Q147K+V159M+L184M+G237M+V251E+S256Q+Q271 E+I294E+Q329E+S402-Q+E489R+V505L,
A83E+P111Q+S123P+Q137K+Q147K+V159M+L184M+D210H+G237M+V251E+S256 Q+Q271E+ 1294E+Q329E+S402Q+ E489R+ P4920,
A83E+P111Q+S123P+Q137K+Q147K+V159M+L184M+D210H+T244R+K252E+S256Q +Q271E+I294E+Q329E+S402Q,
A83E+P111Q+S123P+Q137K+Q147K+V159M+L184M+V251E+S256Q+Q271E+I294E +Q329E+S402Q+V431E,
A83E+P111Q+S123P+Q137K+Q147K+V159M+L184M+T244E+V251E+S256Q+Q271E +I294E+Q329E+S402Q+V431E,
A83E+P111Q+S123P+Q137K+Q147K+V159M+L184M+V251E+S256Q+Q271E+I294E +Q329E+K347E+S402Q,
K8E+A83E+S94R+P111Q+S123P+Q137K+Q147K+V159M+L184M+V251E+S256Q+Q 271E+I294E+Q329E+S402Q,
A83E+P111Q+S123P+Q137K+L152P+V159M+L184M+V251E+S256Q+Q271E+I294E+ Q329E+S402Q+V431E,
A83E+P111Q+S123P+Q137K+Q147K+V159M+L184M+V251E+S256Q+Q271E+I294E +Q329E+S402Q+V431E+K445E,
A83E+P111Q+S123P+Q137K+Q147K+V159M+L184M+K252E+S256Q+Q271E+I294E +Q329E+S402Q,

K8E+A83E+P111Q+S123P+Q137K+Q147K+V159M+L184M+V251E+S256Q+Q271E+I 294E+Q329E+S402Q,
A83E+P111Q+S123P+Q137K+Q147K+V159M+L184M+D210H+K240F+V251E+S256Q +Q271E+I294E+Q329E+S402Q,
A83E+P111Q+S123P+Q137K+Q147K+N155D+L184M+G237M+V251E+S256Q+Q271 E+I294E+Q329E+S402Q,
A41L+A83E+P111Q+S123P+Q137K+Q147K+V159M+L184M+K240F+V251E+S256Q+ Q271E+I294E+Q329E+S402Q,
Q82E+P111Q+S123P+Q137K+Q147K+V159M+L184M+V203T+D210H+V251E+S256Q +Q271E+I294E+Q329E+S402Q,
A41L+A83E+P111Q+S123P+Q137K+Q147K+V159M+L184M+V251E+S256Q+Q271E+ I294E+Q329E+S402Q,
A41L+A83E+P111Q+S123P+Q137K+Q147K+V159M+L184M+V251E+S256Q+Q271E+ I294E+Q329E+N383Q+S402Q,
P111Q+S123P+Q137K+Q147K+V159M+V251E+S256E+Q271E+I294E+Q329E+S402 Q,
P111Q+S123P+Q137K+Q147K+V159M+A238T+V251E+S256Q+Q271E+I294E+Q329 E+S402Q,
A83E+P111Q+S123P+Q137K+Q147K+V159M+L184M+V251E+S256Q+Q271E+I294E +Q329E+N383Q+S402Q,
A83E+P111Q+S123P+Q137K+Q147K+V159M+L184M+T244R+W248V+V251E+S256 Q+Q271E+I294E+Q329E+S402Q,
A83E+P111Q+S123P+Q137K+Q147K+V159M+L184M+V203T+T244R+V251E+S256Q +Q271E+I294E+Q329E+S402Q,
A83E+P111Q+S123P+Q137K+Q147K+V159M+L184M+V251E+S256Q+Q271E+R295K +N298D+Q329E+S402Q+L447M,
A83E+P111Q+S123P+Q137K+Q147K+V159M+L184M+S256E+Q271D+I294E+Q329E +S402Q,
Q82E+P111Q+S123P+V159M+K169R+V203T+G237M+T244R+S256E+Q271E+I294E +Q329E+N383E,
Q82E+P111Q+S123P+Q137K+V159M+K169R+A189G+V203T+G237M+T244R+S256E +I294E,
Q82E+P111Q+S123P+Q137K+V159M+K169R+V203T+G237M+T244R+V251E+S256E +I294E,
Q82E+P111Q+S123P+L152P+V159M+K169R+V203T+G237M+T244R+S256E+I294E,
Q82E+P111Q+S123P+V159M+K169R+V203T+G237M+T244R+S256E+I294E+N383Q +V431E,
R20K+S76E+Q82E+P111Q+S123P+V159M+K169R+V203T+G237M+T244R+S256E+1 294E+Q329E+P492D,
S76E+Q82E+P111Q+S123P+V159M+K169R+V203T+G237M+T244R+S256E+I294E+ Q329E+E489R+P492D,
Q82E+P111Q+S123P+Q137K+N155D+V159M+K169R+V203T+G237M+T244R+S256E +I294E+K445E,
S76E+Q82E+S94R+P111Q+S123P+V159M+K169R+V203T+G237M+T244R+S256E+I 294E,
K8E+A41L+S76E+Q82E+P111Q+S123P+V159M+K169R+V203T+G237M+T244R+S25 6E+I294E,
A83E+P111Q+S123P+Q137K+Q147K+V159M+L184M+S256E+Q271E+I294E+Q329E +P339S+N383E+S402Q+V431E,
S76E+Q82E+P111Q+S123P+Q137K+Q147K+V159M+L184M+V203T+V251E+S256Q+ Q271E+1294E+Q329E+N383Q+S402Q+V431E,
A83E+P111Q+S123P+Q137K+Q147K+V159M+L184M+K252E+S256Q+Q271E+I294E +Q329E+N383- E+S402Q+V431E+A491V,
A83E+P111Q+S123P+Q137K+Q147K+V159M+L184M+V203T+V251E+S256Q+Q271E +I294E+Q329E+N383Q+S402Q+V431E,
A83E+P111Q+S123P+Q137K+Q147K+V159M+L184M+V203T+K240F+V251E+S256Q +Q271E+I294E+Q329E+N383E+S402Q+V431E+A491V,
A83E+P111Q+S123P+Q137K+Q147K+V159M+L184M+V219T+G237M+S256Q+Q271 E+I294E+Q329E+S402Q+V431E+S474E,
A83E+P111Q+S123P+Q137K+Q147K+V159M+L184M+V251E+S256Q+Q271E+I294E +Q329E+N383- E+S402Q+V431E+P492D,
S76E+A83E+P111Q+S123P+Q137K+Q147K+N155D+L184M+G237M+V251E+S256Q+ Q271E+I294E+Q329E+N383E+S402Q+V431E+A491V,
A83E+P111Q+S123P+Q137K+Q147K+V159M+L184M+A189G+V251E+S256Q+Q271E +I294E+Q329E+N383Q+S402Q+V431E,
K8E+A41L+A83E+P111Q+S123P+Q137K+Q147K+V159M+L184M+V251E+S256Q+Q2 71E+1294E+Q329E+K394R+S402Q+V431E,
A83E+P111Q+S123P+Q137K+Q147K+V159M+L184M+V251E+S256Q+Q271D+I294E +Q329E+N383- E+S402Q+V431E+A459P,
A83E+P111Q+S123P+Q137K+Q147K+V159M+L184M+V251E+S256Q+Q271E+I294E +Q329E+K347E+N383E+S402Q+V431E+A491V,
K8E+A83E+P111Q+S123P+Q137K+Q147K+V159M+L184M+V251E+S256Q+Q271E+I

294E+Q329E+N383E+S402Q+V431E+A491V, K8R+A83E+P111Q+S123P+Q137K+Q147K+V159M+L184M+V251E+S256Q+Q271E+I294E+Q329E+S402Q+V431E+E489K, A83E+P111Q+S123P+Q137K+Q147K+V159M+L184M+V251E+S256Q+Q271E+I294E +Q329E+N383E+S402Q+V431E+A491V of the polypeptide of SEQ ID NO: 1, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity, but less than 100% sequence identity, to the polypeptide of SEQ ID NO: 1, SEQ ID NO: 2, or SEQ ID NO: 3.

The variants may further comprise a substitution at one or more other positions, such as those described in WO 2009/147210.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for xyloglucanase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide and are described, e.g., in WO 2009/147210.

The variants may consist of 445 to 524 amino acids, *e.g.*, 471 to 524 amino acids, 497 to 524 amino acids.

In an embodiment, the variant has improved wash performance compared to the parent enzyme.

In an embodiment, the variant has improved enzyme detergency benefit compared to the parent enzyme.

In an embodiment, the variant has improved stability compared to the parent enzyme.

In an embodiment, the variant has improved thermostability compared to the parent enzyme.

In an embodiment, the variant has improved in-detergent storage stability compared to the parent enzyme.

In an embodiment, the variant has improved whiteness compared to the parent enzyme.

## Parent Xyloglucanases

The parent xyloglucanase may be a polypeptide having at least 60% sequence identity to the polypeptide of SEQ ID NO: 1.

In an aspect, the parent has a sequence identity to the polypeptide of SEQ ID NO: 1 of at least 60%, *e.g.*, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have xyloglucanase activity. In one aspect, the amino acid sequence of the parent differs by up to 10 amino acids, *e.g.*, 1, 2, 3, 4, 5, 6, 7, 8, 9, or 10, from the polypeptide of SEQ ID NO: 1. In another aspect, the parent comprises or consists of the amino acid sequence of SEQ ID NO: 1. In another aspect, the parent is a fragment of the polypeptide of SEQ ID NO: 1 containing at least 445 amino acid residues, *e.g.*, at least 471 and at least 497 amino acid residues.

In an aspect, the parent has a sequence identity to the polypeptide of SEQ ID NO: 2 of at least 60%, *e.g.*, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have xyloglucanase activity. In

one aspect, the amino acid sequence of the parent differs by up to 10 amino acids, e.g., 1, 2, 3, 4, 5, 6, 7, 8, 9, or 10, from the polypeptide of SEQ ID NO: 2. In another aspect, the parent comprises or consists of the amino acid sequence of SEQ ID NO: 2. In another aspect, the parent is a fragment of the polypeptide of SEQ ID NO: 2 containing at least 445 amino acid residues, *e.g.*, at least 471 and at least 497 amino acid residues.

In an aspect, the parent has a sequence identity to the polypeptide of SEQ ID NO: 3 of at least 60%, *e.g.*, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have xyloglucanase activity. In one aspect, the amino acid sequence of the parent differs by up to 10 amino acids, e.g., 1, 2, 3, 4, 5, 6, 7, 8, 9, or 10, from the polypeptide of SEQ ID NO: 3. In another aspect, the parent comprises or consists of the amino acid sequence of SEQ ID NO: 3. In another aspect, the parent is a fragment of the polypeptide of SEQ ID NO: 3 containing at least 445 amino acid residues, *e.g.*, at least 471 and at least 497 amino acid residues.

The parent polypeptide may be a hybrid polypeptide in which a region of one polypeptide is fused at the N-terminus or the C-terminus of a region of another polypeptide.

The parent may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

The parent may be obtained from microorganisms of any genus. For purposes of the present invention, the term "obtained from" as used herein in connection with a given source shall mean that the parent encoded by a polynucleotide is produced by the source or by a strain in which the polynucleotide from the source has been inserted. In one aspect, the parent is secreted extracellularly.

In a further aspect the parent xyloglucanase may be a bacterial xyloglucanase. For example, the xyloglucanase may be a Gram positive bacterial polypeptide such as a *Bacillus,* preferably from the Bacillus/Lactobacillus subdivision, preferably a species from the genus *Paenibacillus,* especially *Paenibacillus polymyxa,* e.g. *Paenibacillus polymyxa,* ATCC 832, preferably the xyloglucanase is a family 44 xylogluclanase, e.g. as described in WO 01/62903.

It will be understood that for the aforementioned species, the invention encompasses both the perfect and imperfect states, and other taxonomic equivalents, *e.g.*, anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The parent may be identified and obtained from other sources including microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding a parent may then be obtained by similarly screening a genomic DNA or cDNA library of another microorganism or mixed DNA sample. Once a polynucleotide encoding a parent has been detected with the probe(s), the polynucleotide can be isolated or cloned by utilizing techniques that are known to those of ordinary skill in the art (see, *e.g.,* Sambrook *et al.,* 1989, *supra).*

**Preparation of Variants**

The present invention also relates to methods for obtaining a variant having xyloglucanase activity, comprising: (a) introducing into a parent xyloglucanase alteration at one or more positions corresponding to positions selected from the group consisting of 111, 123, 159, 256, 294, 8, 18, 20, 41, 42, 76, 82, 83, 87, 94, 103, 104, 105, 121, 125, 126, 127, 136, 137, 146, 147, 148, 152, 153, 155, 165, 168, 169, 177, 184, 189, 203, 206, 210, 211, 214, 217, 219, 220, 226, 237, 238, 240, 243, 244, 248, 251, 252, 267, 271, 276, 289, 295, 298, 300, 302, 322, 329, 339, 347, 347, 353, 383, 384, 392, 394, 395, 402, 414, 427, 431, 445, 447, 459, 473, 474, 476, 482, 488, 489, 491, 492, 503 and 505 of the polypeptide of SEQ ID NO: 1,

wherein the variant has xyloglucanase activity; and (b) recovering the variant.

The variants can be prepared using any mutagenesis procedure known in the art, such as site-directed mutagenesis, synthetic gene construction, semi-synthetic gene construction, random mutagenesis, shuffling, etc.

Site-directed mutagenesis is a technique in which one or more (*e.g.*, several) mutations are introduced at one or more defined sites in a polynucleotide encoding the parent.

Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, *e.g.,* Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.,* U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide subsequences may then be shuffled.

## Polynucleotides

The present invention also relates to polynucleotides encoding a variant of the present invention.

## Nucleic Acid Constructs

The present invention also relates to nucleic acid constructs comprising a polynucleotide encoding a variant of the present invention operably linked to one or more control sequences that direct the expression of the coding sequence in a suitable host cell under conditions compatible with the control sequences.

The polynucleotide may be manipulated in a variety of ways to provide for expression of a variant. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide which is recognized by a host cell for expression of the polynucleotide. The promoter contains transcriptional control sequences that mediate the expression of the variant. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus*

*licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene *(sacB), Bacillus subtilis xylA* and *xylB* genes, *Bacillus thuringiensis crylllA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger or Aspergillus awamori* glucoamylase *(glaA), Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator sequence is operably linked to the 3'-terminus of the polynucleotide encoding the variant. Any terminator that is functional in the host cell may be used.

Preferred terminators for bacterial host cells are obtained from the genes for *Bacillus clausii* alkaline protease (*aprH*), *Bacillus licheniformis* alpha-amylase *(amyL),* and *Escherichia coli* ribosomal RNA (*rrnB*).

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader sequence is operably linked to the 5'-terminus of the polynucleotide encoding the variant. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the variant-encoding sequence and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular

Biol. 15: 5983-5990.

The control sequence may also be a signal peptide coding region that encodes a signal peptide linked to the N-terminus of a variant and directs the variant into the cell's secretory pathway. The 5'-end of the coding sequence of the polynucleotide may inherently contain a signal peptide coding sequence naturally linked in translation reading frame with the segment of the coding sequence that encodes the variant. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the variant. However, any signal peptide coding sequence that directs the expressed variant into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases *(nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus* niger glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a variant. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of the variant and the signal peptide sequence is positioned next to the N-terminus of the propeptide sequence.

It may also be desirable to add regulatory sequences that regulate expression of the variant relative to the growth of the host cell. Examples of regulatory systems are those that cause expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the variant would be operably linked with the regulatory sequence.

## Expression Vectors

The present invention also relates to recombinant expression vectors comprising a polynucleotide encoding a variant of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the variant at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene.

The vector preferably contains an element(s) that permits integration of the vector into the host cell's genome or autonomous replication of the vector in the cell independent of the genome.

For integration into the host cell genome, the vector may rely on the polynucleotide's sequence encoding the variant or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a variant. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook *et al.,* 1989, *supra).*

## Host Cells

The present invention also relates to recombinant host cells, comprising a polynucleotide encoding a variant of the present invention operably linked to one or more control sequences that direct the production of a variant of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the variant and its source.

The host cell may be any cell useful in the recombinant production of a variant, *e.g.*, a prokaryote or a eukaryote.

The prokaryotic host cell may be any Gram-positive or Gram-negative bacterium. Gram-positive bacteria

include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell, including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397), or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804) or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium hetero-*

*sporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

The present invention also relates to methods of producing a variant, comprising: (a) cultivating a recombinant host cell of the present invention under conditions suitable for expression of the variant; and optionally (b) recovering the variant.

The recombinant host cells are cultivated in a nutrient medium suitable for production of the variant using methods known in the art. For example, the cell may be cultivated by shake flask cultivation, or small-scale or large-scale fermentation (including continuous, batch, fed-batch, or solid-state fermentations) in laboratory or industrial fermentors performed in a suitable medium and under conditions allowing the variant to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the variant is secreted into the nutrient medium, the variant can be recovered directly from the medium. If the variant is not secreted, it can be recovered from cell lysates.

The variant may be detected using methods known in the art that are specific for the variants. These detection methods include, but are not limited to, use of specific antibodies, formation of an enzyme product, or disappearance of an enzyme substrate. For example, an enzyme assay may be used to determine the activity of the variant.

The variant may be recovered using methods known in the art. For example, the variant may be recovered from the nutrient medium by conventional procedures including, but not limited to, collection, centrifugation, filtration, extraction, spray-drying, evaporation, or precipitation.

The variant may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, Janson and Ryden, editors, VCH Publishers, New York, 1989) to obtain substantially pure variants.

In an alternative aspect, the variant is not recovered, but rather a host cell of the present invention expressing the variant is used as a source of the variant.

## Fermentation Broth Formulations or Cell Compositions

The present invention also relates to a fermentation broth formulation or a cell composition comprising a variant of the present invention. The fermentation broth product further comprises additional ingredients used in the fermentation process, such as, for example, cells (including, the host cells containing the gene encoding the variant of the present invention which are used to produce the variant of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. In some embodiments, the fermentation broth contains

spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compositions may further comprise a preservative and/or anti-microbial (*e.g.*, bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.*, filamentous fungal cells) are grown to saturation, incubated under carbon-limiting conditions to allow protein synthesis. In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

**Compositions**

In one embodiment, the invention is directed to cleaning compositions, such as detergent compositions comprising a xyloglucanase variant of the present invention in combination with one or more additional cleaning composition components and preferably a detergent adjunct ingredient as described herein. The choice of additional components is within the skill of the artisan and includes conventional ingredients, including the exemplary non-limiting components set forth below. The detergent compositions can be used for cleaning an item to be cleaned, such as textiles, dishes, and hard surfaces, for pretreating stains on the item, for preventing, reducing, or removing redeposition of soil during a wash cycle, for maintaining or improving the whiteness of an item.

Preferably, the compositions are enriched in such a variant. The term "enriched" indicates that the xyloglucanase activity of the composition has been increased, *e.g.*, with an enrichment factor of at least 1.1.

The compositions may comprise a variant of the present invention as the major enzymatic component, *e.g.*, a mono-component composition. Alternatively, the compositions may comprise multiple enzymatic activities, such as one or more enzymes selected from the group consisting of hydrolase, isomerase, ligase, lyase, oxidoreductase, or transferase, *e.g.*, an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, nuclease, oxidase, pectinolytic enzyme, peroxidase, phytase, polyphenoloxidase, proteolytic enzyme, ribonuclease, transglutaminase, or xylanase.

The compositions may be prepared in accordance with methods known in the art and may be in the form of a liquid or a dry composition. The compositions may be stabilized in accordance with methods known in the art.

Examples are given below of preferred uses of the compositions of the present invention. The dosage of the composition and other conditions under which the composition is used may be determined on the basis of methods known in the art.

The choice of components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

In one embodiment of the invention, the composition, such as a detergent composition comprises a xyloglucanase variant as disclosed herein and a detergent adjunct.

In one embodiment of the invention, the detergent adjunct ingredient is selected from the group consisting of

surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, and/or pigments.

The detergent adjunct ingredient may be a surfactant. One advantage of including a surfactant in a detergent composition comprising a xyloglucanase variant is that the wash performance is improved. In one embodiment, the detergent adjunct ingredient is a builder or a clay soil removal/anti-redeposition agent.

In one embodiment, detergent adjunct ingredient is an enzyme. The detergent composition may comprise one or more enzymes, as specified below. The one or more enzymes may be selected from the group consisting of proteases, lipases, cutinases, amylases, carbohydrases, cellulases, pectinases, mannanases, arabinases, galactanases, xylanases, nucleases and oxidases. Specific enzymes suitable for the detergent compositions of the invention are described below.

The detergent composition may be formulated as a bar, a homogenous tablet, and a tablet having two or more layers, a pouch having one or more compartments, a regular or compact powder, a granule, a paste, a gel, or a regular, compact or concentrated liquid. The detergent composition can be a liquid detergent, a powder detergent or a granule detergent.

The xyloglucanases of the invention are suitable for use in cleaning such as laundry. The invention further relates a method for laundering an item, which method comprises the steps of:

a. exposing an item to a wash liquor comprising a variant polypeptide having xyloglucanase activity or a detergent composition comprising the polypeptide;
b. completing at least one wash cycle; and
c. optionally rinsing the item,

wherein the item is a textile.

The pH of the liquid solution is in the range of 1 to 11, such as in the range 5.5 to 11, such as in the range of 7 to 9, in the range of 7 to 8 or in the range of 7 to 8.5.

The wash liquor may have a temperature in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C. In one embodiment the temperature of the wash liquor is 30°C.

In one embodiment of the invention, the method for laundering an item further comprises draining of the wash liquor or part of the wash liquor after completion of a wash cycle. The wash liquor can then be re-used in a subsequent wash cycle or in a subsequent rinse cycle. The item may be exposed to the wash liquor during a first and optionally a second or a third wash cycle. In one embodiment the item is rinsed after being exposed to the wash liquor. The item can be rinsed with water or with water comprising a conditioner. The invention further concerns an item washed according to the inventive method. The xyloglucanase variants of the invention may be added to a wash liquor.

Thus, one embodiment of the invention relates to a detergent composition comprising one or more anionic surfactants; an enzyme selected from the group consisting of: a protease, a lipase, a cutinase, an amylase, a carbohydrase, a cellulase, a pectinase, a mannanase, an arabinase, a galactanase, a xylanase, a nuclease, and an oxidase; and a xyloglucanase variant of the invention.

One embodiment further relates to a washing method for textile comprising:

a. exposing a textile to a wash liquor comprising a xyloglucanase variant or a detergent composition comprising at least one of the xyloglucanase variants,
b. completing at least one wash cycle; and
c. optionally rinsing the textile,

wherein the xyloglucanase variant comprises a alteration at one or more positions corresponding to positions selected from the group consisting of 111, 123, 159, 256, 294, 8, 18, 20, 41, 42, 76, 82, 83, 87, 94, 103, 104, 105, 121, 125, 126, 127, 136, 137, 146, 147, 148, 152, 153, 155, 165, 168, 169, 177, 184, 189, 203, 206, 210, 211, 214, 217, 219, 220, 226, 237, 238, 240, 243, 244, 248, 251, 252, 267, 271, 276, 289, 295, 298, 300, 302, 322, 329, 339, 347, 347, 353, 383, 384, 392, 394, 395, 402, 414, 427, 431, 445, 447, 459, 473, 474, 476, 482, 488, 489, 491, 492, 503 and 505 of the polypeptide of SEQ ID NO: 1.

Another embodiment relates to a textile washed according to the inventive method.

The concentration of the xyloglucanase variant in the wash liquor is typically in the range of 0.00004-100 ppm enzyme protein, such as in the range of 0.00008-100, in the range of 0.0001-100, in the range of 0.0002-100, in the range of 0.0004-100, in the range of 0.0008-100, in the range of 0.001-100 ppm enzyme protein, 0.01-100 ppm enzyme protein,

preferably 0.05-50 ppm enzyme protein, more preferably 0.1-50 ppm enzyme protein, more preferably 0.1-30 ppm enzyme protein, more preferably 0.5-20 ppm enzyme protein, and most preferably 0.5-10 ppm enzyme protein.

The xyloglucanase variant of the present invention may be added to a detergent composition in an amount corresponding to at least 0.002 mg of xyloglucanase protein, such as at least 0.004 mg of xyloglucanase protein, at least 0.006 mg of xyloglucanase protein, at least 0.008 mg of xyloglucanase protein, at least 0.01 mg of xyloglucanase protein, at least 0.1 mg of protein, preferably at least 1 mg of protein, more preferably at least 10 mg of protein, even more preferably at least 15 mg of protein, most preferably at least 20 mg of protein, and even most preferably at least 25 mg of protein. Thus, the detergent composition may comprise at least 0.00008% xyloglucanase protein, preferably at least 0.002%, 0.003%, 0.004%, 0.005%, 0.006%, 0.008%, 0.01%, 0.02%, 0.03%, 0.05%, 0.1%, 0.2%, 0.3%, 0.4%, 0.6%, 0.7%, 0.8%, 0.9% or 1.0% of xyloglucanase protein.

Enzymes including present in a detergent of the invention may be stabilized using conventional stabilizing agents, e.g. a polyols such as propylene glycol or glycerol, a sugar or sugar alcohol and different salts such as NaCl and KCl. Proteases present in the detergent of the invention may be stabilized using lactic acid, formic acid, boric acid, or a boric acid derivative, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459) or a protease inhibitor of the protein type such as RASI, BASI, WASI (bifunctional alpha-amylase/subtilisin inhibitors of rice, barley and wheat) or CI2 or SSI. The composition may be formulated as described in e.g. WO 92/19709, WO 92/19708 and US 6,472,364. In some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), Tin (II), cobalt (II), copper (II), Nickel (II), and oxovanadium (IV)).

In one embodiment, the polypeptides are stabilized using peptide aldehydes or ketones Suitable peptide aldehydes are described in WO94/04651, WO95/25791, WO98/13458, WO98/13459, WO98/13460, WO98/13461, WO98/13462, WO07/141736, WO07/145963, WO09/118375, WO10/055052 and WO11/036153. A polypeptide of the present invention may also be incorporated in the detergent formulations disclosed in WO 97/07202.

In another embodiment, the polypeptides are stabilized using a phenyl boronic acid derivative is 4-formylphenylboronic acid (4-FPBA) with the following formula:

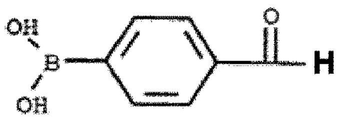

The detergent compositions may comprise two or more stabilizing agents e.g. such as those selected from the group consisting of propylene glycol, glycerol, 4-formylphenyl boronic acid and borate.

The detergent compositions may comprise two or more stabilizing agents e.g. such as those selected from the group consisting of propylene glycol, glycerol, 4-formylphenyl boronic acid and borate.

The stabilizing agent(s) is preferably present in the detergent composition in a quantity of from 0.001 to about 5.0 wt%, from 0.01 to about 2.0 wt%, from 0.1 to about 3 wt% or from 0.5% to about 1.5 wt%.

**Surfactants**

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 5% to 60% by weight, such as about 5% to about 50%, or about 10% to about 50%, or about 20% to about 50%. The surfactant(s) is chosen based on the desired cleaning application, and may include any conventional surfactant(s) known in the art.

When included therein the detergent will usually contain from about 5% to about 60% by weight of one or more anionic surfactants, such as from about 5% to about 40%, including from about 10% to about 25%, Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS),

paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salts of fatty acids (soap) or fatty acids, and combinations thereof.

When included therein the detergent will usually contain from about from about 0,1% to about 10% by weigh of a cationic surfactant, for example from about 0.1% to about 5%, Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 60% by weight of a nonionic surfactant, for example from about 1% to about 40%, in particular from about 5% to about 20%, from about 3% to about 15%, Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or *N*-acyl *N*-alkyl derivatives of glucosamine (glucamides, GA, or fatty acid glucamides, FAGA), methylester ethoxylates (MEE), as well as products available under the trade names SPAN and TWEEN, and combinations thereof.

When included therein the detergent will usually contain from about 0,1% to about 10% by weight of a semipolar surfactant. Non-limiting examples of semipolar surfactants include amine oxides (AO) such as alkyldimethylamineoxide, *N*-(coco alkyl)-*N*,*N*-dimethylamine oxide and N-(tallow-alkyl)-*N,N*-bis(2-hydroxyethyl)amine oxide, and combinations thereof.

When included therein the detergent will usually contain from about 0,1% to about 10% by weight of a zwitterionic surfactant. Non-limiting examples of zwitterionic surfactants include betaines such as alkyldimethylbetaines, sulfobetaines, and combinations thereof.

Solvent system: For dissolution of the surfactant and other detergent ingredients, a solvent system is needed. Solvents are typically water, alcohols, polyols, sugars and/or mixtures thereof. Preferred solvents are water, glycerol, sorbitol, propylene glycol (MPG, 1,2-propanediol or 1,3-propane diol), dipropylene glycol (DPG), polyethylene glycol family (PEG300-600), hexylene glycol, inositol, mannitol, Ethanol, isopropanol, n-butoxy propoxy propanol, ethanolamines (monoethanol amine, diethanol amines and triethanol amines), sucrose, dextrose, glucose, ribose, xylose, and related mono and di pyranosides and furanosides.

The solvent system is present in typically totally 5-90%, 5-60%, 5-40%, 10-30% by weight.

The water content for unit doses wrapped in PVA film is typically in the range 1-15%, 2-12%, 3-10%, 5-10%.

The polyol content for unit doses wrapped in PVA film is typically in the range 5-50%, 10-40% or 20-30%.

In an embodiment, the surfactant is a non-naturally occurring surfactant.

### Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants), however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming micellar, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

**Builders and Co-Builders**

The detergent composition may contain about 0-65%, 0-20%; or 0.5-5% of a detergent builder or co-builder, or a mixture thereof. In a dish wash detergent, the level of builder is typically 10--65%, particularly 20-40%. The builder and/or co-builder may particularly be a chelating agent that forms water-soluble complexes with Ca and Mg. Any builder and/or co-builder known in the art for use in laundry detergents may be utilized. Nonlimiting examples are citrate, sodium carbonate, sodium bicarbonate and sodium citrate, Examples of phosphonates include 1-Hydroxy Ethylidene-1,1-Diphosphonic Acid (HEDP, etidronic acid), Diethylenetriamine Penta(Methylene Phosphonic acid) (DTPMP), Ethylene diamine tetra(methylene phosphonic acid) (EDTMPA), amino tris(methylenephosphonic acid) (ATMP), Nitrilo trimethylene phosphonic acid (NTMP), 2-Amino ethyl phosphonic acid (AEPn), Dimethyl methylphosphonate (DMPP),Tetramethylene diamine tetra(methylene phosphonic acid) (TDTMP), Hexamethylene diamine tetra(methylene phosphonic acid) (HDTMP), Phosphonobutane-tricarboxylic acid (PBTC), N-(phosphonomethyl) iminodiacetic acid (PMIDA), 2-carboxyethyl phosphonic acid (CEPA), 2-Hydroxy phosphonocarboxylic acid (HPAA) and Amino-tris-(methylene-phosphonic acid) (AMP). L-glutamic acid N,N-diacetic acid tetra sodium salt (GLDA)., methylglycinediacetic acid (MGDA). Non-limiting examples of builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-*N,N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-*N*-monoacetic acid (ASMA), aspartic acid-N,N-diacetic acid (ASDA), aspartic acid-*N*-monopropionic acid (ASMP), iminodisuccinic acid (IDA), N-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N,N*-diacetic acid (α-ALDA), serine-*N,N*-diacetic acid (SEDA), isoserine-*N,N*-diacetic acid (ISDA), phenylalanine-*N,N*-diacetic acid (PHDA), anthranilic acid-N,N-diacetic acid (ANDA), sulfanilic acid-*N,N*-diacetic acid (SLDA) , taurine-*N,N*-diacetic acid (TUDA) and sulfomethyl-*N,N*-diacetic acid (SMDA), *N*-(2-hydroxyethyl)ethylenediamine-*N,N',N"*-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

In an embodiment, the builder or co-builder is a non-naturally occurring builder or co-builder.

**Bleaching Systems**

The detergent may contain 0-30% by weight, such as about 1% to about 20%, of a bleaching system. Any bleaching system known in the art for use in laundry detergents may be utilized. Suitable bleaching system components include bleaching catalysts, photobleaches, bleach activators, sources of hydrogen peroxide such as sodium percarbonate, sodium perborates and hydrogen peroxide-urea (1:1), preformed peracids and mixtures thereof. Suitable preformed peracids include, but are not limited to, peroxycarboxylic acids and salts, diperoxydicarboxylic acids, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with hydrogen peroxide to form a peracid via perhydrolysis. The peracid thus formed constitutes the activated bleach. Suitable bleach activators to be used herein include those belonging to the class of esters, amides, imides or anhydrides. Suitable examples are tetraacetylethylenediamine (TAED), sodium 4-[(3,5,5-trimethylhexanoyl)oxy]benzene-1-sulfonate (ISONOBS), 4-(dodecanoyloxy)benzene-1-sulfonate (LOBS), 4-(decanoyloxy)benzene-1-sulfonate, 4-(decanoyloxy)benzoate (DOBS or DOBA), 4-(nonanoyloxy)benzene-1-sulfonate (NOBS), and/or those disclosed in WO98/17767. A particular family of bleach activators of interest was disclosed in EP624154 and particulary preferred in that family is acetyl triethyl citrate (ATC). ATC or a short chain triglyceride like triacetin has the advantage that it is environmentally friendly Furthermore acetyl triethyl citrate and triacetin have good hydrolytical stability in the product upon storage and are efficient bleach activators. Finally, ATC is multifunctional, as the citrate released in the perhydrolysis reaction may function as a builder. Alternatively, the bleaching system may comprise peroxyacids of, for example, the amide, imide, or sulfone type. The bleaching system may also comprise peracids such as 6-(phthalimido) peroxyhexanoic acid (PAP). The bleaching system may also include a bleach catalyst. In some embodiments the bleach component may be an organic catalyst selected from the group consisting of organic catalysts having the following formulae:

(i) 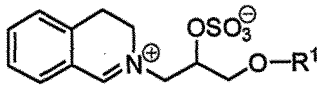

(ii) 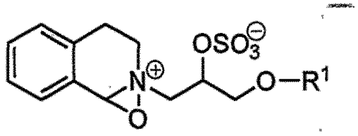

(iii) and mixtures thereof,

wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl. Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Preferably the bleach component comprises a source of peracid in addition to bleach catalyst, particularly organic bleach catalyst. The source of peracid may be selected from (a) preformed peracid; (b) percarbonate, perborate or persulfate salt (hydrogen peroxide source) preferably in combination with a bleach activator; and (c) perhydrolase enzyme and an ester for forming peracid in situ in the presence of water in a textile or hard surface treatment step.

In an embodiment, the bleaching system is a non-naturally occurring bleaching system.

### Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

In an embodiment, the polymer is a non-naturally occurring polymer.

### Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates and may also include pigments. Suitable dyes include small molecule dyes and polymeric dyes. Suitable small molecule dyes include small molecule dyes selected from the group consisting of dyes falling into the Color Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226.. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

## Additional Enzymes

The detergent additive as well as the detergent composition may comprise one or more [additional] enzymes such as hydrolases (EC 3.-.-.-) such as hydrolases acting on ester bonds (EC 3.1.-.-), glycosidases (EC 3.2.-.-), and hydrolases acting on peptide bonds (EC 3.4.-.-), oxidoreductases (EC 1.-.-.-) such as laccases (EC 1.10.-.-) or peroxidases (EC 1.11.-.-) or lyases (EC 4.-.-.-) such as carbon-oxygen lyases (EC 4.2.-.-). In a specific embodiment the detergent composition may comprise one or more [additional] enzymes such as a protease, lipase, cutinase, an amylase, carbohydrase, cellulase, pectinase, mannanase, arabinase, galactanase, xylanase, nuclease, oxidase, *e.g*., a laccase, and/or peroxidase.

In general, the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

## Cellulases

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases providing or maintaining whiteness and preventing redeposition or having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

## Mannanases

Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly *B. agaradhaerens, B. licheniformis, B. halodurans, B. clausii,* or *H. insolens.* Suitable mannanases are described in WO 1999/064619. A commercially available mannanase is Mannaway (Novozymes A/S).

## Proteases

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Eng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin* 147 and *subtilisin* 168 described in WO89/06279 and protease PD138 described in (WO93/18140). Other useful proteases may be those described in WO92/175177, WO01/016285, WO02/026024 and WO02/016547. Examples of trypsin-like proteases are trypsin (e.g. of porcine or bovine origin) and the *Fusarium* protease described in WO89/06270,

WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Blaze®, Blaze® Evity®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® Esperase®, Progress Excel®, and Progress Uno® (Novozymes A/S), those sold under the tradename Maxatase®, Maxacal®, Maxapem®, Purafect®, Purafect Prime®, Preferenz™, Purafect MA®, Purafect Ox®, Purafect OxP®, Puramax®, Properase®, Effectenz™, FN2®, FN3® , FN4®, Excellase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

## Lipases and Cutinases

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*), e.g. *P. alcaligenes* or *P. pseudoalcaligenes* (EP218272), *P. cepacia* (EP331376), *P. sp.* strain SD705 (WO95/06720 & WO96/27002), *P*. *wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™:; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

## Amylases

Suitable amylases which can be used together with the variants of the invention may be an alpha-amylase or a glucoamylase and may be of bacterial or fungal origin. Chemically modified or protein engineered mutants are included. Amylases include, for example, alpha-amylases obtained from *Bacillus*, *e.g.*, a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181

and 182 and a substitution in position 193.

Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those having the substitutions:
M 197T;

H156Y+A181T+N190F+A209V+Q264S;

or

G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;

N128C+K178L+T182G+F202Y+Y305R+D319T+G475K;

S125A+N128C+K178L+T182G+Y305R+G475K;

or

S125A+N128C+T131I+T165I+K178L+T182G+Y305R+G475K

wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183,

G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™ and BAN™ (from Novozymes A/S), and Rapidase™, Purastar™/Effectenz™, Powerase™, Preferenz S1000™ Preferenz S110™ and Preferenz S100™ (from Genencor International Inc./DuPont).

## Peroxidases/Oxidases

A peroxidase is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase may also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase can include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

## Nucleases

Suitable nucleases include deoxyribonucleases (DNases) and ribonucleases (RNases) which are any enzyme that catalyzes the hydrolytic cleavage of phosphodiester linkages in the DNA or RNA backbone respectively, thus degrading DNA and RNA. There are two primary classifications based on the locus of activity. Exonucleases digest nucleic acids from the ends. Endonucleases act on regions in the middle of target molecules. The nuclease is preferably a DNase, which is preferable is obtainable from a microorganism, preferably a fungi or bacterium. In particular, a DNase which is obtainable from a species of Bacillus is preferred; in particular a DNase which is obtainable from *Bacillus cibi, Bacillus subtilis* or *Bacillus licheniformis* is preferred. Examples of such DNases are described in WO 2011/098579,

WO2014/087011 and WO2017/060475. Particularly preferred is also a DNase obtainable from a species of *Aspergillus;* in particular a DNase which is obtainable from *Aspergillus oryzae,* such as a DNase described in WO 2015/155350.

## Licheninases

Suitable licheninases (lichenases) include enzymes that catalyse the hydrolysis of the beta-1,4-glucosidic bonds to give beta-glucans. Licheninases (or lichenases) (*e.g.* EC 3.2.1.73) hydrolyse (1,4)-beta-D-glucosidic linkages in beta-D-glucans containing (1,3)- and (1,4)-bonds and can act on lichenin and cereal beta-D-glucans, but not on beta-D-glucans containing only 1,3- or 1,4-bonds. Examples of such licheninases are described in patent application WO 2017/097866 and in WO 2017/129754.

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.* as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

**Adjunct materials-**Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

**Dispersants** - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

**Dye Transfer Inhibiting Agents** - The detergent compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

**Fluorescent whitening agent** - The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl] benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

**Soil release polymers** - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

**Anti-redeposition agents** - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

**Rheology Modifiers** - are structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

**Other suitable adjunct materials** include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, structurants for liquid detergents and/or structure elasticizing agents.

### Formulation of enzyme in co-granule

The xyloglucanase variants may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulate for the detergent industry is disclosed in the IP.com disclosure IPCOM000200739D.

Another example of formulation of enzymes by the use of co-granulates are disclosed in WO 2013/188331, which relates to a detergent composition comprising (a) a multi-enzyme co-granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component. WO 2013/188331 also relates to a method of treating and/or cleaning a surface, preferably a fabric surface comprising the steps of (i) contacting said surface with the detergent composition as claimed and described herein in aqueous wash liquor, (ii) rinsing and/or drying the surface.

### Formulation of detergent products

The detergent composition of the invention may be in any convenient form, e.g., a bar, a homogenous tablet, a tablet having two or more layers, a unit dose product such as a pouch having one or more compartments, a regular or compact powder, a granule, a paste, a gel, or a regular, compact or concentrated liquid.

Detergent ingredients can be separated physically from each other by compartments in water dissolvable pouches. Thereby negative storage interaction between components can be avoided. Different dissolution profiles of each of the compartments can also give rise to delayed dissolution of selected components in the wash solution.

The detergent composition may take the form of a unit dose product. A unit dose product is the packaging of a single dose in a non-reusable container. It is increasingly used in detergents for laundry. A detergent unit dose product is the packaging (*e.g.*, in a pouch made from a water soluble film) of the amount of detergent used for a single wash.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water-soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water-soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water-soluble film. The compartment for liquid components can be different in composition than compartments containing solids. Ref: (US2009/0011970 A1).

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

The present invention is further described by the following examples that should not be construed as limiting the scope of the invention

## EXAMPLES

### Materials and Methods

General methods of PCR, cloning, ligation nucleotides etc. are well-known to a person skilled in the art and may for example be found in in "Molecular cloning: A laboratory manual", Sambrook et al. (1989), Cold Spring Harbor lab., Cold Spring Harbor, NY; Ausubel, F. M. et al. (eds.); "Current protocols in Molecular Biology", John Wiley and Sons, (1995); Harwood, C. R., and Cutting, S. M. (eds.); "DNA Cloning: A Practical Approach, Volumes I and II", D.N. Glover ed. (1985); "Oligonucleotide Synthesis", M.J. Gait ed. (1984); "Nucleic Acid Hybridization", B.D. Hames & S.J. Higgins eds (1985); "A Practical Guide To Molecular Cloning", B. Perbal, (1984).

### Composition of model detergent A (liquid)

Composition of detergent A (liquid): Ingredients: 12% LAS, 11% AEO Biosoft N25-7 (NI), 7% AEOS (SLES), 6% MPG (monopropylene glycol), 3% ethanol, 3% TEA, 2.75% cocoa soap, 2.75% soya soap, 2% glycerol, 2% sodium hydroxide, 2% sodium citrate, 1% sodium formiate, 0.2% DTMPA and 0.2% PCA, water to 100% (all percentages are w/w).

### Composition of model detergent A2 (liquid)

Composition of detergent A2 (liquid): Ingredients: 12% LAS, 12% AEO Biosoft N25-7 (NI), 4% AEOS (SLES), 2% MPG (monopropylene glycol), 3% ethanol, 2% TEA (triethyl amine), 3% soap, 0.5% sodium hydroxide, 3.9% sodium citrate, 1.5% DTMPA, Na7 (diethylenetraminepentakis (methylene)pentakis(phosphonic acid), heptasodium salt), 0.5% phenoxyethanol, water to 100% (all percentages are w/w).

## WASH ASSAYS

Launder-O-Meter (LOM) Model Wash System

The Launder-O-Meter (LOM) is a medium scale model wash system that can be applied to test up to 20 different wash conditions simultaneously. A LOM is basically a large temperature-controlled water bath with 20 closed metal beakers rotating inside it. Each beaker constitutes one small washing machine and during an experiment, each will contain

a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time-consuming full-scale experiments in front loader washing machines.

Mini Launder-O-Meter (MiniLOM) Model Wash System

MiniLOM is a modified mini wash system of the Launder-O-Meter (LOM), which is a medium scale model wash system that can be applied to test up to 20 different wash conditions simultaneously. A LOM is basically a large temperature-controlled water bath with 20 closed metal beakers rotating inside it. Each beaker constitutes one small washing machine and during an experiment, each will contain a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time-consuming full-scale experiments in front loader washing machines.

In miniLOM, washes are performed in 50 ml test tubes placed in Stuart rotator.

Terg-O-Tometer (TOM) wash assay

The Tergo-To-Meter (TOM) is a medium scale model wash system that can be applied to test 12 different wash conditions simultaneously. A TOM is basically a large temperature controlled water bath with up to 12 open metal beakers submerged into it. Each beaker constitutes one small top loader style washing machine and during an experiment, each of them will contain a solution of a specific detergent/enzyme system and the soiled and unsoiled fabrics its performance is tested on. Mechanical stress is achieved by a rotating stirring arm, which stirs the liquid within each beaker. Because the TOM beakers have no lid, it is possible to withdraw samples during a TOM experiment and assay for information on-line during wash.

The TOM model wash system is mainly used in medium scale testing of detergents and enzymes at US or LA/AP wash conditions. In a TOM experiment, factors such as the ballast to soil ratio and the fabric to wash liquor ratio can be varied. Therefore, the TOM provides the link between small scale experiments, such as AMSA and mini-wash, and the more time consuming full scale experiments in top loader washing machines.

Equipment: The water bath with 12 steel beakers and 1 rotating arm per beaker with capacity of 500 or 1200mL of detergent solution. Temperature ranges from 5 to 80°C. The water bath has to be filled up with deionised water. Rotational speed can be set up to 70 to 120rpm/min.

Set temperature in the Terg-O-Tometer and start the rotation in the water bath. Wait for the temperature to adjust (tolerance is +/- 0,5°C). All beakers shall be clean and without traces of prior test material.

The wash solution with desired amount of detergent, temperature and water hardness is prepared in a bucket. The detergent is allowed to dissolve during magnet stirring for 10 min. Wash solution shall be used within 30 to 60 min after preparation.

800ml wash solution is added into a TOM beaker. The wash solution is agitated at 120rpm and optionally one or more enzymes are added to the beaker. The swatches are sprinkled into the beaker and then the ballast load. Time measurement starts when the swatches and ballast are added to the beaker. The swatches are washed for 20 minutes after which agitation is terminated. The wash load is subsequently transferred from the TOM beaker to a sieve and rinse with cold tap water. The soiled swatches are separated from the ballast load. The soil swatches are transferred to a 5L beaker with cold tap water under running water for 5 minutes. The ballast load is kept separately for the coming inactivation. The water is gently pressed out of the swatches by hand and placed on a tray covered with a paper. Another paper is placed on top of the swatches. The swatches are allowed to dry overnight before subjecting the swatches to analysis, such as measuring the color intensity using a Color Eye.

**Example 1 - Production and purification of xyloglucanase variants**

The xyloglucanase variants of the present invention are prepared by standard procedures, in brief: Introducing random and/or site-directed mutations into the gene, transforming Bacillus subtilis host cells with the mutated genes, fermenting the transformed host cells, and obtaining the xyloglucanase variant from the fermentation broth.

Fermentation was carried out in shake flask cultures at 37°C for 3 days shaking of 300 ml TB-Gly medium

containing 13.3 g/L Tryptone, 26.6 g/L Yeast Extract (Bacto) and 4.4 g/L Sorbitol 0.44% added with 0.6 ml of trace element solution containing 0.7% $FeCl_2.2H_2O$, 0.1% $MnCl_2.4H_2O$ and 1.5% $MgSO_4.7H_2O$ was added.

After fermentation, the culture broth was harvested by centrifugation (13000 x g, 20 min). The supernatant was then filtered through a 0.2μ cut off hollow fiber filter (CFP-2-E-4MA) tangential flow filtration system (GE QuixStand™ bench-top system) to remove any host cells. The conductivity of the filtered sample was then raised by addition of 1M $(NH_4)_2SO_4$, this sample was then applied on Butyl Toyopearl 650M (Tosoh Biosciences) equilibrated in 25 mM Tris/HCl, 1M $(NH_4)_2SO_4$, 1 mM $CaCl_2$, pH 8.0. After washing the column with equilibration buffer followed by another round of extensive washing in wash buffer containing 25 mM Tris/HCl, 0.85 M $(NH4)_2SO4$, 1 mM $CaCl_2$, pH 8.0 the protein was eluted by isocratic elution with 25 mM Tris/HCl, 0.3 M NaCl, 1 mM $CaCl_2$, pH 8.0, elution fraction corresponding to UV peak was on column buffer exchanged to 25 mM Tris/HCl, 1 mM $CaCl_2$, pH 8.0 through Sephadex G25 column (Merck). The buffer exchanged sample was then applied to Nuvia Q Resin (Bio-Rad) equilibrated with 25 mM Tris/HCl, 1mM $CaCl_2$, pH 8.0, after washing the column with equilibration buffer the protein was then eluted in 25 mM Tris/HCl, 150 mM NaCl, 1 mM $CaCl_2$, pH 8.0. Elution fractions corresponding to UV elution peak on the chromatogram were collected pooled as purified protein.

**Example 2** - **Xyloglucanase assay**

The xyloglucanase activity of enzyme samples, e.g. from purification, are measured in an AZCL-xyloglucan assay.

AZCL-xyloglucan (Megazyme) is incubated with the xyloglucanase and the liberated blue colour was measured at 650nm. The xyloglucanase activity is calculated as the increase in blue colour during incubation after subtraction of the proper blank value.

| | |
|---|---|
| AZCL-xyloglucan substrate : | 4 mg/ml AZCL-xyloglucan (Megazyme) homogeneously suspended in 0.01% Triton X-100 by stirring. |
| Assay temperature : | 37°C. |
| Assay buffer : | 50mM succinic acid/NaOH, 0.01% Triton X-100, pH 5.0. |

500μl AZCL-xyloglucan substrate suspension is placed on ice in an Eppendorf tube. 500μl Assay buffer is added and the mixture is allowed to become ice-cold. 20μl enzyme sample (diluted in 0.01% Triton X-100) is added. The assay is initiated by transferring the Eppendorf tube to an Eppendorf thermomixer, which is set to the assay temperature. The tube is incubated for 15 minutes on the Eppendorf thermomixer at its highest shaking rate (1400 rpm). The incubation is stopped by transferring the tube back to the ice bath. When the tube has become ice-cold, the tube is centrifuged shortly in an ice-cold centrifuge to precipitate unreacted substrate. 200μl supernatant is transferred to a microtiter plate and $A_{650}$ is read. A buffer blank (20μl 0.01% Triton X-100 instead of enzyme) is included in the assay and the difference in $A_{650}$ between enzyme sample and buffer blank is a measure of the xyloglucanase activity.

**Example 3** - **Stability of Xyloglucanase variants**

The detergent stability of xyloglucanase variants in the present invention was assessed by measuring their activities after subjecting them to stress by incubating in Model A2 liquid detergent containing protease at elevated temperature of 42°C or 45°C for a defined time. After the prescribed incubation time, the enzyme activities of these stressed samples were determined and compared with the activities of equivalent samples stored at 4°C for the same time period. Stability is inferred from the residual activity found in the stressed samples stored at elevated temperature, expressed as percentage of the activities found in the unstressed cold stored samples. The % residual activity (%RA) results for the xyloglucanase variants were compared to those for the parental xyloglucanase, tested under the same conditions. The ratio between these two stability results is the Stability Improvement Factor (SIF). Additionally, we determined the variant half-life ($T_{1/2}$) or the time required for the variants to reduce to 50% of their original activities based on their incubation time in detergent and % residual activity. The ratio between half-lives of the variants and the parental xyloglucanase is the Half-life Improvement Factor (HIF). Variants having SIF or HIF >1 are more stable than the parental xyloglucanase under the tested conditions. Preferred variants are those that have high SIF or HIF in this test.

Materials and Reagents

(A) Dilution buffer: 100 mM MOPS, pH 8.0 containing 0.01% Triton X-100

- Prepare 1M MOPS by adding 209.06g of MOPS (Sigma, M1254) in 1L of Milli-Q water and adjusting pH to 8.0 using 5M NaOH solution. Prepare 10% Triton-X100 stock solution by adding 10 ml of Triton-X100 (Sigma, T8787) 90 ml of Milli-Q water.
- For making dilution buffer, add 100 ml of 1M MOPS and 1ml of 10% Triton-X100, and make up the volume to 1L with Milli-Q water using a measuring cylinder.

(B) Detergent mix: Model A2 detergent (as above) + 1% protease of SEQ ID NO: 4 having mutations: S9E+N42-R+N74D+V1991+Q200L+Y203W+S253D+N255W+L256E

- In a glass bottle, add 100 ml Model A2 detergent and 1ml of protease. Add a magnetic stir bar and leave it for mixing on a magnetic stirrer for 30 min or until it is uniformly mixed.

(C) Substrate: 1.3% Cellazyme T (1 Cellazyme T tablet per 5 ml of dilution buffer)

- Cellazyme T tablets (Megazymes, T-CTZ-1000T) are tablets that contain azurine-crosslinked dyed xyloglucan. This is an insoluble substrate that is highly sensitive for assays of xyloglucanases and endo-cellulases. Prepare 1.3% substrate fresh on the day of assay by adding 20 Cellazyme T tablets in 100 ml of dilution buffer in a beaker. Add a magnetic stir bar and leave it for stirring for 10 min.

(D) Purified proteins: Xyloglucanase of SEQ ID NO: 2 was purified and used as the parental xyloglucanase reference. Xylogucanase variants were fermented and purified as in Example 1, above. All protein concentrations were normalized to 200 ppm using dilution buffer.
(E) Consumables: 96-well plate (Nunc, 260836), 384-well plate (Nunc, 262160), 50 μl MCA 96 nested tips (Tecan, 30038609), 100 μl MCA 96 nested tips (Tecan, 30038614), 200 μl MCA 96 nested tips (Tecan, 30038619), Wide-bore tips (Axygen Scientific, T-205-WB-C), Trough (Axygen Scientific, RES-SW96-HP)
(F) Sample Incubator: Stress samples were incubated in the temperature controlled Liconic incubator (set at 42°C) and unstress samples were incubated at 4°C in fridge (Samsung)
(G) Thermomixer used for assay: Eppendorf ThermoMixer C
(H) Tecan Evo Freedom 150 liquid handler for automated sample handling and Magellan 200 Pro for measuring absorbance

Procedure

(A) Dispense 100 μl of normalized 200 ppm purified proteins as 4 replicates in a 96-well plate. Also include buffer blank where dilution buffer is added as 4 replicates.
(B) Pour detergent mix in a trough and dispense 90 μl/well in 96-well plates in Tecan.
(C) Add 10 μl from enzyme plate to 90 μl detergent plates in Tecan. Seal the plates and mix at 1400 rpm, 25 °C for 30 min using thermomixer.
(D) Store the plates at 4 °C for unstress and 42 °C for stress for 15 hr.
(E) Make fresh 1.3% Cellazyme T substrate in a beaker on the day of assay. While continuously stirring on the magnetic stirrer, dispense 180 μl substrate/well using wide-bore tips in a 96-well plate. Continuous stirring of substrate helps the insoluble substrate particles to stay in motion and prevents sedimentation, thereby ensuring that the substrate is uniformly dispensed in the 96-well plate.
(F) Bring the unstress and stress plates to room temperature by mixing at 1400 rpm, 25 °C for 10 min. Add 20 μl unstressed and stressed samples to 180 μl substrate plates in Tecan, so that the final in-assay protein concentration is 2 ppm. Place the plate on thermomixer and mix at 800 rpm, 25 °C for 45 min.
(G) Allow the assay plates to rest for 10 min so that the unreacted substrate particles settle down, and then transfer 50 μl supernatant from 96-well plates to a 384-well plate in Tecan. Read absorbance at 590 nm.

Data analysis

(A) There were two OD590 measurements for each sample:

OD(US): absorbance at 590 nm for the unstressed enzyme sample at 4 °C

OD(S): absorbance at 590 nm for the stressed enzyme sample at 42 °C
Similarly, there were two OD590 measurements for buffer blank samples at unstress and stress conditions [OD(Blank_US) and OD(Blank_S)] that have equivalent amount of detergent but no enzyme, that would be averaged across the four blank replicates.

(B) Calculate the blanked OD values for enzyme samples by subtracting the absorbance of buffer blank sample and taking an average across the four enzyme replicates.

$$BOD(US) = OD(US) - OD(Blank_US)$$

$$BOD(S) = OD(S) - OD(Blank_S)$$

For each enzyme, the lower limit for BOD(US) was set at 0.3 and that for B(OD) at 0.05, below which the values are in noise range and hence not used for any stability calculations.
(C) Stability is represented as percent residual activity (%RA)

$$\%RA = \frac{BOD(S)}{BOD(US)} * 100$$

(D) Stability Improvement Factor (SIF) is calculated by taking a ratio of residual activities of variant and reference (here, Xyloglucanase of SEQ ID NO: 2 or SEQ ID NO: 3)

$$SIF = \frac{\%RA(variant)}{\%RA(reference)}$$

(E) Enzyme half-life ($T_{1/2}$) or the time in hours it takes for the enzyme to lose 50% of its original activity is calculated based on the residual activity and the total duration of sample incubation in detergent ($T_{det}$, here 15 hr)

$$T_{1/2} = \frac{\ln(0.5)}{\ln(\%RA/100)} * T_{det}$$

(F) Half-life Improvement Factor (HIF) is calculated by taking a ratio of half-lives of variant and reference

$$HIF = \frac{T_{1/2}(variant)}{T_{1/2}(reference)}$$

Table 1. Samples stored for 16 h at 42°C (HIF compared to reference xyloglucanase of SEQ ID NO: 2)

| **Mutations compared to SEQ ID NO: 1** | **Mutations compared to SEQ ID NO: 2** | **90% Model A2 + 1% protease** | |
|---|---|---|---|
| | | **IF** | **HIF** |
| Q68H+T92V+K118A+K129A+R156Y+G200P+ N331F | - | 1.0 | 1.0 |
| Q68H+T92V+K118A+K129A+R156Y+G200P+ N331F | A129T | 1.7 | 1.4 |
| Q68H+T92V+K118A+S127W+K129A+R156Y+ +G200P+N331F | S127W | 1.5 | 1.3 |
| Q68H+T92V+K118A+E126P+K129A+R156Y, +G200P+N331F | E126P | 1.4 | 1.2 |
| Q68H+T92V+K118A+S127H+K129A+R156Y+ G200P+N331F | S127H | 1.4 | 1.2 |
| Q68H+T92V+T104G+K118A+K129A+R156Y+ G200P+N331F | T104G | 1.4 | 1.2 |
| Q68H+T92V+K118A+Q125K+K129A+R156Y+G20 0P+N331F | Q125K | 1.4 | 1.2 |
| Q68H+T92V+K118A+Q125P+K129A+R156Y+G20 0P+N331F | Q125P | 1.4 | 1.2 |

(continued)

| Mutations compared to SEQ ID NO: 1 | Mutations compared to SEQ ID NO: 2 | 90% Model A2 + 1% protease | |
|---|---|---|---|
| | | IF | HIF |
| Q68H+T92V+K118A+Q125S+K129A+R156Y+G20 0P+N331F | Q125S | 1.3 | 1.2 |
| Q68H+T92V+K118A+K129A+R156Y+G200P+ N331F+D395P | D395P | 1.2 | 1.1 |
| Q68H+T92V+G103V+K118A+K129A+R156Y+ G200P+N331F | G103V | 1.2 | 1.1 |
| Q68H+T92V+T104R+K118A+K129A+R156Y+ G200P+N331F | T104R | 1.2 | 1.1 |
| Q68H+T92V+K118A+Q125L+K129A+R156Y+ G200P+N331F | Q125L | 1.2 | 1.1 |
| A41E+Q68H+T92V+K118A+K129A+R156Y+ G200P+N331F | A41E | 1.1 | 1.1 |
| A41R+Q68H+T92V+K118A+K129A+R156Y+ G200P+N331F | A41R | 1.1 | 1.0 |
| Q68H+T92V+K118A+Q125F+K129A+R156Y+ G200P+N331F | Q125F | 0.9 | 0.9 |
| Q68H+T92V+K118A+S127L+K129A+R156Y+ G200P+N331F | S127L | 0.6 | 0.8 |
| Q68H+T92V+K118A+K129A+R156Y+G200P+ A226K+N331F | A226K | 0.3 | 0.6 |
| A41L+Q68H+T92V+K118A+K129A+R156Y+ G200P+N331F | A41L | * | |
| Q68H+T92V+K118A+K129A+R156Y+G200P+ A226D+N331F | A226D | * | |
| * not stable under given condition | | | |

**Table 2. Samples stored for 16 h at 42°C (HIF compared to reference xyloglucanase of SEQ ID NO: 3)**

| Mutations compared to SEQ ID NO: 1 | Mutations compared to SEQ ID NO: 3 | HIF [90% Model A2 + 1% protease] |
|---|---|---|
| Q68H+T92V+K118A+K129T+R1 56Y+G200P+N331F | - | 1.0 |
| Q68H+T92V+K118A+S123P+K1 29T+R156Y+G200P+N331F | S123P | 1.21 |
| Q68H+T92V+K118A+K129T+R1 56Y+G200P+N331F+K488T | K488T | 1.13 |
| Q68H+T92V+K118A+K129T+R1 56Y+G200P+S256Q+N331F | S256Q | 1.12 |
| Q68H+T92V+K118A+K129T+R1 56Y+G200P+Q271D+N331F | Q271D | 1.15 |
| Q68H+T92V+K118A+K129T+R1 56Y+G200P+S214Q+N331F | S214Q | 1.14 |
| Q68H+T92V+K118A+K129T+R1 56Y+G200P+N331F+K482R | K482 R | 1.10 |
| Q68H+T92V+K118A+K129T+L1 52D+R156Y+G200P+N331F | L152D | 1.10 |
| Q68H+T92V+K118A+K129T+R1 56Y+G200P+R267K+N331F | R267K | 1.12 |
| Q68H+T92V+K118A+K129T+L1 52E+R156Y+G200P+N331F | L152E | 1.10 |
| Q68H+T92V+K118A+K129T+R1 56Y+G200P+N331F+D210R | D210R | 1.21 |
| Q68H+T92V+K118A+K129T+ L152* +R156Y+G200P+N331F | L152* | 1.28 |

(continued)

| Mutations compared to SEQ ID NO: 1 | Mutations compared to SEQ ID NO: 3 | HIF [90% Model A2 + 1% protease] |
|---|---|---|
| Q68H+T92V+K118A+K129T+R1 56Y+G200P+N331F R295K | R295K | 1.65 |
| Q68H+T92V+K118A+K129T+R1 56Y+G200P+R267C+N331F+T4 27V | R267C+T427V | 1.12 |
| Q68H+T92V+K118A+K129T+R1 56Y+G200P+ Q243E+N331F | Q243E | 1.20 |
| Q68H+T92V+K118A+K129T+R1 56Y+G200P+N331F+K414E | K414E | 1.10 |
| Q68H+T92V+K118A+K129T+R1 56Y+G200P+N331F+K445E | K445E | 1.10 |
| R20K+Q68H+T92V+K118A+K12 9T+S123P+R156Y+G200P+N33 1F | R20K+S123P | 1.25 |
| Q68H+T92V+K118A+K206R+S1 23P+K129T+R156Y+ G200P+N331F | S123P+K206R | 1.25 |
| R20K+Q68H+T92V+K118A+S12 3P+K129T+R156Y+G200P+R21 1K+N331F | R20K+S123P+R211K | 1.24 |
| Q68H+T92V+K118A+K129T+R1 56Y+G200P+N331F+S123P+K3 47R+K353R+D395P | S123P+K347R+K353R+ D395P | 1.25 |
| Q68H+T92V+K118A+S123P+K1 29T+R156Y+G200P+N331F+D3 95P | S123P+D395P | 1.15 |
| Q68H+T92V+K118A+S123P+K1 29T+R156Y+G200P+N331F | S123P | 1.25 |
| Q68H+T92V+K118A+K129T+R1 56Y+V159M+G200P+N331F | V159M | 1.15 |
| Q68H+Q82E+T92V+K118A+K12 9T+R156Y+G200P+N331F | Q82E | 1.12 |
| Q68H+S76E+T92V+K118A+K12 9T+R156Y+G200P+N331F | S76E | 1.24 |
| A83E+Q68H+T92V+K118A+K12 9T+R156Y+G200P+N331F | A83E | 1.26 |
| Q68H+T92V+K118A+K129T+R1 56Y+G200P+1294E+N331F | I294E | 1.44 |
| Q68H+T92V+K118A+K129T+R1 56Y+G200P+N331F+Q329E | Q329E | 1.15 |
| R20K+Q68H+T92V+ Y105E+K118A+ S123P+K129T+R156Y+ Q147K+G200P+R267K+N331F | R20K+Y105E+S123P+Q 147K+R267K | 1.25 |
| R20K+Q68H+T92V+K118A+ S123P+K129T+K220R+R156Y+ G200P+N331F | R20K+S123P+K220R | 1.15 |
| R20K+Q68H+T92V+ Y105E+K118A+S123P+K129T+ N136D+R156Y+G200P+N331F | R20K+Y105E+S123P+N 136D | 1.29 |
| R20K+Q68H+T92V+K118A+S12 3P+K129T+Q137K+ Q147K+R156Y+G200P+N331F | R20K+S123P+Q137K+Q 147K | 1.33 |

(continued)

| Mutations compared to SEQ ID NO: 1 | Mutations compared to SEQ ID NO: 3 | HIF [90% Model A2 + 1% protease] |
|---|---|---|
| Q68H+T92V+Y105E+A118K+ S123P+K129T+R156Y+G200P+ K206R+R267K | Y105E+A118K+S123P+ K206R+K220R+R267K | 1.25 |
| Q68H+T92V+P111Q+K118A+ S123P+K129T+R156Y+ V159M+G200P+N331F | P111Q+S123P+V159M | 1.28 |
| Q68H+T92V+S94R+K118A+ P111Q+ S123P+K129T+R156Y+ V159M+G200P+N331F | S94R+P111Q+S123P+V 159M | 1.31 |
| Q68H+ K87E+T92V+ P111Q+K118A+ S123P+K129T+R156Y+ V159M+G200P+N331F+ S402Q | K87E+P111Q+S123P+V 159M+S402Q | 1.41 |
| Q68H+ K87E+T92V+ P111Q+ K118A+ S123P+K129T+R156Y+ V159M+G200P+N331F | K87E+P111Q+S123P+V 159M | 1.36 |
| Q68H+T92V+S94R+ P111Q+K118A+ S123P+K129T+R156Y+ V159M+G200P+N331F | S94R+P111Q+S123P+V 159M | 1.30 |
| Q68H+ K87E+T92V+ P111Q+K118A+ S123P+K129T+R156Y+ V159M+G200P+ N331F+ S402Q | K87E+P111Q+S123P+V 159M+S402Q | 1.34 |
| Q68H+T92V+P111Q+ K118A+ S123P+K129T+R156Y+ V159M+G200P+N331F+S402Q | P111Q+S123P+V159M+ S402Q | 1.53 |
| Q68H+T92V+ P111Q+K118A++S123P K129T+R156Y+V159M G200P+N331F | P111Q+S123P+V159M | 1.51 |
| Q68H+T92V+S94R+ P111Q+K118A++S123P K129T+R156Y+V159M G200P+N331F | S94R+P111Q+S123P+V 159M | 1.23 |
| Q68H+K87E+T92V+ P111Q+K118A+K129T+R156Y+ V159M+G200P+ I294Q+N331F+I473T | K87E+P111Q+V159M+I 294Q+I473T | 1.19 |
| K8E+Q68H+T92V+ P111Q+K118A+K129T+R156Y+ V159M+G200P+N331F | K8E+P111Q+V159M | 1.24 |
| K8E+ Q68H+T92V+ P111Q+K118A+K129T+R156Y+ V159M+G200P+N331F | K8E+P111Q+V159M | 1.16 |
| K8E+ K18E+ Q68H+T92V+ P111Q+K118A+K129-T+R156Y+ V159M+ G200P+ K206E+N331F | K8E+K18E+P111Q+V15 9M+K206E | 1.32 |
| R20K+Q68H+T92V+ P111Q+K118A+S123P+ S127D+K129T+R156Y+ V159M+G200P+N331F | R20K+P111Q+S123P+S 127D+V159M | 1.14 |
| Q68H+T92V+P111Q+ K118A+ S123P+K129T+R156Y+ V159M+G200P+N331F | P111Q+S123P+V159M | 1.27 |
| Q68H+T92V+ P111Q+K118A+K129T+ Q147K+R156Y+ V159M+ G200P+ K220R+N331F | P111Q+Q147K+V159M+ K220R | 1.20 |
| Q68H+T92V+ P111Q+K118A+K129T+R156Y+ V159M+ G200P+ K206E+1294Q+N331F | P111Q+V159M+K206E+ I294Q | 1.13 |
| Q68H+T92V+P111Q+ K118A+K129T+R156Y+ V159M+ G200P+ K206E+ I294Q+ N331F+K347E | P111Q+ V159M+ K206E+ I294Q+ K347E | 1.39 |
| Q68H+T92V+ P111Q+K118A+K129T+ Q137K+ Q147K+R156Y+ V159M+G200P+ K252E+N331F | P111Q+ Q137K+ Q147K+ V159M+ K252E | 1.69 |
| Q68H+T92V+P111Q K118A+K129T+Q137K+ Q147K+ N155D R156Y+G200P+K252E+N331F | P111Q+ Q137K+ Q147K+ N155D+ K252E | 1.35 |

(continued)

| Mutations compared to SEQ ID NO: 1 | Mutations compared to SEQ ID NO: 3 | HIF [90% Model A2 + 1% protease] |
|---|---|---|
| Q68H+T92V+ P111Q+K118A+K129T+ Q137K+ L152D+R156Y+G200P+ V203T+ K217R+N331F | P111Q+ Q137K+ L152D+ V203T+ K217R | 1.24 |
| Q68H+T92V+ P111Q+K118A+K129T+ Q137K+ R156Y+ V159M+G200P+N331F | P111Q+ Q137K+ V159M | 1.46 |
| K8E+ Q68H+T92V+ P111Q+K118A+K129T+N155D+ R156Y+ V159M+G200P+N331F | K8E+ P111Q+ N155D+ V159M | 1.40 |
| A41L+Q68H+T92V+ P111Q+K118A+S123P+K129T+ Q147K+R156Y+V159M G200P+V203T+N331F | A41L+ P111Q+ S123P+ Q147K+ V159M+ V203T | 1.61 |
| A41L+Q68H+T92V+ P111Q+K118A+ S123P+K129T+ Q147K+R156Y+ V159M+G200P+ K217R+N331F | A41L+ P111Q+ S123P+ Q147K+ V159M+ K217R | 1.79 |
| Q68H+T92V+P111Q+K118A+ S123P+K129T+ Q137K+ Q147K+R156Y+ V159M+G200P+ S256Q+N331F+ S402Q | P111Q+ S123P+ Q137K+ Q147K+ V159M+ S256Q+ S402Q | 2.25 |
| Q68H+T92V+P111Q+ K118A+ S123P+K129T+ Q147K+R156Y+ V159M+G200P+ I294Q+N331F+S402Q | P111Q+ S123P+ Q147K+ V159M+ I294Q+ S402Q | 2.77 |
| A41L+ Q68H+T92V+ P111Q+K118A+K129T+Q137K+ R156Y+ V159M+N168R+G200P+Q271D+ N331F+K488T | A41L+ P111Q+ Q137K+ V159M+ N168R+ Q271D+ K488T | 1.58 |
| Q68H+T92V+ P111Q+K118A+K129T+ Q137K+ Q147K+ R156Y+ V159M+ G200P+ V203T+ K217R+N331F | P111Q+ Q137K+ Q147K+ V159M+ V203T+ K217R | 1.81 |
| Q68H+T92V+P111Q+ K118A+K129T+ Q147K+R156Y+ V159M+G200P+N331F | P111Q+ Q147K+ V159M | 1.62 |
| Q68H+T92V+P111Q+ K118A+K129T+ Q137K+ Q147K+ R156Y+ V159M+G200P+N331F | P111Q+ Q137K+ Q147K+ V159M | 1.58 |
| Q68H+T92V+P111Q+K118A+ S123P+K129T+ Q137K+R156Y+ V159M+G200P+N331F+ K488T | P111Q+ S123P+ Q137K+ V159M+ K488T | 1.87 |
| Q68H+T92V+P111Q+K118A+ S123P+K129T+ Q137K+R156Y+ V159M+G200P+ S256Q+N331F | P111Q+ S123P+ Q137K+ V159M+ S256Q | 1.77 |
| Q68H+T92V+P111Q+ K118A+K129T+ Q137K+R156Y+ V159M+G200P+N331F | P111Q+ Q137K+ V159M | 1.65 |
| K87E+Q68H+T92V+ P111Q+K118A+K129T+ L152D+R156Y+ V159M+G200P+ V203T+I294Q+N331F | K87E+ P111Q+ L152D+ V159M+ V203T+ I294Q | 1.49 |
| K87E+Q68H+T92V+ P111Q+K118A+K129T+ Q147K+ L152D+R156Y+ V159M+G200P+N331F | K87E+ P111Q+ Q147K+ L152D+ V159M | 1.57 |
| R20K+Q68H+ A83E+T92V+K118A+ S123P+K129T+R156Y+G200P+ K220R+S256E+N331F | R20K+ A83E+ S123P+ K220R+ S256E | 1.94 |
| R20K+ Q68H+ S76E+ A83E+T92V+K118A+ S123P+K129T+R156Y+G200P+ K220R+ N331F+K252E | R20K+ S76E+ A83E+ S123P+ K220R+ K252E | 1.88 |
| R20K+Q68H+ A83E+T92V+K118A+S123P+K129-T+R156Y+G200P+ K220R+K252E+N331F | R20K+ A83E+ S123P+ K220R+ K252E | 1.98 |
| R20K+A42V+Q68H+T92V+K118 A+S123P+K129T+R156Y+G200 P+K220R+K252E+1294E+N331F | R20K+ A42V+ S123P+ K220R+ K252E+ I294E | 2.38 |
| R20K+A42V+ Q68H+T92V+K118A+ S123P+K129T+ K220R+R156Y+G200P+N331F K252E+ I294E | R20K+ A42V+ S123P+ K220R+ K252E+ I294E | 2.32 |

(continued)

| Mutations compared to SEQ ID NO: 1 | Mutations compared to SEQ ID NO: 3 | HIF [90% Model A2 + 1% protease] |
|---|---|---|
| R20K+Q68H+Q82E+T92V+K118 A+S123P+K129T+Q147K+R156 Y+G200P+K220R+N331F | R20K+ Q82E+ S123P+ Q147K+ K220R | 1.54 |
| R20K+ Q68H+ A83E+ T92V+K118A+ S123P+K129T+R156Y+G200P+ K220R+S256Q+N331F | R20K+ A83E+ S123P+ K220R+ S256Q | 1.73 |
| R20K+Q68H+ Q82E+T92V+K118A+ S123P+K129T+ N155D+R156Y+G200P+ K220R+N331F | R20K+ Q82E+ S123P+ N155D+ K220R | 1.40 |
| R20K+Q68H+T92V+K118A+ S123P+K129T+R156Y+G200P+ V203T+ K220R+ K252E+ N331F | R20K+ S123P+ V203T+ K220R+ K252E | 1.67 |
| R20K+Q68H+T92V+K118A+ S123P+K129T+R156Y+G200P+ K220R+ I294E+ N331F | R20K+ S123P+ K220R+ I294E | 2.26 |
| R20K+Q68H+ Q82E+ T92V+K118A+ S123P+K129T+ Q147K+R156Y+G200P+ K220R+N331F | R20K+ Q82E+ S123P+ Q147K+ K220R | 1.49 |
| R20K+Q68H+ A83E+T92V+K118A+ S123P+K129T+R156Y+G200P+ V203T+ K220R+ K252E+ N331F | R20K+ A83E+ S123P+ V203T+ K220R+ K252E | 1.63 |
| R20K+ A41L+ Q68H+ Q82E+T92V+K118A+ S123P+K129T+R156Y+G200P+ K220R+N331F | R20K+ A41L+ Q82E+ S123P+ K220R | 1.62 |
| R20K+Q68H+T92V+K118A+ S123P+K129T+ N155D+R156Y+G200P+ K220R+N331F | R20K+ S123P+ N155D+ K220R | 1.64 |
| R20K+Q68H+ A83E+T92V+K118A+ S123P+K129T+R156Y+G200P+ K220R+ S256E N331F | R20K+ A83E+ S123P+ K220R+ S256E | 2.27 |
| R20K+ A42V+ Q68H+ S76E+ T92V+K118A+ S123P+ K129T+R156Y+G200P+ K220R+N331F | R20K+ A42V+ S76E+ S123P+ K220R | 1.67 |
| R20K+Q68H+T92V+K118A+ S123P+K129T+R156Y+G200P+ V203T+ V219T+N331F | R20K+ S123P+ V203T+ V219T | 1.66 |
| R20K+ A42V+ Q68H+ A83E+ T92V+K118A+ S123P+K129T+R156Y+G200P+ K220R+N331F | R20K+ A42V+ A83E+ S123P+ K220R | 2.02 |
| R20K+Q68H+T92V+K118A+ S123P+K129T+ N155D+R156Y+G200P+ K220R+N331F | R20K+ S123P+ N155D+ K220R | 1.56 |
| R20K+ A42V+ Q68H+ S76E+T92V+K118A+ S123P+K129T+R156Y+G200P+ V203T+ K220R+N331F | R20K+ A42V+ S76E+ S123P+ V203T+ K220R | 2.28 |
| Q68H+ A83E+T92V+ P111Q+K118A+ S123P+K129T+R156Y+ V159M+G200P+ S256E+ I294E+ N331F | A83E+ P111Q+ S123P+ V159M+ S256E+ I294E | 5.30 |
| Q68H+ Q82E+T92V+ P111Q+K118A+ S123P+ K129T+R156Y+ V159M+ G200P+ S256E+ I294E+N331F | Q82E+ P111Q+ S123P+ V159M+ S256E+ I294E | 4.88 |
| Q68H+ Q82E+T92V+ P111Q+K118A+ S123P+ K129T+ Q147K+ R156Y+ V159M+ G200P+ I294E+N331F | Q82E+ P111Q+ S123P+ Q147K+ V159M+ I294E | 3.98 |
| Q68H+ S76E+ Q82E+ K87E+ T92V+ P111Q+K118A+ S123P+ K129T+R156Y+ V159M+G200P+ V203T+ N331F | S76E+ Q82E+ K87E+ P111Q+ S123P+ V159M+ V203T | 2.51 |

(continued)

| Mutations compared to SEQ ID NO: 1 | Mutations compared to SEQ ID NO: 3 | HIF [90% Model A2 + 1% protease] |
|---|---|---|
| A83E+Q68H+T92V+ P111Q+K118A+ S123P+ K129T+ Q137K+ Q147K+ R156Y+ V159M+G200P+ S256Q+ S402Q+N331F | A83E+ P111Q+ S123P+ Q137K+ Q147K+ V159M+ S256Q+ S402Q | 2.44 |
| Q68H+T92V+P111Q+ K118A+ S123P+K129T+ Q137K+ Q147K+ N155D+ R156Y+G200P+ S256Q+ A289T+ N302H+ N331F | P111Q+ S123P+ Q137K+ Q147K+ N155D+ S256Q+ A289T+ N302H | 2.42 |
| Q68H+ S76E+ T92V+ P111Q+K118A+ S123P+K129T+ Q137E+ Q147K+ R156Y+G200P+ V159M+ K252E+ S256Q+ N331F +S402Q | S76E+ P111Q+ S123P+ Q137E+ Q147K+ V159M+ K252E+ S256Q+ S402Q | 3.21 |
| Q68H+T92V+P111Q+K118A+ S123P+K129T+ Q137K+ Q147K+ R156Y+ V159M+ G200P+ K252E+ S256Q+ N331F+ S402Q | P111Q+ S123P+ Q137K+ Q147K+ V159M+ K252E+ S256Q+ S402Q | 1.90 |
| Q68H+T92V+P111Q+K118A+ S123P+K129T+ Q137K+ Q147K+ N155D+ R156Y+G200P+ S256Q+ I294E+ S402Q+ N331F | P111Q+ S123P+ Q137K+ Q147K+ N155D+ S256Q+ I294E+ S402Q | 3.28 |
| Q68H+ Q82E+T92V+ P111Q+K118A+ S123P+K129T+ Q137K+ Q147K+R156Y+ V159M+ L184M+ G200P+ A238S+ S256Q+ I294E+ N331F+S402Q | Q82E+ P111Q+ S123P+ Q137K+ Q147K+ V159M+ L184M+ A238S+ S256Q+ I294E+ S402Q | 3.91 |
| Q68H+ A83E+T92V+ P111Q+K118A+ S123P+K129T+ Q137K+ Q147K+ R156Y+ V159M+ L184M+G200P+ S256Q+ I294E+N331F+S402Q | A83E+ P111Q+ S123P+ Q137K+ Q147K+ V159M+ L184M+ S256Q+ I294E+ S402Q | 4.54 |
| Q68H+T92V+P111Q+K118A+ N121E+K129T+ Q137K+ Q147K+ R156Y+ V159M+G200P+ K169R+ S256Q+ I294E+ N331F+S402Q | P111Q+ N121E+ Q137K+ Q147K+ V159M+ K169R+ S256Q+ I294E+ S402Q | 7.68 |
| Q68H+T92V+P111Q+K118A+ S123P+K129T+ Q137K+ Q147K+R156Y+ V159M+ K169R+G200P+ S256Q+ I294E+ N331F+S402Q | P111Q+ S123P+ Q137K+ Q147K+ V159M+ K169R+ S256Q+ I294E+ S402Q | 3.39 |
| Q68H+T92V+P111Q+K118A+ S123P+K129T+ Q137K+ Q147K+R156Y+ V159M+ L184M+G200P+ V219T+ S256Q+ N331F+S402Q | P111Q+ S123P+ Q137K+ Q147K+ V159M+ L184M+ V219T+ S256Q+ S402Q | 2.23 |
| Q68H+ A83E+T92V+ P111Q+K118A+ S123P+K129T+ Q137K+ Q147K+ R156Y+ V159M+ L184M+ G200P+ S256Q+ N331F+S402Q | A83E+ P111Q+ S123P+ Q137K+ Q147K+ V159M+ L184M+ S256Q+ S402Q | 2.59 |
| Q68H+ A83E+T92V+ P111Q+K118A+ S123P+K129T+ Q137K+ Q147K+R156Y+ V159M+G200P+N331F S256Q+ S402Q | A83E+ P111Q+ S123P+ Q137K+ Q147K+ V159M+ S256Q+ S402Q | 2.64 |
| Q68H+T92V+ P111Q+K118A+K129T+ S123P+ Q137K+ Q147K+R156Y+ V159M+G200P+ K252E+ S256Q+ N331F+S402Q | P111Q+ S123P+ Q137K+ Q147K+ V159M+ K252E+ S256Q+ S402Q | 2.27 |
| Q68H+ Q82E+ T92V+ P111Q+K118A+ S123P+K129T+ Q137K+ Q147K+R156Y+ V159M+ G200P+ K252E+ S256Q+ N331F+ S402Q | Q82E+ P111Q+ S123P+ Q137K+ Q147K+ V159M+ K252E+ S256Q+ S402Q | 2.61 |
| Q68H+ S76E+T92V+ P111Q+K118A+ S123P+ K129T+ Q137K+ Q147K+R156Y+ V159M+G200P+ S256Q+N331F+ S402Q | S76E+ P111Q+ S123P+ Q137K+ Q147K+ V159M+ S256Q+ S402Q | 2.24 |

(continued)

| Mutations compared to SEQ ID NO: 1 | Mutations compared to SEQ ID NO: 3 | HIF [90% Model A2 + 1% protease] |
|---|---|---|
| K8R+ Q68H+T92V P111Q++K118A+ S123P+K129T+ Q137K+ Q147K+R156Y+ V159M+ G200P+ S256Q+ N331F + S402Q | K8R+ P111Q+ S123P+ Q137K+ Q147K+ V159M+ S256Q+ S402Q | 1.81 |
| Q68H+T92V+ P111Q+K118A+ S123P+K129T+ Q137K+ Q147K+R156Y+ V159M+G200P+ S256Q+N331F+S402Q | P111Q+ S123P+ Q137K+ Q147K+ V159M+ S256Q+ S402Q | 1.86 |
| Q68H+T92V+K118A+K129T+R1 56Y+G200P+N331F S123P+ S127D+ N136D+ Q137K+ Q147K+ L152E+ N153E+ N155E | S123P+ S127D+ N136D+ Q137K+ Q147K+ L152E+ N153E+ N155E | 1.51 |
| Q68H+T92V+K118A+ S123P+ S127D+ K129T+R156Y+ N136D+ Q137K+ Q147K+ L152E+ N153E+ N155E+ G200P+N331F+A491E | S123P+ S127D+ N136D+ Q137K+ Q147K+ L152E+ N153E+ N155E+ A491E | 1.31 |
| R20K+Q68H+T92V+K118A+ S123P+K129T+R156Y+ K169R+G200P+ K217T+ K240F+ S256Q+ R267H+ I294E+ N331F | R20K+ S123P+ K169R+ K217T+ K240F+ S256Q+ R267H+ I294E | 4.14 |
| Q68H+ K87E+T92V+ P111Q+K118A+ S123P+K129T+R156Y+ V159M+G200P+ K217T+ I294E+N331F | K87E+ P111Q+ S123P+ V159M+ K217T+ I294E | 2.27 |
| Q68H+ K87E+ T92V+K118A+ P111Q+ S123P+K129T+R156Y+ V159M+G200P+ K217T+ I294E+N331F | K87E+ P111Q+ S123P+ V159M+ K217T+ I294E | 2.71 |
| Q68H+ A83E+T92V+ P111Q+K118A+ S123P+K129T+R156Y+ V159M+G200P+ K240F+ K252E+N331F | A83E+ P111Q+ S123P+ V159M+ K240F+ K252E | 3.12 |
| Q68H+ A83E+T92V+ P111Q+K118A+ S123P+K129T+R156Y+ V159M+G200P+ K252E+N331F | A83E+ P111Q+ S123P+ V159M+ K252E | 1.86 |
| Q68H+ K87E+T92V+ P111Q+K118A+ S123P+K129T+R156Y+V159M+G200P+ S256Q+ I294E+N331F | K87E+ P111Q+ S123P+ V159M+ S256Q+ I294E | 2.69 |
| Q68H+ K87E+ T92V+K118A+ P111Q+ S123P+ K129T+R156Y+ V159M+G200P+ S256Q+ I294E+N331F | K87E+ P111Q+ S123P+ V159M+ S256Q+ I294E | 2.65 |
| Q68H+ K87E+T92V+ P111Q+K118A+ S123P+K129T+R156Y+ V159M+G200P+N331F+K347E+ N383E | K87E+ P111Q+ S123P+ V159M+ K347E+ N383E | 1.99 |
| Q68H+ Q82E+T92V+ P111Q+K118A+ S123P+K129T+R156Y+ V159M+G200P+V203T+ G237M+ S256E+ I294E+N331F | Q82E+ P111Q+ S123P+ V159M+ V203T+ G237M+ S256E+ I294E | 13.06 |
| Q68H+ Q82E+ T92V+ P111Q+ K118A+ S123P+K129T+R156Y+ V159M+G200P+ V203T+ G237M+ S256E+ I294E+N331F | Q82E+ P111Q+ S123P+ V159M+ V203T+ G237M+ S256E+ I294E | 9.05 |
| Q68H+ Q82E+T92V+ P111Q+K118A+ S123P+K129T+ N155D+R156Y+G200P+ S256E+ I294E+ N331F | Q82E+ P111Q+ S123P+ N155D+ S256E+ I294E | 4.80 |
| Q68H+ Q82E+ T92V+ P111Q+K118A+ S123P+K129T+ N155D+ R156Y+ K169R+ G200P+ G237M+ S256E+ I294E+N331F | Q82E+ P111Q+ S123P+ N155D+ K169R+ G237M+ S256E+ I294E | 7.05 |
| Q68H+ Q82E+ T92V+ P111Q+ K118A+ S123P+ K129T+ Q147K+R156Y+ V159M+ G200P+ S256E+ I294E+N331F | Q82E+ P111Q+ S123P+ Q147K+ V159M+ S256E+ I294E | 5.05 |

(continued)

| Mutations compared to SEQ ID NO: 1 | Mutations compared to SEQ ID NO: 3 | HIF [90% Model A2 + 1% protease] |
|---|---|---|
| Q68H+ Q82E+T92V+ P111Q+K118A+K129T+ S123P+ Q137K+R156Y+ V159M+G200P+ G237M+ S256E+ I294E+N331F | Q82E+ P111Q+ S123P+ Q137K+ V159M+ G237M+ S256E+ I294E | 11.65 |
| Q68H+ Q82E+ T92V+ P111Q+K118A+ S123P+K129T+ Q137K+R156Y+ V159M+ G200P+ K240F+ S256E+ I294E+N331F | Q82E+ P111Q+ S123P+ Q137K+ V159M+ K240F+ S256E+ I294E | 11.28 |
| Q68H+ Q82E+T92V+ P111Q+K118A+ S123P+K129T+ Q147K+ N155D+R156Y+G200P+ K240F+ S256E+ I294E+N331F | Q82E+ P111Q+ S123P+ Q147K+ N155D+ K240F+ S256E+ I294E | 9.51 |
| K8E+Q68H+ Q82E+T92V+ P111Q+K118A+ S123P+K129T+R156Y+ V159M+G200P+ S256E+ I294E+N331F | K8E+ Q82E+ P111Q+ S123P+ V159M+ S256E+ I294E | 5.35 |
| Q68H+ A83E+T92V+ P111Q+K118A+ S123P+K129T+ Q147K+R156Y+ V159M+G200P+ S256E+ I294E+ Q329E+N331F | A83E+ P111Q+ S123P+ Q147K+ V159M+ S256E+ I294E+ Q329E | 15.82 |
| Q68H+ Q82E+T92V+ P111Q+ K118A+ S123P+ K129T+R156Y+ V159M+G200P+ K240F+ S256E+ I294E+N331F | Q82E+ P111Q+ S123P+ V159M+ K240F+ S256E+ I294E | 10.80 |
| Q68H+ Q82E+T92V+ P111Q+K118A+ S123P+K129T+R156Y+ V159M+ A177G+G200P+ K240F+ S256E+ I294E+N331F+D384G | Q82E+ P111Q+ S123P+ V159M+ A177G+ K240F+ S256E+ I294E+ D384G | 9.45 |
| K8R+ Q68H+ Q82E+T92V+ P111Q+K118A+ S123P+K129T+R156Y+ V159M+ K169R+G200P+ K240F+ S256E+ I294E+N331F | K8R+ Q82E+ P111Q+ S123P+ V159M+ K169R+ K240F+ S256E+ I294E | 7.25 |
| Q68H+Q82E+T92V+K118A+K12 9T+R156Y+G200P+N331F+ P111Q+ S123P+ V159M+ K169R+ S256E+ I294E+ V431E | Q82E+ P111Q+ S123P+ V159M+ K169R+ S256E+ I294E+ V431E | 6.01 |
| Q68H+ Q82E+T92V+ P111Q+K118A+ S123P+K129T+R156Y+ V159M+G200P+ G237M+ V251E+ I294E+N331F | Q82E+ P111Q+ S123P+ V159M+ G237M+ V251E+ I294E | 7.95 |
| A41L+Q68H+ Q82E+T92V+ P111Q+K118A+ S123P+ K129T+R156Y+ V159M+G200P+ S256E+ I294E+ N331F + N383E | A41L+ Q82E+ P111Q+ S123P+ V159M+ S256E+ I294E+ N383E | 6.19 |
| Q68H+ A83E+T92V+ P111Q+K118A+ S123P+K129T+ Q137K+R156Y+ F146D+ Q147G+ L148P+ V159M+ L184M+G200P+ S256Q+ I294E+ N331F+S402Q | A83E+ P111Q+ S123P+ Q137K+ F146D+ Q147G+ L148P+ V159M+ L184M+ S256Q+ I294E+ S402Q | 4.30 |
| Q68H+ A83E+T92V+ P111Q+K118A+ S123P+K129T+ Q137K+ Q147K+R156Y+ V159M+ L184M+G200P+ S256Q+ I294E+ N298D+ V300L+N331F+ S402Q | A83E+ P111Q+ S123P+ Q137K+ Q147K+ V159M+ L184M+ S256Q+ I294E+ N298D+ V300L+ S402Q | 8.11 |
| Q68H+ A83E+ T92V+ P111Q+ K118A+K129T+ S123P+ Q137K+ Q147K+ R156Y+ V159M+ K169R+ L184M+G200P+ K240L+ S256Q+ I294E+ N331F+S402Q | A83E+ P111Q+ S123P+ Q137K+ Q147K+ V159M+ K169R+ L184M+ K240L+ S256Q+ I294E+ S402Q | 13.60 |
| Q68H+ A83E+ T92V+ P111Q+K118A+ S123P+K129T+ Q137K+ Q147K+R156Y+V159M+ L184M+ G200P+ G237M+ S256Q+ I294E+ N331F+ S402Q+ K488T | A83E+ P111Q+ S123P+ Q137K+ Q147K+ V159M+ L184M+G237M+ S256Q+ I294E+ S402Q+ K488T | 11.47 |

(continued)

| Mutations compared to SEQ ID NO: 1 | Mutations compared to SEQ ID NO: 3 | HIF [90% Model A2 + 1% protease] |
|---|---|---|
| Q68H+ A83E+T92V+ P111Q+K118A+ S123P+K129T+ Q137K+ Q147K+ N155D+R156Y+ L184M+G200P+ G237M+ S256Q+ I294E+ N331F+S402Q | A83E+ P111Q+ S123P+ Q137K+ Q147K+ N155D+ L184M+ G237M+ S256Q+ I294E+ S402Q | 10.43 |
| Q68H+ A83E+ T92V+ P111Q+ K118A+ S123P+ K129T+ Q137K+ Q147K+ R156Y+ V159M+L184M+G200P+ V203T+ K240F+ S256Q+ I294E+ N331F+ S402Q | A83E+ P111Q+ S123P+ Q137K+ Q147K+ V159M+ L184M+ V203T+ K240F+ S256Q+ I294E+ S402Q | 6.60 |
| Q68H+ A83E+T92V+ P111Q+K118A+ S123P+K129T+ Q137K+ Q147K+R156Y+ V159M+ L184M+G200P+ G237M+ S256E+ I294E+ N331F+ S402Q | A83E+ P111Q+ S123P+ Q137K+ Q147K+ V159M+ L184M+ G237M+ S256E+ I294E+ S402Q | 16.17 |
| Q68H+ A83E+T92V+ P111Q+K118A+ S123P+K129T+ Q137K+ Q147K+R156Y+ V159M+ L184M+G200P+ K240F+ S256Q+ I294E+ N331F+S402Q+ E489R | A83E+ P111Q+ S123P+ Q137K+ Q147K+ V159M+ L184M+ K240F+ S256Q+ I294E+ S402Q+ E489R | 10.48 |
| Q68H+ A83E+T92V+ P111Q+K118A+ S123P+ K129T+ Q137K+ Q147K+R156Y+ V159M+ L184M+ G200P+ K240F+ S256Q+ I294E+ N331F+ S402Q+ E489R | A83E+ P111Q+ S123P+ Q137K+ Q147K+ V159M+ L184M+ K240F+ S256Q+ I294E+ S402Q+ E489R | 14.72 |
| Q68H+ A83E+T92V+ P111Q+K118A+ S123P+K129T+ Q137K+ Q147K+R156Y+ V159M+ L184M+G200P+ G237M+ S256Q+ I294E+N331F+ S402Q+ K488T | A83E+ P111Q+ S123P+ Q137K+ Q147K+ V159M+ L184M+ G237M+ S256Q+ I294E+ S402Q+ K488T | 9.31 |
| Q68H+ A83E+ T92V+ P111Q+K118A+ S123P+K129T+ Q137K+ Q147K+R156Y+ V159M+ F165H+ L184M+ G200P+ S256Q+ I294E+ N331 F+S402Q+ V431E | A83E+ P111Q+ S123P+ Q137K+ Q147K+ V159M+ F165H+ L184M+ S256Q+ I294E+ S402Q+ V431E | 6.11 |
| Q68H+ A83E+T92V+ P111Q+K118A+ S123P+K129T+ Q137K+ Q147K+R156Y+ V159M+ F165H+ L184M+G200P+ S256Q+ I294E+ N331 F+S402Q+ V431E | A83E+ P111Q+ S123P+ Q137K+ Q147K+ V159M+ F165H+ L184M+ S256Q+ I294E+ S402Q+ V431E | 4.86 |
| Q68H+ A83E+T92V+ P111Q+K118A+ S123P+K129T+ Q137K+ Q147K+R156Y+ V159M+ L184M+G200P+ V251E+ Q271E+ I294E+ Q329E+N331F+S402Q | A83E+ P111Q+ S123P+ Q137K+ Q147K+ V159M+ L184M+ V251E+ Q271E+ I294E+ Q329E+ S402Q | 11.00 |
| Q68H+ A83E+ T92V+ P111Q+ K118A+ S123P+K129T+ Q137K+ Q147K+R156Y+ V159M+L184M+G200P+ V251E+ S256Q+ Q271E+I294E+N331F+Q329E+ S402Q | A83E+ P111Q+ S123P+ Q137K+ Q147K+ V159M+ L184M+ V251E+ S256Q+ Q271E+ I294E+ Q329E+ S402Q | 20.61 |
| Q68H+ A83E+ T92V+ P111Q+ K118A+ S123P+K129T+ Q137K+ Q147K+ N155D+ R156Y+ L184M+ G200P+ S256Q+ I294E+ N331F+S402Q | A83E+ P111Q+ S123P+ Q137K+ Q147K+ N155D+ L184M+ S256Q+ I294E+ S402Q | 4.25 |
| R20K+ Q68H+T92V+K118A+ S123P+K129T+ Q147K+ N155D+R156Y+ K169R+ G200P+ K217T+ K240F+ S256Q+ R267H+ I294E+N331F | R20K+ S123P+ Q147K+ N155D+ K169R+ K217T+ K240F+ S256Q+ R267H+ I294E | 4.27 |
| R20K+Q68H+T92V+K118A+ S123P+K129T+ Q137K+R156Y+ K169R+G200P+ K217T+ K240F+ S256Q+ R267H+ I294E N331F | R20K+ S123P+ Q137K+ K169R+ K217T+ K240F+ S256Q+ R267H+ I294E | 4.16 |
| R20K+Q68H+T92V+K118A+ S123P+K129T+ Q137K+R156Y+ K169R+ G200P+ K217T+ K240F+ S256Q+ R267H+ I294E+N331F | R20K+ S123P+ Q137K+ K169R+ K217T+ K240F+ S256Q+ R267H+ I294E | 4.73 |
| R20K+Q68H+T92V+K118A+ S123P+K129T+ N155D+R156Y+ K169R+G200P+ K217T+ K240F+ S256Q+ R267H+ I294E+ N331F+D384G+ E489R | R20K+ S123P+ N155D+ K169R+ K217T+ K240F+ S256Q+ R267H+ I294E+ D384G+ E489R | 4.45 |

(continued)

| Mutations compared to SEQ ID NO: 1 | Mutations compared to SEQ ID NO: 3 | HIF [90% Model A2 + 1% protease] |
|---|---|---|
| R20K+ Q68H+T92V+K118A+ S123P+ K129T+ N155D+ R156Y+ K169R+G200P+ K217T+ K240F+ S256Q+ R267H+ I294E+ N331F+S474E | R20K+ S123P+ N155D+ K169R+ K217T+ K240F+ S256Q+ R267H+ I294E+ S474E | 4.29 |
| R20K+Q68H+T92V+K118A+ S123P+K129T+ N155D+R156Y+ K169R+G200P+ K217T+ K240F+ S256Q+ R267H+ I294E+N331F | R20K+ S123P+ N155D+ K169R+ K217T+ K240F+ S256Q+ R267H+ I294E | 4.43 |
| R20K+Q68H+T92V+K118A+ S123P+ K129T+ Q147K+ R156Y+ K169R+ G200P+ V219T+ K240F+ S256Q+ R267H+ I294E+N331F | R20K+ S123P+ Q147K+ K169R+ V219T+ K240F+ S256Q+ R267H+ I294E | 3.87 |
| R20K+Q68H+T92V+K118A+ S123P+ K129T+ Q147K+R156Y+ K169R+ G200P+ V219T+ K240F+ S256Q+ R267H+ I294E+N331F | R20K+ S123P+ Q147K+ K169R+ V219T+ K240F+ S256Q+ R267H+ I294E | 4.08 |
| R20K+ A41L+ Q68H+T92V+K118A+ S123P+K129T+ L152P+ R156Y+ K169R+ G200P+ K217T+ K240F+ S256Q+ R267H+ I294E+N331F | R20K+ A41L+ S123P+ L152P+ K169R+ K217T+ K240F+ S256Q+ R267H+ I294E | 4.22 |
| R20K+ A41L+Q68H+T92V+K118A+ S123P+K129T+ L152P+R156Y+ K169R+G200P+ K217T+ K240F+ S256Q+ R267H+ I294E+N331F | R20K+ A41L+ S123P+ L152P+ K169R+ K217T+ K240F+ S256Q+ R267H+ I294E | 4.55 |
| K8R+ R20K+Q68H+T92V+K118A+ S123P+K129T+R156Y+ K169R+G200P+ D210H+ K217T+ K240F+ S256Q+ R267H+ I294E+N331F | K8R+ R20K+ S123P+ K169R+ D210H+ K217T+ K240F+ S256Q+ R267H+ I294E | 4.05 |
| R20K+ Q68H+T92V+K118A+ S123P+K129T+R156Y+ K169R+G200P+ K217T+ K240F+ S256Q+ R267H+ I294E+ Q329E+ N331F +V431E+ E489R | R20K+ S123P+ K169R+ K217T+ K240F+ S256Q+ R267H+ I294E+ Q329E+ V431E+ E489R | 5.56 |
| Q68H+T92V+ P111Q+K118A+ S123P+ K129T+ Q137K+ Q147K+R156Y+ V159M+ K169R+G200P+ D210H+ K240F+ S256E+ I294E+ N331F+S402Q+ K488T | P111Q+ S123P+ Q137K+ Q147K+ V159M+ K169R+ D210H+ K240F+ S256E+ I294E+ S402Q+ K488T | 7.32 |
| Q68H+T92V+ P111Q+K118A+ S123P+ K129T+ Q137K+ Q147K+R156Y+ V159M+ K169R+ G200P+ K252E+ S256Q+ I294E+N331F+K322E+ S402Q | P111Q+ S123P+ Q137K+ Q147K+ V159M+ K169R+ K252E+ S256Q+ I294E+ K322E+ S402Q | 2.82 |
| Q68H+T92V+K118A+ P111Q+ S123P+K129T+ Q137K+ Q147K+R156Y+ V159M+ K169R+G200P+ K240F+ S256Q+ I294E+ K322E+ N331F+S402Q | P111Q+ S123P+ Q137K+ Q147K+ V159M+ K169R+ K240F+ S256Q+ I294E+ K322E+ S402Q | 6.03 |
| Q68H+ S76E+T92V+ P111Q+K118A+ S123P+ K129T+ Q137K+ Q147K+ R156Y+ V159M+ K169R+G200P+ G237M+ S256Q+ I294E+ N331F+ S402Q | S76E+ P111Q+ S123P+ Q137K+ Q147K+ V159M+ K169R+ G237M+ S256Q+ I294E+ S402Q | 10.00 |
| Q68H+ S76E+T92V+ P111Q+K118A+ S123P+K129T+ Q137K+ Q147K+R156Y+ V159M+ K169R+ G200P+ V203T+ G237M+ S256Q+ I294E+ N331F+ S402Q | S76E+ P111Q+ S123P+ Q137K+ Q147K+ V159M+ K169R+ V203T+ G237M+ S256Q+ I294E+ S402Q | 10.82 |
| Q68H+T92V+ P111Q+K118A+ S123P+K129T+ Q137K+ Q147K+R156Y+ V159M+ K169R+G200P+ G237M+ S256Q+ I294E+ N331F+P339S+ S402Q+ K488T | P111Q+ S123P+ Q137K+ Q147K+ V159M+ K169R+ G237M+ S256Q+ I294E+ P339S+ S402Q+ K488T | 6.47 |
| Q68H+T92V+ P111Q+ K118A+ S123P+K129T+ Q137K+ Q147K+R156Y+ V159M+ K169R+ G200P+ G237M+ T244R+ S256Q+ I294E+ N331F+ S402Q | P111Q+ S123P+ Q137K+ Q147K+ V159M+ K169R+ G237M+ T244R+ S256Q+ I294E+ S402Q | 7.10 |

(continued)

| Mutations compared to SEQ ID NO: 1 | Mutations compared to SEQ ID NO: 3 | HIF [90% Model A2 + 1% protease] |
|---|---|---|
| Q68H+T92V+ P111Q+K118A+ S123P+K129T+ Q137K+ Q147K+ R156Y+ V159M+G200P+ G237M+ T244R+ S256Q+ I294E+ N331F+ S402Q | P111Q+ S123P+ Q137K+ Q147K+ V159M+ G237M+ T244R+ S256Q+ I294E+ S402Q | 7.03 |
| Q68H+T92V+ P111Q+K118A+ S123P+K129T+ Q137K+ Q147K+R156Y+ V159M+ K169R+ G200P+ V219T+ K240F+ S256Q+ I294E+ N331F+S402Q | P111Q+ S123P+ Q137K+ Q147K+ V159M+ K169R+ V219T+ K240F+ S256Q+ I294E+ S402Q | 6.74 |
| Q68H+T92V+ P111Q+ K118A+ S123P+K129T+ Q137K+ Q147K+R156Y+ V159M+ K169R+G200P+ V203T+ S256Q+ I294E+ N331F+S402Q | P111Q+ S123P+ Q137K+ Q147K+ V159M+ K169R+ V203T+ S256Q+ I294E+ S402Q | 3.84 |
| Q68H+ Q82E+T92V+ P111Q+K118A+ S123P+ K129T+ Q137K+R156Y+ V159M+ G200P+ V203T+ D210H+ G237M+ S256E+ I294E+ N331F | Q82E+ P111Q+ S123P+ Q137K+ V159M+ V203T+ D210H+ G237M+ S256E+ I294E | 13.62 |
| Q68H+ Q82E+T92V+ P111Q+K118A+ S123P+K129T+R156Y+ V159M+G200P+ V203T+ G237M+ K252E+ S256E+ I294E+N331F | Q82E+ P111Q+ S123P+ V159M+ V203T+ G237M+ K252E+ S256E+ I294E | 13.03 |
| Q68H+ Q82E+T92V+ P111Q+K118A+ S123P+K129T+R156Y+ V159M+G200P+ V203T+ G237M+ S256E+ I294E+N331F | Q82E+ P111Q+ S123P+ V159M+ V203T+ G237M+ S256E+ I294E | 11.7 |
| Q68H+ S76E+ Q82E+T92V+ P111Q+K118A+ S123P+K129T+R156Y+ V159M+G200P+ V203T+ G237M+ S256E+ I294E+N331F | S76E+ Q82E+ P111Q+ S123P+ V159M+ V203T+ G237M+ S256E+ I294E | 9.97 |
| Q68H+ Q82E+T92V+ P111Q+K118A+ S123P+K129T+ Q147K+R156Y+ V159M+G200P+ V203T+ G237M+ S256E+ I294E+N331F | Q82E+ P111Q+ S123P+ Q147K+ V159M+ V203T+ G237M+ S256E+ I294E | 12.70 |
| Q68H+ S76E+ Q82E+ T92V+ P111Q+ K118A+ S123P+ K129T+R156Y+ V159M+ G200P+ V203T+ G237M+ S256E+ I294E+ N331F+S474E+ E489R+ P492D | S76E+ Q82E+ P111Q+ S123P+ V159M+ V203T+ G237M+ S256E+ I294E+ S474E+ E489R+ P492D | 11.42 |
| Q68H+ Q82E+T92V+ P111Q+K118A+ S123P+K129T+R156Y+ V159M+ K169R+ G200P+ V203T+ G237M+ T244R+ S256E+ I294E+N331F | Q82E+ P111Q+ S123P+ V159M+ K169R+ V203T+ G237M+ T244R+ S256E+ I294E | 12.34 |
| Q68H+ Q82E+T92V+ P111Q+K118A+ S123P+K129T+R156Y+ V159M+ K169R+G200P+V203T+ G237M+ T244R+ S256E+ I294E+N331F | Q82E+ P111Q+ S123P+ V159M+ K169R+ V203T+ G237M+ T244R+ S256E+ I294E | 13.31 |
| Q68H+ Q82E+T92V+ P111Q+K118A+ S123P+K129T+R156Y+ V159M+G200P+ V203T+ G237M+ T244R+ S256E + I294E+N331F | Q82E+ P111Q+ S123P+ V159M+ V203T+ G237M+ T244R+ S256E+ I294E | 15.61 |

**Table 3. Samples stored for 16 h at 45°C (HIF compared to reference xyloglucanase of SEQ ID NO: 3)**

| Mutations compared to SEQ ID NO: 1 | Mutations compared to SEQ ID NO: 3 | HIF [90% Model A2 + 5% protease ] |
|---|---|---|
| Q68H+A83E+T92V+P111Q+K118A +S123P+K129T+R156Y+V159M+G 200P+V159M+S256E+I294E+N331 F | A83E+ P111Q+ S123P+ V159M+ S256E+ I294E | 3.30 |

(continued)

| Mutations compared to SEQ ID NO: 1 | Mutations compared to SEQ ID NO: 3 | HIF [90% Model A2 + 5% protease ] |
|---|---|---|
| R20K+Q68H+T92V+K118A+ S123P+K129T+R156Y+K169R+G200P+K217T+K240F+S256Q+ R267H+ I294E+N331F | R20K+ S123P+ K169R+ K217T+ K240F+ S256Q+ R267H+ I294E | 2.73 |
| Q68H+ Q82E+ T92V+ P111Q+K118A+ S123P+K129T+R156Y+ V159M+G200P+ V203T+ S256E+G237M+ I294E+N331F | Q82E+ P111Q+ S123P+ V159M+ V203T+ G237M+ S256E+ I294E | 10.06 |
| Q68H+ A83E+T92V+ P111Q+K118A+ S123P+K129T+ Q147K+R156Y+ V159M+ G200P+ S256E+ I294E+N331-F+Q329E | A83E+ P111Q+ S123P+ Q147K+ V159M+ S256E+ I294E+ Q329E | 11.26 |
| Q68H+ A83E+T92V+ P111Q+K118A+ S123P+ K129T+Q137K+Q147K+ R156Y+V159M+L184M+ G200P+G237M+S256E+ I294E+N331F+S402Q | A83E+ P111Q+ S123P+ Q137K+ Q147K+ V159M+ L184M+ G237M+ S256E+ I294E+ S402Q | 9.81 |
| Q68H+ S76E+T92V+ P111Q+ K118A+ S123P+K129T+ Q137K+ Q147K+ R156Y+ V159M+ K169R+ G200P+ V203T+ G237M+ S256Q+ I294E+ N331F+S402Q | S76E+ P111Q+ S123P+ Q137K+ Q147K+ V159M+ K169R+ V203T+ G237M+ S256Q+ I294E+ S402Q | 7.60 |
| Q68H+T92V+ P111Q+K118A+ S123P+K129T+ Q137K+ Q147K+R156Y+ V159M+ K169R+ G200P+ G237M+ T244R+ S256Q+ I294E+ N331F+S402Q | P111Q+ S123P+ Q137K+ Q147K+ V159M+ K169R+ G237M+ T244R+ S256Q+ I294E+ S402Q | 7.44 |
| Q68H+ Q82E+ T92V+ P111Q+K118A+ S123P+K129T+ Q137K+ R156Y+ V159M+ G200P+ V203T+ D210H+ G237M+ S256E+ I294E+N331F | Q82E+ P111Q+ S123P+ Q137K+ V159M+ V203T+ D210H+ G237M+ S256E+ I294E | 9.28 |
| Q68H+ S76E+ Q82E+ T92V+ P111Q+K118A+ S123P+ K129T+R156Y+ V159M+G200P+ V203T+ G237M+ S256E+ I294E+ N331F+S474E+ E489R+ P492D | S76E+ Q82E+ P111Q+ S123P+ V159M+ V203T+ G237M+ S256E+ I294E+ S474E+ E489R+ P492D | 9.89 |
| Q68H+ Q82E+ T92V+ P111Q+K118A+ S123P+K129T+R156Y+ V159M+ K169R+ G200P+ V203T+ G237M+ T244R+ S256E+ I294E+N331F | Q82E+ P111Q+ S123P+ V159M+ K169R+ V203T+ G237M+ T244R+ S256E+ I294E | 11.57 |
| Q68H+ S76E+ A83E+ T92V+ P111Q+K118A+ S123P+K129T+ Q137K+ Q147K+ R156Y+ V159M+ L184M+G200P+ V251E+ S256Q+ Q271E+ I294E+ Q329E+N331F+ S402Q | S76E+ A83E+ P111Q+ S123P+ Q137K+ Q147K+ V159M+ L184M+ V251E+ S256Q+ Q271E+ I294E+ Q329E+ S402Q | 11.28 |
| Q68H+ A83E+T92V+K118A+ P111Q+ S123P+K129T+ Q137K+ Q147K+ R156Y+ V159M+ L184M+G200P+ V219T+ G237M+ V251E+ S256Q+ Q271E+ I294E+ Q329E+N331F+S402Q | A83E+ P111Q+ S123P+ Q137K+ Q147K+ V159M+ L184M+ V219T+ G237M+ V251E+ S256Q+ Q271E+ I294E+ Q329E+ S402Q | 14.71 |
| Q68H+ A83E+T92V+ P111Q+ K118A+ S123P+K129T+ Q137K+ Q147K+R156Y+ V159M+ K169R+ L184M+ G200P+ K252E+ S256Q+ Q271E+ I294E+ Q329E+N331F+S402Q | A83E+ P111Q+ S123P+ Q137K+ Q147K+ V159M+ K169R+ L184M+ K252E+ S256Q+ Q271E+ I294E+ Q329E+ S402Q | 9.73 |

(continued)

| Mutations compared to SEQ ID NO: 1 | Mutations compared to SEQ ID NO: 3 | HIF [90% Model A2 + 5% protease ] |
|---|---|---|
| Q68H+ S76E+ A83E+ T92V+ P111Q+ K118A+K129T+ S123P+ Q137K+ Q147K+ R156Y+ V159M+ L184M+ G200P+ V203T+ A238S+ S256E+ Q271E+ I294E+ Q329E+ N331F + S402Q | S76E+ A83E+ P111Q+ S123P+ Q137K+ Q147K+ V159M+ L184M+ V203T+ A238S+ S256E+ Q271E+ I294E+ Q329E+ S402Q | 14.47 |
| Q68H+ A83E+T92V+ P111Q+ K118A+ S123P+K129T+ Q137K+ Q147K+ R156Y V159M+ L184M+ +G200P+ V203T+ A238S+ S256E+ Q271E+ I294E+ Q329E+ N331F +S402Q | A83E+ P111Q+ S123P+ Q137K+ Q147K+ V159M+ L184M+ V203T+ A238S+ S256E+ Q271E+ I294E+ Q329E+ S402Q | 11.67 |
| Q68H+ A83E+ T92V+ P111Q+K118A+ S123P+ K129T+ Q137K+ Q147K+ R156Y+ V159M+ L184M+ G200P+ G237M+ V251E+ S256Q+ Q271E+ I294E+ Q329E+ N331F + S402Q+ E489R+ V505L | A83E+ P111Q+ S123P+ Q137K+ Q147K+ V159M+ L184M+ G237M+ V251E+ S256Q+ Q271E+ I294E+ Q329E+ S402Q+ E489R+ V505L | 14.63 |
| Q68H+ A83E+ T92V+ P111Q+K118A+ S123P+K129T+ Q137K+ Q147K+ R156Y+ V159M+ L184M+G200P+ D210H+ G237M+ V251E+ S256Q+ Q271E+ I294E+ Q329E+ N331F+ S402Q+ E489R+ P492D | A83E+ P111Q+ S123P+ Q137K+ Q147K+ V159M+ L184M+ D210H+ G237M+ V251E+ S256Q+ Q271E+ I294E+ Q329E+ S402Q+ E489R+ P492D | 18.41 |
| Q68H+ A83E+T92V+ P111Q+K118A+ S123P+K129T+ Q137K+ Q147K+ R156Y+ V159M+ L184M+G200P+ D210H+ T244R+ K252E+ S256Q+ Q271E+ I294E+ N331F+ Q329E+ S402Q | A83E+ P111Q+ S123P+ Q137K+ Q147K+ V159M+ L184M+ D210H+ T244R+ K252E+ S256Q+ Q271E+ I294E+ Q329E+ S402Q | 11.09 |
| Q68H+ A83E+ T92V+ P111Q+K118A+ S123P+K129T+ Q137K+ Q147K+R156Y+ V159M+ L184M+G200P+ V251E+ S256Q+ Q271E+ I294E+ Q329E+ N331F+S402Q+ V431E | A83E+ P111Q+ S123P+ Q137K+ Q147K+ V159M+ L184M+ V251E+ S256Q+ Q271E+ I294E+ Q329E+ S402Q+ V431E | 12.21 |
| Q68H+ A83E+T92V+ P111Q+K118A+ S123P+K129T+ Q137K+ Q147K+R156Y+ V159M+ L184M+ G200P+ T244E+ V251E+ S256Q+ Q271E+ I294E+ Q329E+N331F+S402Q+ V431E | A83E+ P111Q+ S123P+ Q137K+ Q147K+ V159M+ L184M+ T244E+ V251E+ S256Q+ Q271E+ I294E+ Q329E+ S402Q+ V431E | 12.03 |
| Q68H+A83E+T92V+P111Q+K118A +S123P+K129T+Q137K+Q147K+R 156Y+V159M+L184M+G200P+V251E+S256Q+Q271E+I294E+ Q329E+N331F+K347E+S402Q | A83E+ P111Q+ S123P+ Q137K+ Q147K+ V159M+ L184M+ V251E+S256Q+ Q271E+ I294E+ Q329E+ K347E+ S402Q | 9.77 |
| K8E+ Q68H+ A83E+ T92V+ S94R+ P111Q+ K118A+ S123P+K129T+ Q137K+ Q147K+ R156Y+ V159M+ L184M+ G200P+ V251E+ S256Q+ Q271E+ I294E+ Q329E+ N331F+S402Q | K8E+ A83E+ S94R+ P111Q+ S123P+ Q137K+ Q147K+ V159M+ L184M+ V251E+ S256Q+ Q271E+ I294E+ Q329E+ S402Q | 8.27 |

(continued)

| Mutations compared to SEQ ID NO: 1 | Mutations compared to SEQ ID NO: 3 | HIF [90% Model A2 + 5% protease ] |
|---|---|---|
| Q68H+A83E+T92V+P111Q+K118A + S123P+K129T+ Q137K+ L152P+ R156Y+ V159M+ L184M + V251E+ G200P+ S256Q+ Q271E+ I294E+ Q329E+ N331F+S402Q+ V431E | A83E+ P111Q+ S123P+ Q137K+ L152P+ V159M+ L184M+ V251E+ S256Q+ Q271E+ I294E+ Q329E+ S402Q+ V431E | 11.72 |
| Q68H+A83E+T92V+P111Q+K118A +S123P+K129T+Q137K+R156Y+ Q147K+ V159M+ L184M+ G200P+ V251E+ S256Q+ Q271E+ I294E+ Q329E+ N331F+S402Q+ V431E+ K445E | A83E+ P111Q+ S123P+ Q137K+ Q147K+ V159M+ L184M+ V251E+ S256Q+ Q271E+ I294E+ Q329E+ S402Q+ V431E+ K445E | 12.12 |
| Q68H+ A83E+ T92V+ P111Q+ K118A+ S123P+K129T+ Q137K+ Q147K+R156Y+ V159M+ L184M+ G200P+ V251E+ S256Q+ Q271E+ I294E+ Q329E+ N331F+S402Q+ V431E+ K445E | A83E+ P111Q+ S123P+Q137K+ Q147K+V159M+ L184M+ V251E+ S256Q+ Q271E+ I294E+ Q329E+S402Q+ V431E+ K445E | 11.73 |
| Q68H+ A83E+ T92V+ P111Q+ K118A+ S123P+ K129T+ Q137K+ Q147K+R156Y+ V159M+ L184M+ G200P+ K252E+ S256Q+ Q271E+ I294E+ Q329E+ N331F+ S402Q | A83E+ P111Q+ S123P+ Q137K+ Q147K+ V159M+ L184M+ K252E+ S256Q+ Q271E+ I294E+ Q329E+ S402Q | 10.18 |
| K8E+Q68H+A83E+T92V+P111Q+K 118A+ S123P+K129T+ Q137K+ Q147K+R156Y+ V159M+ L184M+G200P+ V251E+ S256Q+ Q271E+ I294E+ Q329E+ N331F+ S402Q | K8E+ A83E+ P111Q+ S123P+ Q137K+ Q147K+ V159M+ L184M+ V251E+ S256Q+ Q271E+ I294E+ Q329E+ S402Q | 7.68 |
| K8E+Q68H+ A83E+T92V+ P111Q+ K118A+ S123P+K129T+ Q137K+ Q147K+R156Y+ V159M+ L184M+ G200P+ V251E+ S256Q+ Q271E+ I294E+ Q329E+ N331F +S402Q | K8E+ A83E+ P111Q+ S123P+ Q137K+ Q147K+ V159M+ L184M+ V251E+ S256Q+ Q271E+ I294E+ Q329E+ S402Q | 8.43 |
| Q68H+ A83E+T92V+ P111Q+K118A+ S123P+K129T+ Q137K+ Q147K+ R156Y+ V159M+ L184M+ G200P+ D210H+ K240F+ V251E+ S256Q+ Q271E+ I294E+ Q329E+ N331F+S402Q | A83E+ P111Q+ S123P+ Q137K+ Q147K+ V159M+ L184M+ D210H+ K240F+ V251E+ S256Q+ Q271E+ I294E+ Q329E+ S402Q | 18.55 |
| Q68H+ A83E+T92V+ P111Q+K118A+ S123P+K129T+ Q137K+ Q147K+ N155D+ R156Y+ L184M+ G200P+ G237M+ V251E+S256Q+ Q271E+ I294E+ Q329E+ N331F+S402Q | A83E+ P111Q+ S123P+ Q137K+ Q147K+ N155D+ L184M+ G237M+ V251E+ S256Q+ Q271E+ I294E+ Q329E+ S402Q | 13.63 |
| A41L+ Q68H+ A83E+ T92V+ P111Q+ K118A+ S123P+ K129T+ Q137K+ Q147K+R156Y+ V159M+ L184M+ G200P+ K240F+ V251E+ S256Q+ Q271E+ I294E+ Q329E+ N331F + S402Q | A41L+ A83E+ P111Q+ S123P+ Q137K+ Q147K+ V159M+ L184M+ K240F+ V251E+ S256Q+ Q271E+ I294E+ Q329E+ S402Q | 19.71 |

(continued)

| Mutations compared to SEQ ID NO: 1 | Mutations compared to SEQ ID NO: 3 | HIF [90% Model A2 + 5% protease ] |
|---|---|---|
| Q68H+ Q82E+ T92V+ P111Q+K118A+ S123P+K129T+ Q137K+ Q147K+ R156Y+ V159M+ L184M+ G200P+ V203T+ D210H+ V251E+ S256Q+ Q271E+ I294E+ Q329E+ N331F+S402Q | Q82E+ P111Q+ S123P+ Q137K+ Q147K+ V159M+ L184M+ V203T+ D210H+ V251E+ S256Q+ Q271E+ I294E+ Q329E+ S402Q | 8.65 |
| Q68H+T92V+K118A+K129T+ Q147K+ R156Y+ V159M+ L184M+G200P+ V251E+ S256Q+ Q271E+ I294E+ Q329E+ N331F+S402Q | A41L+ A83E+ P111Q+ S123P+ Q137K+ Q147K+ V159M+ L184M+ V251E+ S256Q+ Q271E+ I294E+ Q329E+ S402Q | 8.36 |
| A41L+Q68H+ A83E+T92V+ P111Q+K118A+ S123P+K129T+ Q137K+ Q147K+R156Y+ V159M+ L184M+ G200P+ V251E+ S256Q+ Q271E+ I294E+ Q329E+ N331F+ N383Q+ S402Q | A41L+ A83E+ P111Q+ S123P+ Q137K+ Q147K+ V159M+ L184M+ V251E+ S256Q+ Q271E+ I294E+ Q329E+ N383Q+ S402Q | 9.21 |
| Q68H+T92V+ P111Q+K118A+ S123P+ K129T+ Q137K+ Q147K+ R156Y+V159M+ G200P + V251E+ S256E+ Q271E+ I294E+ Q329E+N331F+ S402Q | P111Q+ S123P+ Q137K+ Q147K+ V159M+ V251E+ S256E+ Q271E+ I294E+ Q329E+ S402Q | 13.38 |
| Q68H+T92V+ P111Q+K118A+ S123P+K129T+ Q137K+ Q147K+ R156Y+V159M+G200P+ A238T+ V251E+ S256Q+ Q271E+ I294E+ Q329E+ N331F+S402Q | P111Q+ S123P+ Q137K+ Q147K+ V159M+ A238T+ V251E+ S256Q+ Q271E+ I294E+ Q329E+ S402Q | 7.90 |
| Q68H+ A83E+ T92V+ P111Q+ K118A+ S123P+K129T+ Q137K+ Q147K+ R156Y+ V159M+ L184M+ G200P+ V251E+ S256Q+ Q271E+ I294E+ Q329E+N331F+N383Q+ S402Q | A83E+ P111Q+ S123P+ Q137K+ Q147K+ V159M+ L184M+ V251E+ S256Q+ Q271E+ I294E+ Q329E+ N383Q+ S402Q | 9.90 |
| Q68H+ A83E+ T92V+ P111Q+K118A+ S123P+K129T+ Q137K+ Q147K+ R156Y+ V159M+ L184M+ G200P+ V251E+ S256Q+ Q271E+ I294E+ Q329E+ N331F + N383Q+ S402Q | A83E+ P111Q+ S123P+ Q137K+ Q147K+ V159M+ L184M+ V251E+ S256Q+ Q271E+ I294E+ Q329E+ N383Q+ S402Q | 9.92 |
| Q68H+ A83E+T92V+K118A+ P111Q+ S123P+K129T+ Q137K+ Q147K+ R156Y+ V159M+ L184M+ G200P+ T244R+ W248V+ V251E+ S256Q+ Q271E+ I294E+ Q329E+ N331F + S402Q | A83E+ P111Q+ S123P+ Q137K+ Q147K+ V159M+ L184M+ T244R+ W248V+ V251E+ S256Q+ Q271E+ I294E+ Q329E+ S402Q | 10.05 |
| Q68H+ A83E+T92V+ P111Q+K118A+ S123P+ K129T+ Q137K+ Q147K+ R156Y+ V159M+ L184M+ G200P+ V203T+ T244R+ V251E+ S256Q+ Q271E+ I294E+ Q329E+ N331F+ S402Q | A83E+ P111Q+ S123P+ Q137K+ Q147K+ V159M+ L184M+ V203T+ T244R+ V251E+ S256Q+ Q271E+ I294E+ Q329E+ S402Q | 11.21 |
| Q68H+ A83E+T92V+ P111Q+ K118A+ S123P+ K129T+ Q137K+ Q147K+ R156Y+ V159M+ L184M+ G200P+ V251E+ S256Q+ Q271E+ R295K+ N298D+ Q329E+ N331F + S402Q+ L447M | A83E+ P111Q+ S123P+ Q137K+ Q147K+ V159M+ L184M+ V251E+ S256Q+ Q271E+ R295K+ N298D+ Q329E+ S402Q+ L447M | 11.74 |

(continued)

| Mutations compared to SEQ ID NO: 1 | Mutations compared to SEQ ID NO: 3 | HIF [90% Model A2 + 5% protease ] |
|---|---|---|
| Q68H+ A83E+ T92V+ P111Q+ K118A+ S123P+K129T+ Q137K+ Q147K+ R156Y+ V159M+ L184M+ G200P+ S256E+ Q271D+ I294E+ Q329E+ N331F + S402Q | A83E+ P111Q+ S123P+ Q137K+ Q147K+ V159M+ L184M+ S256E+ Q271D+ I294E+ Q329E+ S402Q | 11.91 |
| Q68H+ Q82E+T92V+ P111Q+ K118A+ S123P+ K129T+R156Y+ V159M+ K169R+G200P+ V203T+ G237M+ T244R+ S256E+ Q271E+ I294E+ Q329E+N331F + N383E | Q82E+ P111Q+ S123P+ V159M+ K169R+ V203T+ G237M+ T244R+ S256E+ Q271E+ I294E+ Q329E+ N383E | 18.38 |
| Q68H+ Q82E+ T92V+ P111Q+K118A+ S123P+K129T+R156Y+ V159M+ K169R+ G200P+ V203T+ G237M+ T244R+ S256E+ Q271E+ I294E+ Q329E+ N331F + N383E | Q82E+ P111Q+ S123P+ V159M+ K169R+ V203T+ G237M+ T244R+ S256E+ Q271E+ I294E+ Q329E+ N383E | 18.40 |
| Q68H+ Q82E+ T92V+ P111Q+K118A+ S123P+K129T+ Q137K+R156Y+ V159M+ K169R+ A189G+ G200P+ V203T+ G237M+ T244R+ S256E+ I294E+ N331 F | Q82E+ P111Q+ S123P+ Q137K+ V159M+ K169R+ A189G+ V203T+ G237M+ T244R+ S256E+ I294E | 15.80 |
| Q68H+ Q82E+T92V+ P111Q+ K118A+ S123P+K129T+ Q137K+R156Y+ V159M+ K169R+ G200P+ V203T+ G237M+ T244R+ V251E+ S256E+ I294E+N331F | Q82E+ P111Q+ S123P+ Q137K+ V159M+ K169R+ V203T+ G237M+ T244R+ V251E+ S256E+ I294E | 17.56 |
| Q68H+ Q82E+ T92V+ P111Q+ K118A+ S123P+K129T+ L152P+ R156Y+ V159M+ K169R+G200P+ V203T+ G237M+ T244R+ S256E+ 1294E+N331F | Q82E+ P111Q+ S123P+ L152P+ V159M+ K169R+ V203T+ G237M+ T244R+ S256E+ I294E | 14.40 |
| Q68H+ Q82E+ T92V+ P111Q+K118A+ S123P+K129T+R156Y+ V159M+ K169R+ G200P+ V203T+ G237M+ T244R+ S256E+ I294E+ N331 F+N383Q+ V431 E | Q82E+ P111Q+ S123P+ V159M+ K169R+ V203T+ G237M+ T244R+ S256E+ I294E+ N383Q+ V431E | 15.84 |
| R20K+Q68H+ S76E+ Q82E+T92V+ P111Q+K118A+ S123P+ K129T+R156Y+ V159M+ K169R+ G200P+ V203T+ G237M+ T244R+S256E+ I294E+ Q329E+ N331F+ P492D | R20K+ S76E+ Q82E+ P111Q+ S123P+ V159M+ K169R+ V203T+ G237M+ T244R+ S256E+ I294E+ Q329E+ P492D | 22.50 |
| Q68H+ S76E+ Q82E+ T92V+ P111Q+K118A+ S123P+K129T+R156Y+ V159M+ K169R+ G200P+ V203T+ G237M+ T244R+ S256E+ I294E+ Q329E+ N331F + E489R+ P492D | S76E+ Q82E+ P111Q+ S123P+ V159M+ K169R+ V203T+ G237M+ T244R+ S256E+ I294E+ Q329E+ E489R+ P492D | 24.61 |
| Q68H+ Q82E+T92V+ P111Q+K118A+ S123P+K129T+ Q137K+ N155D+ R156Y+ V159M+ K169R+ G200P+ V203T+ G237M+ T244R+ S256E+ I294E+ N331F + K445E | Q82E+ P111Q+ S123P+ Q137K+ N155D+ V159M+ K169R+ V203T+ G237M+ T244R+ S256E+ I294E+ K445E | 16.48 |
| Q68H+ S76E+ Q82E+ T92V+ S94R+ P111Q+ K118A+ S123P+K129T+R156Y+ V159M+ K169R+G200P+ V203T+ G237M+ T244R+ S256E+ I294E + N331 F | S76E+ Q82E+ S94R+ P111Q+ S123P+ V159M+ K169R+ V203T+ G237M+ T244R+ S256E+ I294E | 14.39 |

(continued)

| Mutations compared to SEQ ID NO: 1 | Mutations compared to SEQ ID NO: 3 | HIF [90% Model A2 + 5% protease ] |
|---|---|---|
| K8E+ A41L+ S76E+Q68H+ Q82E+T92V+ P111Q+K118A+ S123P+K129T+R156Y+ V159M+ K169R+ G200P+ V203T+ G237M+ T244R+ S256E+ I294E +N331F | K8E+ A41L+ S76E+ Q82E+ P111Q+ S123P+ V159M+ K169R+ V203T+ G237M+ T244R+ S256E+ I294E | 14.94 |
| Q68H+ A83E+ T92V+ P111Q+K118A+ S123P+ K129T+ Q137K+ Q147K+R156Y+ V159M+ L184M+G200P+ S256E+ Q271E+ I294E+ Q329E+N331F+ P339S+ N383E+ S402Q+ V431E | A83E+ P111Q+ S123P+ Q137K+ Q147K+ V159M+ L184M+ S256E+ Q271E+ I294E+ Q329E+ P339S+ N383E+ S402Q+ V431E | 20.02 |
| Q68H+ S76E+ Q82E+T92V+ P111Q+K118A+ S123P+K129T+ Q137K+ Q147K+ R156Y+ V159M+ L184M+G200P+ V203T+ V251E+ S256Q+ Q271E+ I294E+ Q329E+ N331F + N383Q+ S402Q+ V431E | S76E+ Q82E+ P111Q+ S123P+ Q137K+ Q147K+ V159M+ L184M+ V203T+ V251E+ S256Q+ Q271E+ I294E+ Q329E+ N383Q+ S402Q+ V431E | 20.94 |
| Q68H+ A83E+T92V+ P111Q+ K118A+ S123P+K129T+ Q137K+ Q147K+ R156Y+ V159M+ L184M+ G200P+ K252E+ S256Q+ Q271E+ I294E+ Q329E+ N331F+ N383E+ S402Q+ V431E+ A491V | A83E+ P111Q+ S123P+ Q137K+ Q147K+ V159M+ L184M+ K252E+ S256Q+ Q271E+ I294E+ Q329E+ N383E+ S402Q+ V431E+ A491V | 15.93 |
| Q68H+ A83E+T92V+ P111Q+ K118A+ S123P+K129T+ Q137K+ Q147K+ R156Y+ V159M+ L184M+G200P+ V203T+ V251E+ S256Q+ Q271E+ I294E+ Q329E+ N331F+ N383Q+ S402Q+ V431E | A83E+ P111Q+ S123P+ Q137K+ Q147K+ V159M+ L184M+ V203T+ V251E+ S256Q+ Q271E+ I294E+ Q329E+ N383Q+ S402Q+ V431E | 17.39 |
| Q68H+ A83E+T92V+ P111Q+K118A+ S123P+ K129T+ Q137K+ Q147K+ R156Y+ V159M+ L184M+ G200P+ V203T+ K240F+ V251E+ S256Q+ Q271E+ I294E+Q329E+ N331F+ N383E+ S402Q+ V431E+ A491V | A83E+ P111Q+ S123P+ Q137K+ Q147K+ V159M+ L184M+ V203T+ K240F+ V251E+ S256Q+ Q271E+ I294E+ Q329E+ N383E+ S402Q+ V431E+ A491V | 28.32 |
| Q68H+ A83E+T92V+ P111Q+K118A+ S123P+ V219T+ Q137K+ Q147K+ R156Y+ V159M+ L184M+G200P+G237M+ S256Q+ Q271E+ I294E+ Q329E+ N331F+ S402Q+ V431E+ S474E | A83E+ P111Q+ S123P+ Q137K+ Q147K+ V159M+ L184M+ V219T+ G237M+ S256Q+ Q271E+ I294E+ Q329E+ S402Q+ V431E+ S474E | 19.25 |
| Q68H+ A83E+ T92V+ P111Q+K118A+ S123P+ K129T+ Q137K+ Q147K+ R156Y+ V159M+ L184M+ G200P+ V251E+ S256Q+ Q271E+ I294E+ Q329E+ N331F+ N383E+ S402Q+ V431E+ P492D | A83E+ P111Q+ S123P+ Q137K+ Q147K+ V159M+ L184M+ V251E+ S256Q+ Q271E+ I294E+ Q329E+ N383E+ S402Q+ V431E+ P492D | 13.92 |

(continued)

| Mutations compared to SEQ ID NO: 1 | Mutations compared to SEQ ID NO: 3 | HIF [90% Model A2 + 5% protease ] |
|---|---|---|
| Q68H+ S76E+ A83E+ T92V+ P111Q+ K118A+ S123P+K129T+ Q137K+ Q147K+ N155D+R156Y+ L184M+G200P+ G237M+ V251E+ S256Q+ Q271E+ I294E+ Q329E+ N331F+ N383E+ S402Q+ V431E+ A491V | S76E+ A83E+ P111Q+ S123P+ Q137K+ Q147K+ N155D+ L184M+ G237M+ V251E+ S256Q+ Q271E+ I294E+ Q329E+ N383E+ S402Q+ V431E+ A491V | 23.14 |
| Q68H+ A83E+ T92V+ P111Q+K118A+ S123P+ K129T+ Q137K+ Q147K+ R156Y+ V159M+ L184M+ A189G+G200P+ V251E+ S256Q+ Q271E+ I294E+ Q329E+ N331F+ N383Q+ S402Q+ V431E | A83E+ P111Q+ S123P+ Q137K+ Q147K+ V159M+ L184M+ A189G+ V251E+ S256Q+ Q271E+ I294E+ Q329E+ N383Q+ S402Q+ V431E | 14.04 |
| K8E+ A41L+Q68H+ A83E+T92V+ P111Q+ K118A+ S123P+K129T+ Q137K+ Q147K+ R156Y+ V159M+ L184M+ G200P+ V251E+ S256Q+ Q271E+ I294E+ Q329E+ N331F+ K394R+ S402Q+ V431E | K8E+ A41L+ A83E+ P111Q+ S123P+ Q137K+ Q147K+ V159M+ L184M+ V251E+ S256Q+ Q271E+ I294E+ Q329E+ K394R+ S402Q+ V431E | 22.20 |
| Q68H+ A83E+ T92V+ P111Q+K118A+ S123P+K129T+ Q137K+ Q147K+ R156Y+ V159M+ L184M+ G200P+ V251E+ S256Q+ Q271D+ I294E+ Q329E+ N331F+ N383E+ S402Q+ V431E+ A459P | A83E+ P111Q+ S123P+ Q137K+ Q147K+ V159M+ L184M+ V251E+ S256Q+ Q271D+ I294E+ Q329E+ N383E+ S402Q+ V431E+ A459P | 14.74 |
| Q68H+ A83E+T92V+ P111Q+K118A+ S123P+K129T+ Q137K+ Q147K+R156Y+ V159M+ L184M+ G200P+ V251E+ S256Q+ Q271E+ I294E+ Q329E+N331F+K347E+ N383E+ S402Q+ V431E+ A491V | A83E+ P111Q+ S123P+ Q137K+ Q147K+ V159M+ L184M+ V251E+ S256Q+ Q271E+ I294E+ Q329E+ K347E+ N383E+ S402Q+ V431E+ A491V | 14.64 |
| K8E+Q68H+ A83E+ T92V+ P111Q+K118A+K129T + S123P+ Q137K+ Q147K+R156Y+ V159M+ L184M+ G200P+ V251E+ S256Q+ Q271E+ I294E+ Q329E+ N331 F+N383E+ S402Q+ V431E+ A491V | K8E+ A83E+ P111Q+ S123P+ Q137K+ Q147K+ V159M+ L184M+ V251E+ S256Q+ Q271E+ I294E+ Q329E+ N383E+ S402Q+ V431E+ A491V | 14.94 |
| K8R+Q68H+ A83E+T92V+ P111Q+K118A+ S123P+K129T+ Q137K+ Q147K+ R156Y+ V159M+ L184M+ G200P+ V251E+ S256Q+ Q271E+ I294E+ Q329E+ N331 F+S402Q+ V431E+ E489K | K8R+ A83E+ P111Q+ S123P+ Q137K+ Q147K+ V159M+ L184M+ V251E+ S256Q+ Q271E+ I294E+ Q329E+ S402Q+ V431E+ E489K | 13.58 |
| Q68H+ A83E+T92V+ P111Q+ K118A+ S123P+K129T+ Q137K+ Q147K+ R156Y+ V159M+ L184M+G200P+ V251E+ S256Q+ Q271E+ I294E+ Q329E+ N331 F+N383E+ S402Q+ V431E+ A491V | A83E+ P111Q+ S123P+ Q137K+ Q147K+ V159M+ L184M+ V251E+ S256Q+ Q271E+ I294E+ Q329E+ N383E+ S402Q+ V431E+ A491V | 15.56 |

## Claims

1. A xyloglucanase variant of the polypeptide of SEQ ID NO: 2 comprising the substitutions P111Q+S123P+V159M+A129T, wherein the variant has xyloglucanase activity and wherein said variant has at least 85%, at least 90%, at least 91%, at least 92%, at least 93%, at least 94%, at least 95%, such as at least 96%, at least 97%, at least 98%, or at least 99% sequence identity to the polypeptide of SEQ ID NO: 1, SEQ ID NO: 2, or SEQ ID NO: 3, wherein the variant has a half-life improvement factor (HIF) and/or a stability improvement factor (SIF) of at least 1.1, such as at least 1.2, at least 1.3, at least 1.4, at least 1.5 compared to a reference polypeptide of SEQ ID NO: 2 or SEQ ID NO: 3.

2. The xyloglucanase variant of claim 1, comprising substitutions selected from the group consisting of

A41L+P111Q+S123P+Q147K+V159M+V203T+A129T,A41L+P111Q+S123P+Q147K+V159M+K217R+A129T,P111Q+S123P+Q147K+V159M+I294Q+S402Q+A129T,

A83E+P111Q+S123P+V159M+S256E+I294E+A129T,

Q82E+P111Q+S123P+V159M+S256E+I294E+A129T,

Q82E+P111Q+S123P+Q147K+V159M+I294E+A129T,K87E+P111Q+S123P+V159M+K217T+I294E+A129T,

A83E+P111Q+S123P+V159M+K240F+K252E+A129T,K87E+P111Q+S123P+V159M+S256Q+I294E+A129T,

and

K87E+P111Q+S123P+V159M+K347E+N383E+A129T

3. A detergent composition comprising the variant of any of claims 1-2.

4. The detergent composition of claim 3, in the form of a bar, a homogenous tablet, a tablet having two or more layers, a unit dose product such as a pouch having one or more compartments, a regular or compact powder, a granule, a paste, a gel, or a regular, compact or concentrated liquid.

5. An isolated polynucleotide encoding the variant of any of claims 1-2.

6. A nucleic acid construct or expression vector comprising the polynucleotide of claim 5.

7. A recombinant host cell transformed with the polynucleotide of claim 5.

8. A method of producing a variant comprising:

   a) cultivating the recombinant host cell of claim 7 under conditions suitable for expression of the variant; and
   b) recovering the variant.

9. Use of the variant according to any one of claims 1-2 or the detergent composition of claims 3-4 for cleaning an item, for pretreating stains on the item, for preventing, reducing, or removing redeposition of soil during a wash cycle, and/or for

maintaining or improving the whiteness of an item.

**10.** Use of claim 9, wherein the item is a textile or a hard surface, such as dishware.

**11.** A laundering method for laundering an item comprising

a) exposing an item to a wash liquor comprising the variant of any of claims 1-2 or the detergent composition of any of claims 3-4;
b) completing at least one wash cycle; and
c) optionally rinsing the item,

wherein the item is a textile.

## Patentansprüche

**1.** Xyloglucanase-Variante des Polypeptids von SEQ ID NO: 2, umfassend die Substitutionen P111Q+S123P+V159M+A129T, wobei die Variante Xyloglucanase-Aktivität aufweist und wobei die Variante mindestens 85 %, mindestens 90 %, mindestens 91 %, mindestens 92 %, mindestens 93 %, mindestens 94 %, mindestens 95 %, wie mindestens 96 %, mindestens 97 %, mindestens 98 % oder mindestens 99 % Sequenzidentität mit dem Polypeptid von SEQ ID NO: 1, SEQ ID NO: 2 oder SEQ ID NO: 3 aufweist, wobei die Variante einen Halbwertszeit-Verbesserungsfaktor (HIF) und/oder einen Stabilitätsverbesserungsfaktor (SIF) von mindestens 1,1, wie mindestens 1,2, mindestens 1,3, mindestens 1,4, mindestens 1,5, aufweist, verglichen mit einem Referenzpolypeptid von SEQ ID NO: 2 oder SEQ ID NO: 3.

**2.** Xyloglucanase-Variante nach Anspruch 1, umfassend Substitutionen, die ausgewählt sind aus der Gruppe bestehend aus A41L+P111Q+S123P+Q147K+V159M+V203T+A129T,A41L+P111Q+S123P+Q147K+V159M+K217R+A129T,P111Q+S123P+Q147K+V159M+I294Q+S402Q+A129T,

A83E+P111Q+S123P+V159M+S256E+I294E+A129T,

Q82E+P111Q+S123P+V159M+S256E+I294E+A129T,

Q82E+P111Q+S123P+Q147K+V159M+I294E+A129T,K87E+P111Q+S123P+V159M+K217T+I294E+A129T,

A83E+P111Q+S123P+V159M+K240F+K252E+A129T,K87E+P111Q+S123P+V159M+S256Q+I294E+A129T,

und

K87E+P111Q+S123P+V159M+K347E+N383E+A129T

**3.** Waschmittelzusammensetzung, umfassend die Variante nach einem der Ansprüche 1 bis 2.

**4.** Waschmittelzusammensetzung nach Anspruch 3, in Form eines Stücks, einer homogenen Tablette, einer Tablette mit zwei oder mehr Schichten, eines Einzeldosisprodukts, wie ein Beutel, mit einem oder mehreren Fächern, eines regulären oder kompakten Pulvers, eines Granulats, einer Paste, eines Gels oder einer regulären, kompakten oder konzentrierten Flüssigkeit.

5. Isoliertes Polynukleotid, das die Variante nach einem der Ansprüche 1 bis 2 codiert.

6. Nukleinsäurekonstrukt oder Expressionsvektor, umfassend das Polynukleotid nach Anspruch 5.

7. Rekombinante Wirtszelle, die mit dem Polynukleotid nach Anspruch 5 transformiert ist.

8. Verfahren zur Herstellung einer Variante, umfassend:

a) Kultivieren der rekombinanten Wirtszelle nach Anspruch 7 unter Bedingungen, die für die Expression der Variante geeignet sind; und
b) Gewinnen der Variante.

9. Verwendung der Variante nach einem der Ansprüche 1 bis 2 oder der Waschmittelzusammensetzung nach den Ansprüchen 3 bis 4 zum Reinigen eines Gegenstands, zum Vorbehandeln von Flecken auf dem Gegenstand, zum Verhindern, Verringern oder Entfernen von Wiederablagerung von Schmutz während eines Waschzyklus und/oder zum Aufrechterhalten oder Verbessern des Weißgrads eines Gegenstands.

10. Verwendung nach Anspruch 9, wobei der Gegenstand ein Textil oder eine harte Oberfläche, wie Geschirr, ist.

11. Waschverfahren zum Waschen eines Gegenstands, umfassend

a) Aussetzen eines Gegenstands einer Waschflotte, umfassend die Variante nach einem der Ansprüche 1 bis 2 oder die Waschmittelzusammensetzung nach einem der Ansprüche 3 bis 4;
b) Beenden von mindestens einem Waschzyklus; und
c) gegebenenfalls Spülen des Gegenstands,

wobei der Gegenstand ein Textil ist.

## Revendications

1. Variant de xyloglucanase du polypeptide de SEQ ID NO: 2 comprenant les substitutions P111Q+S123P+V159M+A129T, dans lequel le variant a une activité de xyloglucanase et dans lequel ledit variant a au moins 85 %, au moins 90 %, au moins 91 %, au moins 92 %, au moins 93 %, au moins 94 %, au moins 95 %, par exemple au moins 96 %, au moins 97 %, au moins 98 % ou au moins 99 % d'identité de séquence avec le polypeptide de SEQ ID NO: 1, SEQ ID NO: 2, ou SEQ ID NO: 3, dans lequel le variant a un facteur d'amélioration de demi-vie (HIF) et/ou un facteur d'amélioration de stabilité (SIF) d'au moins 1,1, par exemple d'au moins 1,2, d'au moins 1,3, d'au moins 1,4, d'au moins 1,5 par rapport à un polypeptide de référence de SEQ ID NO: 2 ou SEQ ID NO: 3.

2. Variant de xyloglucanase selon la revendication 1, comprenant des substitutions choisies dans le groupe constitué par A41L+P111Q+S123P+Q147K+V159M+V203T+A129T,A41L+P111Q+S123P+Q147K+V159M+K217R+A129T,P111Q+S123P+Q147K+V159M+I294Q+S402Q+A129T,

A83E+P111Q+S123P+V159M+S256E+I294E+A129T,

Q82E+P111Q+S123P+V159M+S256E+I294E+A129T,

Q82E+P111Q+S123P+Q147K+V159M+I294E+A129T,K87E+P111Q+S123P+V159M+K217T+I294E+A129T,

A83E+P111Q+S123P+V159M+K240F+K252E+A129T,K87E+P111Q+S123P+V159M+S256Q+I294E+A129T,

et

K87E+P111Q+S123P+V159M+K347E+N383E+A129T.

**3.** Composition de détergent comprenant le variant selon l'une quelconque des revendications 1 à 2.

**4.** Composition de détergent selon la revendication 3, sous la forme d'une barre, d'un comprimé homogène, d'un comprimé ayant deux couches ou plus, d'un produit en dose unitaire tel qu'une poche ayant un ou plusieurs compartiments, d'une poudre ordinaire ou compacte, d'un granulé, d'une pâte, d'un gel ou d'un liquide ordinaire, compact ou concentré.

**5.** Polynucléotide isolé codant pour le variant selon l'une quelconque des revendications 1 à 2.

**6.** Construction d'acide nucléique ou vecteur d'expression comprenant le polynucléotide selon la revendication 5.

**7.** Cellule hôte recombinante transformée avec le polynucléotide selon la revendication 5.

**8.** Procédé de production d'un variant comprenant :

a) la culture de la cellule hôte recombinante selon la revendication 7 dans des conditions appropriées pour l'expression du variant ; et
b) la récupération du variant.

**9.** Utilisation du variant selon l'une quelconque des revendications 1 à 2 ou de la composition de détergent selon les revendications 3 à 4 pour nettoyer un article, pour prétraiter des taches sur l'article, pour empêcher, réduire ou éliminer un redépôt de salissures pendant un cycle de lavage, et/ou pour maintenir ou améliorer la blancheur d'un article.

**10.** Utilisation selon la revendication 9, dans laquelle l'article est un textile ou une surface dure, telle que de la vaisselle.

**11.** Procédé de lessivage pour lessiver un article comprenant

a) exposer un article à une liqueur de lavage comprenant le variant selon l'une quelconque des revendications 1 à 2 ou la composition de détergent selon l'une quelconque des revendications 3 à 4 ;
b) effectuer au moins un cycle de lavage ; et
c) éventuellement rincer l'article,

dans lequel l'article est un textile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9414953 A
- WO 9902663 A
- WO 01062903 A
- WO 2009147210 A
- WO 2009148983 A
- WO 2010056652 A
- WO 0162903 A
- US 20040171154
- WO 9517413 A
- WO 9522625 A
- US 5223409 A
- WO 9206204 A
- WO 9943835 A
- WO 9600787 A
- WO 0056900 A
- WO 9425612 A
- WO 9533836 A
- WO 0024883 A
- EP 238023 A
- WO 9015861 A
- WO 2010096673 A
- WO 09118375 A
- WO 9813459 A
- WO 9219709 A
- WO 9219708 A
- US 6472364 B
- WO 9404651 A
- WO 9525791 A
- WO 9813458 A
- WO 9813460 A
- WO 9813461 A
- WO 9813462 A
- WO 07141736 A
- WO 07145963 A
- WO 10055052 A
- WO 11036153 A
- WO 9707202 A
- WO 09102854 A
- US 5977053 A
- WO 9817767 A
- EP 624154 A
- WO 2007087258 A
- WO 2007087244 A
- WO 2007087259 A
- EP 1867708 A, Vitamin K
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- WO 1999064619 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A, Genencor Int.
- WO 9219729 A
- WO 96034946 A
- WO 9820115 A
- WO 9820116 A
- WO 99011768 A
- WO 0144452 A

- WO 03006602 A
- WO 0403186 A
- WO 04041979 A
- WO 07006305 A
- WO 11036263 A
- WO 11036264 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 2011098579 A
- WO 2014087011 A
- WO 2017060475 A
- WO 2015155350 A
- WO 2017097866 A
- WO 2017129754 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- EP 2169040 A
- WO 2013188331 A
- US 20090011970 A1

**Non-patent literature cited in the description**


- **VINCKEN et al.** *Carbohydrate Research*, 1997, vol. 298 (4), 299-310
- **HENRISSAT, B.** *Biochem. J.*, 1991, vol. 280, 309-316
- **HENRISSAT, B.** ; **BAIROCH, A.** *Biochem. J.*, 1993, vol. 293, 781-788
- **ZHANG et al.** *Biotechnology Advances*, 2006, vol. 24, 452-481
- **GHOSE**. *Pure Appl. Chem.*, 1987, vol. 59, 257-68
- **COOPER et al.** *EMBO J.*, 1993, vol. 12, 2575-2583
- **DAWSON et al.** *Science*, 1994, vol. 266, 776-779
- **MARTIN et al.** *J. Ind. Microbiol. Biotechnol.*, 2003, vol. 3, 568-576

- **SVETINA et al.** *J. Biotechnol.*, 2000, vol. 76, 245-251
- **RASMUSSEN-WILSON et al.** *Appl. Environ. Microbiol.*, 1997, vol. 63, 3488-3493
- **WARD et al.** *Biotechnology*, 1995, vol. 13, 498-503
- **CONTRERAS et al.** *Biotechnology*, 1991, vol. 9, 378-381
- **EATON et al.** *Biochemistry*, 1986, vol. 25, 505-512
- **COLLINS-RACIE et al.** *Biotechnology*, 1995, vol. 13, 982-987
- **CARTER et al.** *Proteins: Structure, Function, and Genetics*, 1989, vol. 6, 240-248
- **STEVENS**. *Drug Discovery World*, 2003, vol. 4, 35-48
- **NEEDLEMAN** ; **WUNSCH**. *J. Mol. Biol.*, 1970, vol. 48, 443-453
- **RICE et al.** *Trends Genet.*, 2000, vol. 16, 276-277
- **RICE et al.** *EMBOSS: The European Molecular Biology Open Software Suite*, 2000
- **RICE et al.** EMBOSS: The European Molecular Biology Open Software Suite. *Trends Genet.*, 2000, vol. 16, 276-277
- **H. NEURATH** ; **R.L. HILL**. The Proteins. Academic Press, 1979
- **CUNNINGHAM** ; **WELLS**. *Science*, 1989, vol. 244, 1081-1085
- **HILTON et al.** *J. Biol. Chem.*, 1996, vol. 271, 4699-4708
- **VOS et al.** *Science*, 1992, vol. 255, 306-312
- **SMITH et al.** *J. Mol. Biol.*, 1992, vol. 224, 899-904
- **WLODAVER et al.** *FEBS Lett.*, 1992, vol. 309, 59-64
- **SCHERER** ; **DAVIS**. *Proc. Natl. Acad. Sci. USA*, 1979, vol. 76, 4949-4955
- **BARTON et al.** *Nucleic Acids Res.*, 1990, vol. 18, 7349-4966
- **STORICI et al.** *Nature Biotechnol.*, 2001, vol. 19, 773-776
- **KREN et al.** *Nat.*, 1998, vol. 4, 285-290
- **CALISSANO** ; **MACINO**. *Fungal Genet. Newslett.*, 1996, vol. 43, 15-16
- **TIAN et al.** *Nature*, 2004, vol. 432, 1050-1054
- **REIDHAAR-OLSON** ; **SAUER**. *Science*, 1988, vol. 241, 53-57
- **BOWIE** ; **SAUER**. *Proc. Natl. Acad. Sci. USA*, 1989, vol. 86, 2152-2156
- **LOWMAN et al.** *Biochemistry*, 1991, vol. 30, 10832-10837
- **DERBYSHIRE et al.** *Gene*, 1986, vol. 46, 145
- **NER et al.** *DNA*, 1988, vol. 7, 127
- **NESS et al.** *Nature Biotechnology*, 1999, vol. 17, 893-896
- **AGAISSE** ; **LERECLUS**. *Molecular Microbiology*, 1994, vol. 13, 97-107
- **EGON et al.** *Gene*, 1988, vol. 69, 301-315
- **VILLA-KAMAROFF et al.** *Proc. Natl. Acad. Sci. USA*, 1978, vol. 75, 3727-3731
- **DEBOER et al.** *Proc. Natl. Acad. Sci. USA*, 1983, vol. 80, 21-25
- **GILBERT et al.** *Scientific American*, 1980, vol. 242, 74-94
- **ROMANOS et al.** *Yeast*, 1992, vol. 8, 423-488
- **HUE et al.** *Journal of Bacteriology*, 1995, vol. 177, 3465-3471
- **GUO** ; **SHERMAN**. *Mol. Cellular Biol.*, 1995, vol. 15, 5983-5990
- **SIMONEN** ; **PALVA**. *Microbiological Reviews*, 1993, vol. 57, 109-137
- **GEMS et al.** *Gene*, 1991, vol. 98, 61-67
- **CULLEN et al.** *Nucleic Acids Res.*, 1987, vol. 15, 9163-9175
- **CHANG** ; **COHEN**. *Mol. Gen. Genet.*, 1979, vol. 168, 111-115
- **YOUNG** ; **SPIZIZEN**. *J. Bacteriol.*, 1961, vol. 81, 823-829
- **DUBNAU** ; **DAVIDOFF-ABELSON**. *J. Mol. Biol.*, 1971, vol. 56, 209-221
- **SHIGEKAWA** ; **DOWER**. *Biotechniques*, 1988, vol. 6, 742-751
- **KOEHLER** ; **THORNE**. *J. Bacteriol.*, 1987, vol. 169, 5271-5278
- **HANAHAN**. *J. Mol. Biol.*, 1983, vol. 166, 557-580
- **DOWER et al.** *Nucleic Acids Res.*, 1988, vol. 16, 6127-6145
- **GONG et al.** *Folia Microbiol. (Praha)*, 2004, vol. 49, 399-405
- **MAZODIER et al.** *J. Bacteriol.*, 1989, vol. 171, 3583-3585
- **BURKE et al.** *Proc. Natl. Acad. Sci. USA*, 2001, vol. 98, 6289-6294
- **CHOI et al.** *J. Microbiol. Methods*, 2006, vol. 64, 391-397
- **PINEDO** ; **SMETS**. *Appl. Environ. Microbiol.*, 2005, vol. 71, 51-57
- **PERRY** ; **KURAMITSU**. *Infect. Immun.*, 1981, vol. 32, 1295-1297
- **CATT** ; **JOLLICK**. *Microbios*, 1991, vol. 68, 189-207
- **BUCKLEY et al.** *Appl. Environ. Microbiol.*, 1999, vol. 65, 3800-3804
- **CLEWELL**. *Microbiol. Rev.*, 1981, vol. 45, 409-436
- **HAWKSWORTH et al.** n, Ainsworth and Bisby's Dictionary of The Fungi. University Press, 1995
- Biology and Activities of Yeast. Soc. App. Bacteriol. Symposium Series. 1980
- **YELTON et al.** *Proc. Natl. Acad. Sci. USA*, 1984, vol. 81, 1470-1474
- **CHRISTENSEN et al.** *Bio/Technology*, 1988, vol. 6, 1419-1422

- **MALARDIER et al.** *Gene*, 1989, vol. 78, 147-156
- Guide to Yeast Genetics and Molecular Biology. Methods in Enzymology. Academic Press, vol. 194, 182-187
- **ITO et al.** *J. Bacteriol.*, 1983, vol. 153, 163
- **HINNEN et al.** *Proc. Natl. Acad. Sci. USA*, 1978, vol. 75, 1920
- Protein Purification. VCH Publishers, 1989
- **HODGDON** ; **KALER**. *Current Opinion in Colloid & Interface Science*, 2007, vol. 12, 121-128
- **SIEZEN et al.** *Protein Eng.*, 1991, vol. 4, 719-737
- **SIEZEN et al.** *Protein Science*, 1997, vol. 6, 501-523
- Powdered Detergents, Surfactant science series. Marcel Dekker, Inc, vol. 71
- Powdered Detergents, Surfactant science. Marcel Dekker, Inc., vol. 71
- **SAMBROOK et al.** Molecular cloning: A laboratory manual. Cold Spring Harbor lab.
- Current protocols in Molecular Biology. John Wiley and Sons, 1995
- DNA Cloning: A Practical Approach, Volumes I and II. 1985
- Oligonucleotide Synthesis. 1984
- Nucleic Acid Hybridization. 1985
- **B. PERBAL**. A Practical Guide To Molecular Cloning. 1984